(19) **Europäisches Patentamt / European Patent Office / Office européen des brevets**

(11) **EP 4 079 758 A1**

(12) **EUROPEAN PATENT APPLICATION**
published in accordance with Art. 153(4) EPC

(43) Date of publication:
**26.10.2022  Bulletin 2022/43**

(21) Application number: **21744026.2**

(22) Date of filing: **21.01.2021**

(51) International Patent Classification (IPC):
*C07K 16/00* (2006.01)     *C12N 15/13* (2006.01)
*A61K 39/395* (2006.01)     *A61P 35/00* (2006.01)

(52) Cooperative Patent Classification (CPC):
**A61K 39/395; A61P 35/00; C07K 16/00**

(86) International application number:
**PCT/CN2021/073100**

(87) International publication number:
**WO 2021/147954 (29.07.2021 Gazette 2021/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **21.01.2020  CN 202010072952**

(71) Applicant: **Shanghai Biotroy Biotechnique Co., Ltd.**
**Shanghai 200131 (CN)**

(72) Inventor: **LI, Zhaoli**
**Beijing 102299 (CN)**

(74) Representative: **Engelhard, Markus**
**Boehmert & Boehmert**
**Anwaltspartnerschaft mbB**
**Pettenkoferstrasse 22**
**80336 München (DE)**

(54) **SEMG2 ANTIBODY AND USE THEREOF**

(57)    The present invention provides a compound agonizing or antagonizing the interaction between SEMG2 and CD27, comprising a small molecule inhibitor, a polypeptide, an antibody, or an antigen-binding fragment. The present invention further discloses methods of preparing antibodies for blocking the binding between SEMG2 and CD27 using the polypeptides as an immunogen with high efficiency. The present invention discloses methods of promoting anti-tumor immunity by blocking the contact of SEMG2 expressed by tumor cells with CD27 expressed by immune cells, also discloses a screening method for screening a therapeutic drug by blocking the binding between SEMG2 and CD27.

Figure 1

EP 4 079 758 A1

Sequence listing

<110>   Shanghai Biotroy Biotechnique Co., Ltd.

<120>   SEMG2 antibody and use thereof

<130>   S34420WOEP

<140>   EP21744026.2
<141>   2021-01-21

<150>   CN202010072952.X
<151>   2020-01-21

<160>   103

<170>   SIPOSequenceListing 1.0

<210>   1
<211>   582
<212>   PRT
<213>   Homo sapiens

<220>
<221>   UNSURE
<222>   (1)..(582)
<223>   Human SEMG2

<400>   1
Met Lys Ser Ile Ile Leu Phe Val Leu Ser Leu Leu Leu Ile Leu Glu
1               5                   10                  15
Lys Gln Ala Ala Val Met Gly Gln Lys Gly Gly Ser Lys Gly Gln Leu
            20                  25                  30
Pro Ser Gly Ser Ser Gln Phe Pro His Gly Gln Lys Gly Gln His Tyr
        35                  40                  45
Phe Gly Gln Lys Asp Gln Gln His Thr Lys Ser Lys Gly Ser Phe Ser
    50                  55                  60
Ile Gln His Thr Tyr His Val Asp Ile Asn Asp His Asp Trp Thr Arg
65                  70                  75                  80
Lys Ser Gln Gln Tyr Asp Leu Asn Ala Leu His Lys Ala Thr Lys Ser
                85                  90                  95
Lys Gln His Leu Gly Gly Ser Gln Gln Leu Leu Asn Tyr Lys Gln Glu
            100                 105                 110
Gly Arg Asp His Asp Lys Ser Lys Gly His Phe His Met Ile Val Ile
        115                 120                 125
His His Lys Gly Gly Gln Ala His His Gly Thr Gln Asn Pro Ser Gln
    130                 135                 140
Asp Gln Gly Asn Ser Pro Ser Gly Lys Gly Leu Ser Ser Gln Cys Ser
145                 150                 155                 160
Asn Thr Glu Lys Arg Leu Trp Val His Gly Leu Ser Lys Glu Gln Ala
                165                 170                 175
Ser Ala Ser Gly Ala Gln Lys Gly Arg Thr Gln Gly Gly Ser Gln Ser
            180                 185                 190
Ser Tyr Val Leu Gln Thr Glu Glu Leu Val Val Asn Lys Gln Gln Arg
            195                 200                 205
Glu Thr Lys Asn Ser His Gln Asn Lys Gly His Tyr Gln Asn Val Val
        210                 215                 220
Asp Val Arg Glu Glu His Ser Ser Lys Leu Gln Thr Ser Leu His Pro
225                 230                 235                 240
Ala His Gln Asp Arg Leu Gln His Gly Pro Lys Asp Ile Phe Thr Thr
                245                 250                 255
Gln Asp Glu Leu Leu Val Tyr Asn Lys Asn Gln His Gln Thr Lys Asn

```
                    260                    265                    270
Leu Ser Gln Asp Gln Glu His Gly Arg Lys Ala His Lys Ile Ser Tyr
        275                280                285
Pro Ser Ser Arg Thr Glu Glu Arg Gln Leu His His Gly Glu Lys Ser
        290                295                300
Val Gln Lys Asp Val Ser Lys Gly Ser Ile Ser Ile Gln Thr Glu Glu
305                310                315                320
Lys Ile His Gly Lys Ser Gln Asn Gln Val Thr Ile His Ser Gln Asp
                325                330                335
Gln Glu His Gly His Lys Glu Asn Lys Ile Ser Tyr Gln Ser Ser Ser
            340                345                350
Thr Glu Glu Arg His Leu Asn Cys Gly Glu Lys Gly Ile Gln Lys Gly
        355                360                365
Val Ser Lys Gly Ser Ile Ser Ile Gln Thr Glu Glu Gln Ile His Gly
        370                375                380
Lys Ser Gln Asn Gln Val Arg Ile Pro Ser Gln Ala Gln Glu Tyr Gly
385                390                395                400
His Lys Glu Asn Lys Ile Ser Tyr Gln Ser Ser Thr Glu Glu Arg
                405                410                415
Arg Leu Asn Ser Gly Glu Lys Asp Val Gln Lys Gly Val Ser Lys Gly
                420                425                430
Ser Ile Ser Ile Gln Thr Glu Glu Lys Ile His Gly Lys Ser Gln Asn
            435                440                445
Gln Val Thr Ile Pro Ser Gln Asp Gln Glu His Gly His Lys Glu Asn
    450                455                460
Lys Met Ser Tyr Gln Ser Ser Ser Thr Glu Glu Arg Arg Leu Asn Tyr
465                470                475                480
Gly Gly Lys Ser Thr Gln Lys Asp Val Ser Gln Ser Ser Ile Ser Phe
                485                490                495
Gln Ile Glu Lys Leu Val Glu Gly Lys Ser Gln Ile Gln Thr Pro Asn
            500                505                510
Pro Asn Gln Asp Gln Trp Ser Gly Gln Asn Ala Lys Gly Lys Ser Gly
        515                520                525
Gln Ser Ala Asp Ser Lys Gln Asp Leu Leu Ser His Glu Gln Lys Gly
    530                535                540
Arg Tyr Lys Gln Glu Ser Ser Glu Ser His Asn Ile Val Ile Thr Glu
545                550                555                560
His Glu Val Ala Gln Asp Asp His Leu Thr Gln Gln Tyr Asn Glu Asp
                565                570                575
Arg Asn Pro Ile Ser Thr
                580
```

```
<210>   2
<211>   10
<212>   PRT
<213>   Artificial Sequence

<220>
<221>   UNSURE
<222>   (1)..(10)
<223>   Epitope peptides


<400>   2
Gln Ile Glu Lys Leu Val Glu Gly Lys Ser
1               5                   10


<210>   3
<211>   13
<212>   PRT
<213>   Artificial Sequence

<220>
```

```
<221>  UNSURE
<222>  (1)..(13)
<223>  Epitope peptides


<400>  3
Gln Ile Glu Lys Leu Val Glu Gly Lys Ser Gln Ile Gln
1               5                   10


<210>  4
<211>  11
<212>  PRT
<213>  Artificial Sequence


<220>
<221>  UNSURE
<222>  (1)..(11)
<223>  Epitope peptides


<400>  4
Gln Ile Glu Lys Leu Val Glu Gly Lys Ser Gln
1               5                   10


<210>  5
<211>  12
<212>  PRT
<213>  Artificial Sequence


<220>
<221>  UNSURE
<222>  (1)..(12)
<223>  Epitope peptides


<400>  5
Gln Ile Glu Lys Leu Val Glu Gly Lys Ser Gln Ile
1               5                   10


<210>  6
<211>  8
<212>  PRT
<213>  Artificial Sequence


<220>
<221>  UNSURE
<222>  (1)..(8)
<223>  VH CDR1


<400>  6
Gly Tyr Thr Leu Thr Thr Ala Gly
1               5


<210>  7
<211>  8
<212>  PRT
<213>  Artificial Sequence


<220>
<221>  UNSURE
<222>  (1)..(8)
<223>  VH CDR1
```

```
<400>  7
Gly Tyr Thr Phe Thr Ser Tyr Trp
1               5

<210>  8
<211>  8
<212>  PRT
<213>  Artificial Sequence

<220>
<221>  UNSURE
<222>  (1)..(8)
<223>  VH CDR1


<400>  8
Asp Tyr Thr Phe Thr Arg Tyr Trp
1               5

<210>  9
<211>  8
<212>  PRT
<213>  Artificial Sequence

<220>
<221>  UNSURE
<222>  (1)..(8)
<223>  VH CDR1


<400>  9
Gly Phe Asn Ile Lys Asp Tyr Tyr
1               5

<210>  10
<211>  8
<212>  PRT
<213>  Artificial Sequence

<220>
<221>  UNSURE
<222>  (1)..(8)
<223>  VH CDR1


<400>  10
Gly Phe Ser Leu Thr Ser Tyr Ala
1               5

<210>  11
<211>  8
<212>  PRT
<213>  Artificial Sequence

<220>
<221>  UNSURE
<222>  (1)..(8)
<223>  VH CDR1


<400>  11
```

```
Gly Phe Ser Leu Thr Arg Tyr Gly
1               5


<210>  12
<211>  8
<212>  PRT
<213>  Artificial Sequence


<220>
<221>  UNSURE
<222>  (1)..(8)
<223>  VH CDR2



<400>  12
Ile Asn Thr His Ser Gly Val Pro
1               5


<210>  13
<211>  8
<212>  PRT
<213>  Artificial Sequence


<220>
<221>  UNSURE
<222>  (1)..(8)
<223>  VH CDR2



<400>  13
Ile Asp Pro Ser Asp Ser Glu Thr
1               5


<210>  14
<211>  8
<212>  PRT
<213>  Artificial Sequence


<220>
<221>  UNSURE
<222>  (1)..(8)
<223>  VH CDR2



<400>  14
Ile Asp Pro Glu Asn Gly Asp Asn
1               5


<210>  15
<211>  7
<212>  PRT
<213>  Artificial Sequence


<220>
<221>  UNSURE
<222>  (1)..(7)
<223>  VH CDR2



<400>  15
Ile Trp Gly Asp Gly Ser Thr
1               5
```

```
<210>  16
<211>  8
<212>  PRT
<213>  Artificial Sequence

<220>
<221>  UNSURE
<222>  (1)..(8)
<223>  VH CDR2


<400>  16
Ile Asn Thr His Ser Gly Val Ala
1               5

<210>  17
<211>  8
<212>  PRT
<213>  Artificial Sequence

<220>
<221>  UNSURE
<222>  (1)..(8)
<223>  VH CDR3


<400>  17
Ala Arg Leu Gly Leu Leu Gly Tyr
1               5

<210>  18
<211>  13
<212>  PRT
<213>  Artificial Sequence

<220>
<221>  UNSURE
<222>  (1)..(13)
<223>  VH CDR3


<400>  18
Ala Arg Tyr Leu Gly Gly Lys Glu Gly Ser Phe Asp Tyr
1               5                   10

<210>  19
<211>  7
<212>  PRT
<213>  Artificial Sequence

<220>
<221>  UNSURE
<222>  (1)..(7)
<223>  VH CDR3


<400>  19
Asn Val Gly Gly Ala His Tyr
1               5

<210>  20
<211>  16
<212>  PRT
```

<213> Artificial Sequence

<220>
<221> UNSURE
<222> (1)..(16)
<223> VH CDR3


<400> 20
Ala Lys Gln Glu Arg Phe Ser Asp Gly Tyr Tyr Asp Gly Phe Ala Tyr
1               5                   10                  15

<210> 21
<211> 6
<212> PRT
<213> Artificial Sequence

<220>
<221> UNSURE
<222> (1)..(6)
<223> VL CDR1


<400> 21
Gln Ser Ile Gly Thr Thr
1               5

<210> 22
<211> 12
<212> PRT
<213> Artificial Sequence

<220>
<221> UNSURE
<222> (1)..(12)
<223> VL CDR1


<400> 22
Gln Ser Leu Leu Asn Ser Arg Thr Arg Lys Asn Tyr
1               5                   10

<210> 23
<211> 12
<212> PRT
<213> Artificial Sequence

<220>
<221> UNSURE
<222> (1)..(12)
<223> VL CDR1


<400> 23
Gln Ser Leu Phe Asn Ser Arg Thr Arg Lys Asn Tyr
1               5                   10

<210> 24
<211> 11
<212> PRT
<213> Artificial Sequence

<220>

```
<221>  UNSURE
<222>  (1)..(11)
<223>  VL CDR1


<400>  24
Gln Ser Leu Val His Ser Asn Gly Asn Thr Tyr
1                   5                   10


<210>  25
<211>  5
<212>  PRT
<213>  Artificial Sequence

<220>
<221>  UNSURE
<222>  (1)..(5)
<223>  VL CDR1


<400>  25
Ser Ser Val Ser Tyr
1                   5


<210>  26
<211>  2
<212>  PRT
<213>  Artificial Sequence

<220>
<221>  UNSURE
<222>  (1)..(2)
<223>  VL CDR2


<400>  26
Tyr Ala
1


<210>  27
<211>  2
<212>  PRT
<213>  Artificial Sequence

<220>
<221>  UNSURE
<222>  (1)..(2)
<223>  VL CDR2


<400>  27
Trp Ala
1


<210>  28
<211>  2
<212>  PRT
<213>  Artificial Sequence

<220>
<221>  UNSURE
<222>  (1)..(2)
<223>  VL CDR2
```

```
<400>  28
Lys Val
1


<210>  29
<211>  2
<212>  PRT
<213>  Artificial Sequence

<220>
<221>  UNSURE
<222>  (1)..(2)
<223>  VL CDR2


<400>  29
Ser Thr
1


<210>  30
<211>  9
<212>  PRT
<213>  Artificial Sequence

<220>
<221>  UNSURE
<222>  (1)..(9)
<223>  VL CDR3


<400>  30
Gln Gln Ser Asn Ser Trp Pro Trp Thr
1               5


<210>  31
<211>  9
<212>  PRT
<213>  Artificial Sequence

<220>
<221>  UNSURE
<222>  (1)..(9)
<223>  VL CDR3


<400>  31
Lys Gln Ser Tyr Ser Leu Pro Trp Thr
1               5


<210>  32
<211>  9
<212>  PRT
<213>  Artificial Sequence

<220>
<221>  UNSURE
<222>  (1)..(9)
<223>  VL CDR3


<400>  32
```

Lys Gln Ser Tyr Glu Leu Pro Trp Thr
1               5

<210>   33
<211>   9
<212>   PRT
<213>   Artificial Sequence

<220>
<221>   UNSURE
<222>   (1)..(9)
<223>   VL CDR3

<400>   33
Ser Gln Ser Thr His Val Pro Tyr Thr
1               5

<210>   34
<211>   9
<212>   PRT
<213>   Artificial Sequence

<220>
<221>   UNSURE
<222>   (1)..(9)
<223>   VL CDR3

<400>   34
Gln Gln Arg Ser Ser Tyr Pro Phe Thr
1               5

<210>   35
<211>   115
<212>   PRT
<213>   Artificial Sequence

<220>
<221>   UNSURE
<222>   (1)..(115)
<223>   VH

<400>   35
Gln Ile Gln Leu Val Gln Ser Gly Pro Glu Val Lys Lys Pro Gly Glu
1               5                   10                  15
Thr Val Arg Ile Ser Cys Lys Ala Ser Gly Tyr Thr Leu Thr Thr Ala
        20                  25                  30
Gly Ile Gln Trp Val Gln Lys Met Pro Gly Lys Gly Leu Lys Trp Ile
        35                  40                  45
Gly Trp Ile Asn Thr His Ser Gly Val Pro Glu Tyr Ala Glu Asp Phe
        50                  55                  60
Lys Gly Arg Phe Ala Phe Phe Leu Glu Thr Ser Ala Ser Thr Ala Tyr
65                  70                  75                  80
Leu Gln Ile Ser Asn Leu Lys Asn Glu Asp Thr Ala Thr Tyr Phe Cys
                85                  90                  95
Ala Arg Leu Gly Leu Leu Gly Tyr Trp Gly Gln Gly Thr Thr Leu Thr
            100                 105                 110
Val Ser Ser
            115

<210>   36

<211> 120
<212> PRT
<213> Artificial Sequence

<220>
<221> UNSURE
<222> (1)..(120)
<223> VH

<400> 36
Gln Val Gln Leu Gln Gln Pro Gly Ala Glu Leu Val Arg Pro Gly Ala
1               5                   10                  15
Ser Val Lys Leu Ser Cys Glu Ala Ser Gly Tyr Thr Phe Thr Ser Tyr
            20                  25                  30
Trp Met Asn Trp Val Lys Gln Arg Pro Gly Gln Gly Leu Glu Trp Ile
        35                  40                  45
Gly Met Ile Asp Pro Ser Asp Ser Glu Thr His Tyr Asn Gln Met Phe
    50                  55                  60
Lys Asp Lys Ala Thr Leu Thr Val Asp Lys Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Leu Gly Gly Lys Glu Gly Ser Phe Asp Tyr Trp Gly Gln
            100                 105                 110
Gly Thr Thr Leu Thr Val Ser Ser
        115                 120

<210> 37
<211> 134
<212> PRT
<213> Artificial Sequence

<220>
<221> UNSURE
<222> (1)..(134)
<223> VH

<400> 37
Met Asp Trp Leu Trp Thr Leu Leu Phe Leu Met Ala Ala Ala Gln Ser
1               5                   10                  15
Ile Gln Ala Gln Ile Gln Leu Val Gln Ser Gly Pro Glu Leu Lys Lys
            20                  25                  30
Pro Gly Glu Thr Val Arg Ile Ser Cys Lys Ala Ser Gly Tyr Thr Leu
        35                  40                  45
Thr Thr Ala Gly Met Gln Trp Val Gln Lys Ile Pro Gly Lys Gly Leu
    50                  55                  60
Lys Trp Ile Gly Trp Ile Asn Thr His Ser Gly Val Ala Glu Phe Ala
65                  70                  75                  80
Glu Asp Phe Lys Gly Arg Phe Ala Phe Ser Leu Glu Thr Ser Ala Asn
                85                  90                  95
Thr Ala Tyr Leu Gln Ile Arg Asn Leu Lys Asn Glu Asp Thr Ala Thr
            100                 105                 110
Tyr Phe Cys Ala Arg Leu Gly Leu Leu Gly Tyr Trp Gly Gln Gly Thr
        115                 120                 125
Thr Leu Thr Val Ser Ser
    130

<210> 38
<211> 120
<212> PRT
<213> Artificial Sequence

<220>
<221> UNSURE
<222> (1)..(120)
<223> VH


<400> 38
Gln Val Gln Leu Gln Gln Pro Gly Ala Glu Leu Val Arg Pro Gly Ala
1               5                   10                  15
Ser Val Lys Leu Ser Cys Lys Ser Ser Asp Tyr Thr Phe Thr Arg Tyr
            20                  25                  30
Trp Met Asn Trp Val Lys Gln Arg Pro Gly Gln Gly Leu Glu Trp Ile
        35                  40                  45
Gly Met Ile Asp Pro Ser Asp Ser Glu Thr His His Asn Gln Met Phe
    50                  55                  60
Lys Asp Lys Ala Thr Leu Thr Val Asp Lys Ser Ser Ser Thr Ala Tyr
65                  70                  75                  80
Met Gln Leu Ser Ser Leu Thr Ser Glu Asp Ser Ala Val Tyr Phe Cys
                85                  90                  95
Ala Arg Tyr Leu Gly Gly Lys Glu Gly Ser Phe Asp Tyr Trp Gly Gln
            100                 105                 110
Gly Thr Thr Leu Thr Val Ser Ser
            115                 120


<210> 39
<211> 114
<212> PRT
<213> Artificial Sequence


<220>
<221> UNSURE
<222> (1)..(114)
<223> VH


<400> 39
Glu Val Gln Leu Gln Gln Ser Gly Ala Glu Leu Val Arg Ser Gly Ala
1               5                   10                  15
Ser Val Lys Leu Ser Cys Thr Ala Ser Gly Phe Asn Ile Lys Asp Tyr
            20                  25                  30
Tyr Met His Trp Met Lys Gln Arg Pro Glu Gln Gly Leu Glu Trp Ile
        35                  40                  45
Gly Trp Ile Asp Pro Glu Asn Gly Asp Asn Glu Tyr Ala Pro Lys Phe
    50                  55                  60
Gln Gly Lys Ala Thr Met Thr Ala Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Asn Val Gly Gly Ala His Tyr Trp Gly Gln Gly Thr Thr Leu Thr Val
            100                 105                 110
Ser Ser


<210> 40
<211> 122
<212> PRT
<213> Artificial Sequence


<220>
<221> UNSURE
<222> (1)..(122)
<223> VH

<400> 40

```
Gln Val Gln Leu Lys Glu Ser Gly Pro Gly Leu Val Ala Pro Ser Gln
1               5                   10                  15
Ser Leu Ser Ile Thr Cys Thr Val Ser Gly Phe Ser Leu Thr Ser Tyr
            20                  25                  30
Ala Val Ser Trp Val Arg Gln Pro Pro Gly Lys Gly Leu Glu Trp Leu
        35                  40                  45
Gly Ile Ile Trp Gly Asp Gly Ser Thr Asn Tyr His Ser Ala Leu Ile
    50                  55                  60
Ser Arg Leu Ser Ile Ser Lys Asp Asn Ser Lys Ser Gln Val Phe Leu
65                  70                  75                  80
Lys Leu Asn Ser Leu Gln Thr Asp Asp Thr Ala Thr Tyr Tyr Cys Ala
                85                  90                  95
Lys Gln Glu Arg Phe Ser Asp Gly Tyr Tyr Asp Gly Phe Ala Tyr Trp
            100                 105                 110
Gly Gln Gly Thr Leu Val Thr Val Ser Ala
            115                 120
```

<210> 41
<211> 122
<212> PRT
<213> Artificial Sequence

<220>
<221> UNSURE
<222> (1)..(122)
<223> VH


<400> 41

```
Gln Val Gln Leu Lys Glu Ser Gly Pro Gly Leu Val Ala Pro Ser Gln
1               5                   10                  15
Ser Leu Ser Ile Thr Cys Thr Val Ser Gly Phe Ser Leu Thr Arg Tyr
            20                  25                  30
Gly Val Ser Trp Val Arg Gln Thr Pro Gly Lys Gly Leu Glu Trp Leu
        35                  40                  45
Gly Ile Ile Trp Gly Asp Gly Ser Thr Asn Tyr His Ser Ala Leu Ile
    50                  55                  60
Ser Arg Leu Ser Ile Ser Lys Asp Asn Ser Lys Ser Gln Val Phe Leu
65                  70                  75                  80
Lys Leu Asn Ser Leu Gln Thr Asp Asp Thr Ala Thr Tyr Tyr Cys Ala
                85                  90                  95
Lys Gln Glu Arg Phe Ser Asp Gly Tyr Tyr Asp Gly Phe Ala Tyr Trp
            100                 105                 110
Gly Gln Gly Thr Leu Val Thr Val Ser Ala
            115                 120
```

<210> 42
<211> 109
<212> PRT
<213> Artificial Sequence

<220>
<221> UNSURE
<222> (1)..(109)
<223> VL


<400> 42

```
Asp Ile Leu Leu Thr Gln Ser Pro Ala Ile Leu Ser Val Ser Pro Gly
1               5                   10                  15
```

```
        Glu Arg Val Ser Phe Ser Cys Arg Ala Ser Gln Ser Ile Gly Thr Thr
                20                  25                  30
        Ile His Trp Tyr Gln Gln Arg Thr Asn Gly Ser Pro Arg Leu Leu Ile
                35                  40                  45
        Lys Tyr Ala Ser Glu Ser Ile Ser Gly Ile Pro Ser Arg Phe Ser Gly
                50                  55                  60
        Ser Gly Ser Gly Thr Asp Phe Thr Leu Ser Ile Asn Ser Val Glu Ser
        65                  70                  75                  80
        Glu Asp Ile Ala Asp Tyr Tyr Cys Gln Gln Ser Asn Ser Trp Pro Trp
                        85                  90                  95
        Thr Phe Gly Gly Gly Thr Lys Leu Glu Ile Lys Arg Ala
                        100                 105


        <210>   43
        <211>   115
        <212>   PRT
        <213>   Artificial Sequence


        <220>
        <221>   UNSURE
        <222>   (1)..(115)
        <223>   VL


        <400>   43
        Asp Ile Val Met Ser Gln Ser Pro Ser Ser Leu Ala Val Ser Ala Gly
        1               5                   10                  15
        Glu Lys Val Thr Met Ser Cys Lys Ser Ser Gln Ser Leu Leu Asn Ser
                20                  25                  30
        Arg Thr Arg Lys Asn Tyr Leu Ala Trp Tyr Gln Gln Lys Pro Gly Gln
                35                  40                  45
        Ser Pro Lys Leu Leu Ile Tyr Trp Ala Ser Thr Arg Glu Ser Gly Val
                50                  55                  60
        Pro Asp Arg Phe Thr Gly Ser Gly Ser Gly Thr Asp Phe Thr Leu Thr
        65                  70                  75                  80
        Ile Ser Ser Val Gln Ala Glu Asp Leu Ala Val Tyr Tyr Cys Lys Gln
                        85                  90                  95
        Ser Tyr Ser Leu Pro Trp Thr Phe Gly Gly Gly Thr Lys Leu Glu Ile
                        100                 105                 110
        Lys Arg Ala
                115


        <210>   44
        <211>   129
        <212>   PRT
        <213>   Artificial Sequence


        <220>
        <221>   UNSURE
        <222>   (1)..(129)
        <223>   VL


        <400>   44
        Met Val Ser Ser Ala Gln Phe Leu Val Phe Leu Leu Phe Trp Ile Pro
        1               5                   10                  15
        Ala Ser Arg Gly Asp Ile Leu Leu Thr Gln Ser Pro Ala Ile Leu Ser
                20                  25                  30
        Val Ser Pro Gly Glu Arg Val Ser Phe Ser Cys Arg Ala Ser Gln Ser
                35                  40                  45
        Ile Gly Thr Thr Ile His Trp Tyr Gln Gln Arg Thr Asn Gly Ser Pro
                50                  55                  60
        Arg Leu Leu Ile Lys Tyr Ala Ser Glu Ser Ile Ser Gly Ile Pro Ser
```

```
            65                      70                      75                      80
Arg Phe Ser Gly Ser Gly Ser Gly Thr Asp Phe Thr Leu Ser Ile Asn
                        85                      90                      95
Ser Val Glu Ser Glu Asp Ile Ala Asp Tyr Tyr Cys Gln Gln Ser Asn
                        100                     105                     110
Ser Trp Pro Trp Thr Phe Gly Gly Gly Thr Lys Leu Glu Ile Lys Arg
            115                     120                     125
Ala


<210>  45
<211>  115
<212>  PRT
<213>  Artificial Sequence

<220>
<221>  UNSURE
<222>  (1)..(115)
<223>  VL


<400>  45
Asp Ile Val Leu Thr Gln Ser Pro Ser Ser Leu Ala Val Ser Ala Gly
1                   5                       10                      15
Glu Arg Val Thr Met Ser Cys Lys Ser Ser Gln Ser Leu Phe Asn Ser
                20                      25                      30
Arg Thr Arg Lys Asn Tyr Leu Ala Trp Tyr Gln Gln Lys Pro Gly Gln
            35                      40                      45
Ser Pro Lys Leu Leu Leu Tyr Trp Ala Ser Thr Arg Glu Ser Gly Val
        50                      55                      60
Pro Asp Arg Phe Thr Gly Ser Gly Ser Gly Thr Asp Phe Thr Leu Thr
65                      70                      75                      80
Ile Ser Ser Val Lys Thr Glu Asp Leu Ala Val Tyr Tyr Cys Lys Gln
                85                      90                      95
Ser Tyr Glu Leu Pro Trp Thr Phe Gly Gly Gly Thr Lys Leu Glu Met
                100                     105                     110
Lys Arg Ala
            115

<210>  46
<211>  114
<212>  PRT
<213>  Artificial Sequence

<220>
<221>  UNSURE
<222>  (1)..(114)
<223>  VL


<400>  46
Asp Val Val Met Thr Gln Thr Pro Leu Ser Leu Pro Val Ser Leu Gly
1                   5                       10                      15
Asp Gln Ala Ser Ile Ser Cys Arg Ser Ser Gln Ser Leu Val His Ser
                20                      25                      30
Asn Gly Asn Thr Tyr Leu His Trp Tyr Leu Gln Lys Pro Gly Gln Ser
            35                      40                      45
Pro Lys Leu Leu Ile Tyr Lys Val Ser Asn Arg Phe Ser Gly Val Pro
        50                      55                      60
Asp Arg Phe Ser Gly Ser Gly Ser Gly Thr Asp Phe Thr Leu Lys Ile
65                      70                      75                      80
Ser Arg Val Glu Ala Glu Asp Leu Gly Val Tyr Phe Cys Ser Gln Ser
                85                      90                      95
```

```
Thr His Val Pro Tyr Thr Phe Gly Gly Gly Thr Lys Leu Glu Ile Lys
            100             105             110
Arg Ala
```

<210> 47
<211> 108
<212> PRT
<213> Artificial Sequence

<220>
<221> UNSURE
<222> (1)..(108)
<223> VL

<400> 47
```
Gln Ile Val Leu Thr Gln Ser Pro Ala Ile Met Ser Ala Ser Pro Gly
1               5               10              15
Glu Lys Val Thr Ile Thr Cys Ser Ala Ser Ser Ser Val Ser Tyr Met
            20              25              30
His Trp Phe Gln Gln Lys Pro Gly Thr Ser Pro Lys Leu Trp Ile Tyr
            35              40              45
Ser Thr Ser Asn Leu Ala Ser Gly Val Pro Ala Arg Phe Ser Gly Ser
            50              55              60
Gly Ser Gly Thr Ser Tyr Ser Leu Thr Ile Ser Arg Met Glu Ala Glu
65                  70              75                  80
Asp Ala Ala Thr Tyr Tyr Cys Gln Gln Arg Ser Ser Tyr Pro Phe Thr
                85              90              95
Phe Gly Ser Gly Thr Lys Leu Glu Ile Lys Arg Ala
            100             105
```

<210> 48
<211> 120
<212> PRT
<213> Artificial Sequence

<220>
<221> UNSURE
<222> (1)..(120)
<223> VH

<400> 48
```
Gln Val Gln Leu Val Gln Ser Gly Ala Glu Val Lys Lys Pro Gly Ala
1               5               10              15
Ser Val Lys Val Ser Cys Lys Ala Ser Gly Tyr Thr Phe Thr Ser Tyr
            20              25              30
Trp Met Asn Trp Val Arg Gln Ala Pro Gly Lys Gly Leu Glu Trp Val
            35              40              45
Gly Met Ile Asp Pro Ser Asp Ser Glu Thr His Tyr Asn Gln Met Phe
            50              55              60
Lys Asp Arg Val Thr Ile Thr Ala Asp Lys Ser Thr Ser Thr Ala Tyr
65                  70              75                  80
Met Glu Leu Ser Ser Leu Arg Ser Glu Asp Thr Ala Val Tyr Tyr Cys
                85              90              95
Ala Arg Tyr Leu Gly Gly Lys Glu Gly Ser Phe Asp Tyr Trp Gly Gln
            100             105             110
Gly Thr Leu Val Thr Val Ser Ser
            115             120
```

<210> 49
<211> 120

```
<212>   PRT
<213>   Artificial Sequence

<220>
<221>   UNSURE
<222>   (1)..(120)
<223>   VH


<400>   49
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met Asn Trp Val Arg Gln Ala Pro Gly Lys Gly Leu Glu Trp Val
        35                  40                  45
Gly Met Ile Asp Pro Ser Asp Ser Glu Thr His Tyr Asn Gln Met Phe
    50                  55                  60
Lys Asp Arg Val Thr Ile Thr Val Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Tyr Leu Gly Gly Lys Glu Gly Ser Phe Asp Tyr Trp Gly Gln
            100                 105                 110
Gly Thr Leu Val Thr Val Ser Ser
            115                 120


<210>   50
<211>   120
<212>   PRT
<213>   Artificial Sequence

<220>
<221>   UNSURE
<222>   (1)..(120)
<223>   VH


<400>   50
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met Asn Trp Val Arg Gln Ala Pro Gly Gln Gly Leu Glu Trp Val
        35                  40                  45
Gly Met Ile Asp Pro Ser Asp Ser Glu Thr His Tyr Ala Gln Lys Phe
    50                  55                  60
Gln Gly Arg Val Thr Ile Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Tyr Leu Gly Gly Lys Glu Gly Ser Phe Asp Tyr Trp Gly Gln
            100                 105                 110
Gly Thr Leu Val Thr Val Ser Ser
            115                 120


<210>   51
<211>   120
<212>   PRT
<213>   Artificial Sequence

<220>
<221>   UNSURE
```

<222> (1)..(120)
<223> VH

<400> 51

| Gln | Val | Gln | Leu | Val | Gln | Ser | Gly | Ala | Glu | Val | Lys | Lys | Pro | Gly | Ala |
|---|---|---|---|---|---|---|---|---|---|---|---|---|---|---|---|
| 1 | | | | 5 | | | | | 10 | | | | | 15 | |

Ser Val Lys Val Ser Cys Lys Ala Ser Gly Tyr Thr Phe Thr Ser Tyr
20 25 30

Trp Met Asn Trp Val Arg Gln Ala Pro Gly Gln Gly Leu Glu Trp Val
35 40 45

Gly Met Ile Asp Pro Ser Asp Ser Glu Thr His Tyr Ala Gln Lys Phe
50 55 60

Gln Gly Arg Val Thr Ile Thr Ala Asp Lys Ser Thr Ser Thr Val Tyr
65 70 75 80

Met Glu Leu Ser Ser Leu Arg Ser Glu Asp Thr Ala Val Tyr Tyr Cys
85 90 95

Ala Arg Tyr Leu Gly Gly Lys Glu Gly Ser Phe Asp Tyr Trp Gly Gln
100 105 110

Gly Thr Leu Val Thr Val Ser Ser
115 120

<210> 52
<211> 113
<212> PRT
<213> Artificial Sequence

<220>
<221> UNSURE
<222> (1)..(113)
<223> VL

<400> 52

Asp Ile Val Met Thr Gln Ser Pro Asp Ser Leu Ala Val Ser Leu Gly
1 5 10 15

Glu Arg Ala Thr Ile Asn Cys Lys Ser Ser Gln Ser Leu Leu Asn Ser
20 25 30

Arg Thr Arg Lys Asn Tyr Leu Ala Trp Tyr Gln Gln Lys Pro Gly Gln
35 40 45

Pro Pro Lys Leu Leu Ile Tyr Trp Ala Ser Thr Arg Glu Ser Gly Val
50 55 60

Pro Asp Arg Phe Ser Gly Ser Gly Ser Gly Thr Asp Phe Thr Leu Thr
65 70 75 80

Ile Ser Ser Leu Gln Ala Glu Asp Val Ala Val Tyr Tyr Cys Lys Gln
85 90 95

Ser Tyr Ser Leu Pro Trp Thr Phe Gly Gly Gly Thr Lys Val Glu Ile
100 105 110

Lys

<210> 53
<211> 113
<212> PRT
<213> Artificial Sequence

<220>
<221> UNSURE
<222> (1)..(113)
<223> VL

<400> 53

Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Leu Asn Ser
            20                  25                  30
Arg Thr Arg Lys Asn Tyr Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Ser Tyr Ser Leu Pro Trp Thr Phe Gly Gly Gly Thr Lys Val Glu Ile
                100                 105                 110
Lys

<210> 54
<211> 124
<212> PRT
<213> Artificial Sequence

<220>
<221> UNSURE
<222> (1)..(124)
<223> VH

<400> 54
Gln Val Gln Leu Leu Glu Ser Gly Gly Gly Leu Val Gln Pro Gly Gly
1               5                   10                  15
Ser Leu Arg Leu Ser Cys Ser Ala Ser Gly Phe Thr Phe Ser Ser Tyr
            20                  25                  30
Ala Met His Trp Val Arg Gln Ala Pro Gly Lys Gly Leu Glu Tyr Val
        35                  40                  45
Ser Ala Ile Ser Ser Asn Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Ser Ser Leu Arg Ala Glu Asp Thr Ala Val Tyr Tyr Cys
                85                  90                  95
Val Ile Glu Gly Gly Ser Thr Thr Gly Thr Thr Ser Gly Ala Phe Asp
                100                 105                 110
Ile Trp Gly Gln Gly Thr Met Val Thr Val Ser Ser
        115                 120

<210> 55
<211> 123
<212> PRT
<213> Artificial Sequence

<220>
<221> UNSURE
<222> (1)..(123)
<223> VH

<400> 55
Gln Ile Thr Leu Lys Glu Ser Gly Pro Thr Leu Val Lys Pro Thr Gln
1               5                   10                  15
Thr Leu Thr Leu Thr Cys Asn Phe Ser Gly Phe Ser Leu Thr Thr Ser
            20                  25                  30
Gly Val Gly Val Ala Trp Ile Arg Gln Pro Pro Gly Lys Ala Leu Glu

45

```
                35                    40                    45
Trp Leu Ala Leu Ile Tyr Trp Asp Asp Asp Gln Arg Tyr Ser Pro Ser
    50                    55                    60
Leu Lys Ser Arg Leu Ser Val Thr Lys His Thr Ser Lys Asp Gln Val
65                    70                    75                    80
Val Leu Thr Met Thr Asn Val Gly Pro Val Asp Thr Ala Thr Tyr Tyr
                85                    90                    95
Cys Ala His Leu Ser Tyr Gly Pro Gly Trp Gly Tyr Tyr Met Asp Val
                100                   105                   110
Trp Gly Asn Gly Thr Met Val Thr Val Ser Ser
                115                   120
```

```
<210>  56
<211>  118
<212>  PRT
<213>  Artificial Sequence

<220>
<221>  UNSURE
<222>  (1)..(118)
<223>  VH


<400>  56
Gln Val Gln Leu Leu Glu Ser Gly Gly Gly Val Val Gln Pro Gly Arg
1               5                     10                    15
Ser Leu Arg Leu Ser Cys Ala Ala Ser Gly Phe Thr Phe Ser Ser Tyr
                20                    25                    30
Ala Met His Trp Val Arg Gln Ala Pro Gly Lys Gly Leu Glu Trp Val
        35                    40                    45
Ala Val Ile Ser Tyr Asp Gly Ser Asn Lys Tyr Tyr Ala Asp Ser Val
    50                    55                    60
Lys Gly Arg Phe Thr Ile Ser Arg Asp Asn Ser Lys Asn Thr Leu Tyr
65                    70                    75                    80
Leu Gln Met Asn Ser Leu Arg Ala Glu Asp Thr Ala Val Tyr Tyr Cys
                85                    90                    95
Ala Arg Met Asp Gly Ser Gly Ser Pro Asp Tyr Trp Gly Gln Gly Thr
                100                   105                   110
Leu Val Thr Val Ser Ser
                115
```

```
<210>  57
<211>  107
<212>  PRT
<213>  Artificial Sequence

<220>
<221>  UNSURE
<222>  (1)..(107)
<223>  VL


<400>  57
Asp Ile Gln Met Ile Gln Ser Pro Pro Ser Val Ser Ala Ser Val Gly
1               5                     10                    15
Asp Thr Val Thr Ile Ala Cys Arg Ala Asn Gln Gly Ile Asp Ser Trp
                20                    25                    30
Leu Ala Trp Tyr Gln Gln Lys Pro Gly Arg Ala Pro Lys Leu Leu Ile
        35                    40                    45
Tyr Ser Ala Ser Arg Leu Gln Ser Gly Val Pro Ser Arg Phe Ser Gly
        50                    55                    60
Gly Gly Ser Gly Thr Asp Phe Ala Leu Thr Ile Ser Asn Leu Gln Pro
65                    70                    75                    80
```

46

```
Glu Asp Phe Ala Thr Tyr Tyr Cys Gln Gln Ala Leu Ser Leu Pro Ile
                85                  90                  95
Thr Phe Gly Gln Gly Thr Arg Leu Glu Ile Lys
            100                 105


<210>   58
<211>   108
<212>   PRT
<213>   Artificial Sequence


<220>
<221>   UNSURE
<222>   (1)..(108)
<223>   VL


<400>   58
Glu Ile Val Leu Thr Gln Ser Pro Gly Thr Leu Ser Leu Ser Pro Gly
1               5                   10                  15
Glu Arg Ala Ser Leu Ser Cys Arg Ala Ser Gln Ser Val Arg Asn Asn
                20                  25                  30
Tyr Leu Ala Trp Tyr Gln Gln Lys Pro Gly Gln Ala Pro Arg Leu Leu
            35                  40                  45
Ile Phe Gly Ala Ser Asn Arg Ala Thr Gly Ile Pro Asp Thr Phe Ser
        50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly His Ser Pro
                85                  90                  95
Ile Thr Phe Gly Gln Gly Thr Arg Leu Glu Ile Lys
            100                 105


<210>   59
<211>   112
<212>   PRT
<213>   Artificial Sequence


<220>
<221>   UNSURE
<222>   (1)..(112)
<223>   VL


<400>   59
Asp Ile Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
                20                  25                  30
Asp Gly Asn Thr Tyr Leu Asn Trp Phe Gln Gln Arg Pro Gly Gln Ser
            35                  40                  45
Pro Arg Arg Leu Ile Tyr Lys Val Ser Asn Arg Asp Ser Gly Val Pro
        50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Pro Ala Phe Gly Gln Gly Thr Lys Val Glu Ile Lys
            100                 105                 110


<210>   60
<211>   8
<212>   PRT
<213>   Artificial Sequence
```

<220>
<221> UNSURE
<222> (1)..(8)
<223> VH CDR1


<400> 60
Gly Phe Thr Phe Ser Ser Tyr Ala
1               5


<210> 61
<211> 10
<212> PRT
<213> Artificial Sequence


<220>
<221> UNSURE
<222> (1)..(10)
<223> VH CDR1


<400> 61
Gly Phe Ser Leu Thr Thr Ser Gly Val Gly
1               5                   10


<210> 62
<211> 8
<212> PRT
<213> Artificial Sequence


<220>
<221> UNSURE
<222> (1)..(8)
<223> VH CDR2


<400> 62
Ile Ser Ser Asn Gly Gly Ser Thr
1               5


<210> 63
<211> 7
<212> PRT
<213> Artificial Sequence


<220>
<221> UNSURE
<222> (1)..(7)
<223> VH CDR2


<400> 63
Ile Tyr Trp Asp Asp Asp Gln
1               5


<210> 64
<211> 8
<212> PRT
<213> Artificial Sequence


<220>
<221> UNSURE

<222> (1)..(8)
<223> VH CDR2


<400> 64
Ile Ser Tyr Asp Gly Ser Asn Lys
1               5


<210> 65
<211> 16
<212> PRT
<213> Artificial Sequence

<220>
<221> UNSURE
<222> (1)..(16)
<223> VH CDR3


<400> 65
Val Ile Glu Gly Gly Ser Thr Thr Gly Thr Thr Ser Gly Ala Phe Asp
1               5                   10                  15


<210> 66
<211> 15
<212> PRT
<213> Artificial Sequence

<220>
<221> UNSURE
<222> (1)..(15)
<223> VH CDR3


<400> 66
Ala His Leu Ser Tyr Gly Pro Gly Trp Gly Tyr Tyr Met Asp Val
1               5                   10                  15


<210> 67
<211> 11
<212> PRT
<213> Artificial Sequence

<220>
<221> UNSURE
<222> (1)..(11)
<223> VH CDR3


<400> 67
Ala Arg Met Asp Gly Ser Gly Ser Pro Asp Tyr
1               5                   10


<210> 68
<211> 6
<212> PRT
<213> Artificial Sequence

<220>
<221> UNSURE
<222> (1)..(6)
<223> VL CDR1

```
<400>  68
Gln Gly Ile Asp Ser Trp
1               5


<210>  69
<211>  8
<212>  PRT
<213>  Artificial Sequence

<220>
<221>  UNSURE
<222>  (1)..(8)
<223>  VL CDR1


<400>  69
Ser Gln Ser Val Arg Asn Asn Tyr
1               5


<210>  70
<211>  11
<212>  PRT
<213>  Artificial Sequence

<220>
<221>  UNSURE
<222>  (1)..(11)
<223>  VL CDR1


<400>  70
Gln Ser Leu Val Tyr Ser Asp Gly Asn Thr Tyr
1               5                   10


<210>  71
<211>  2
<212>  PRT
<213>  Artificial Sequence

<220>
<221>  UNSURE
<222>  (1)..(2)
<223>  VL CDR2


<400>  71
Ser Ala
1


<210>  72
<211>  2
<212>  PRT
<213>  Artificial Sequence

<220>
<221>  UNSURE
<222>  (1)..(2)
<223>  VL CDR2


<400>  72
Gly Ala
```

<210> 73
<211> 9
<212> PRT
<213> Artificial Sequence

<220>
<221> UNSURE
<222> (1)..(9)
<223> VL CDR3

<400> 73
Gln Gln Ala Leu Ser Leu Pro Ile Thr
1               5

<210> 74
<211> 9
<212> PRT
<213> Artificial Sequence

<220>
<221> UNSURE
<222> (1)..(9)
<223> VL CDR3

<400> 74
Gln Gln Tyr Gly His Ser Pro Ile Thr
1               5

<210> 75
<211> 9
<212> PRT
<213> Artificial Sequence

<220>
<221> UNSURE
<222> (1)..(9)
<223> VL CDR3

<400> 75
Met Gln Gly Thr His Trp Pro Pro Ala
1               5

<210> 76
<211> 8
<212> PRT
<213> Artificial Sequence

<220>
<221> UNSURE
<222> (1)..(8)
<223> VH CDR1

<400> 76
Gly Phe Thr Phe Ser Ser Arg Ala
1               5

<210> 77

```
<211>  11
<212>  PRT
<213>  Artificial Sequence

<220>
<221>  UNSURE
<222>  (1)..(11)
<223>  VH CDR3


<400>  77
Ala Arg Met Asp Asn His Gly Ser Pro Asp Tyr
1               5                   10


<210>  78
<211>  11
<212>  PRT
<213>  Artificial Sequence

<220>
<221>  UNSURE
<222>  (1)..(11)
<223>  VH CDR3


<400>  78
Ala Arg Met Asp Gly His Gly Ser Pro Asp Tyr
1               5                   10


<210>  79
<211>  11
<212>  PRT
<213>  Artificial Sequence

<220>
<221>  UNSURE
<222>  (1)..(11)
<223>  VH CDR3


<400>  79
Ala Arg Met Asp Ser Gly Gly Ser Pro Asp Tyr
1               5                   10


<210>  80
<211>  17
<212>  PRT
<213>  Artificial Sequence

<220>
<221>  UNSURE
<222>  (1)..(17)
<223>  VH CDR3


<400>  80
Val Ile Glu Gly Gly Ser Thr Gly Ser Thr Thr Ser Gly Ala Phe Asp
1               5                   10                  15
Ile


<210>  81
<211>  17
```

```
<212>  PRT
<213>  Artificial Sequence

<220>
<221>  UNSURE
<222>  (1)..(17)
<223>  VH CDR3


<400>  81
Val Ile Glu Gly Gly Ser Thr Gly Ser Thr Val Ser Gly Ala Phe Asp
1               5                   10                  15
Ile


<210>  82
<211>  11
<212>  PRT
<213>  Artificial Sequence

<220>
<221>  UNSURE
<222>  (1)..(11)
<223>  VL CDR1


<400>  82
Gln Ser Leu Val Tyr Lys Asp Gly Asn Thr Tyr
1               5                   10


<210>  83
<211>  2
<212>  PRT
<213>  Artificial Sequence

<220>
<221>  UNSURE
<222>  (1)..(2)
<223>  VL CDR2


<400>  83
Glu Val
1


<210>  84
<211>  2
<212>  PRT
<213>  Artificial Sequence

<220>
<221>  UNSURE
<222>  (1)..(2)
<223>  VL CDR2


<400>  84
Gly Val
1


<210>  85
<211>  9
<212>  PRT
```

```
<213>  Artificial Sequence

<220>
<221>  UNSURE
<222>  (1)..(9)
<223>  VL CDR3


<400>  85
Met Gln Gly Thr His Trp Pro Pro Arg
1                   5

<210>  86
<211>  13
<212>  PRT
<213>  Artificial Sequence

<220>
<221>  UNSURE
<222>  (1)..(13)
<223>  Peptide


<220>
<221>  UNSURE
<222>  (11)..(11)
<223>  Arbitrary amino acid


<220>
<221>  UNSURE
<222>  (13)..(13)
<223>  Arbitrary amino acid


<400>  86
Gln Ile Glu Lys Leu Val Glu Gly Lys Ser Xaa Ile Xaa
1                   5                   10

<210>  87
<211>  12
<212>  PRT
<213>  Artificial Sequence

<220>
<221>  UNSURE
<222>  (1)..(12)
<223>  Peptide


<220>
<221>  UNSURE
<222>  (11)..(11)
<223>  Arbitrary amino acid


<400>  87
Gln Ile Glu Lys Leu Val Glu Gly Lys Ser Xaa Ile
1                   5                   10

<210>  88
<211>  11
<212>  PRT
```

```
<213>  Artificial Sequence

<220>
<221>  UNSURE
<222>  (1)..(11)
<223>  Peptide


<220>
<221>  UNSURE
<222>  (11)..(11)
<223>  Arbitrary amino acid


<400>  88
Gln Ile Glu Lys Leu Val Glu Gly Lys Ser Xaa
1               5                   10


<210>  89
<211>  79
<212>  PRT
<213>  Artificial Sequence

<220>
<221>  UNSURE
<222>  (1)..(79)
<223>  SEMG2-P1


<400>  89
Gly Ser Phe Ser Ile Gln His Thr Tyr His Val Asp Ile Asn Asp His
1               5                   10                  15
Asp Trp Thr Arg Lys Ser Gln Gln Tyr Asp Leu Asn Ala Leu His Lys
            20                  25                  30
Ala Thr Lys Ser Lys Gln His Leu Gly Gly Ser Gln Gln Leu Leu Asn
            35                  40                  45
Tyr Lys Gln Glu Gly Arg Asp His Asp Lys Ser Lys Gly His Phe His
        50                  55                  60
Met Ile Val Ile His His Lys Gly Gly Gln Ala His His Gly Thr
65                  70                  75


<210>  90
<211>  142
<212>  PRT
<213>  Artificial Sequence

<220>
<221>  UNSURE
<222>  (1)..(142)
<223>  SEMG2-P2


<400>  90
Gln Asn Pro Ser Gln Asp Gln Gly Asn Ser Pro Ser Gly Lys Gly Leu
1               5                   10                  15
Ser Ser Gln Cys Ser Asn Thr Glu Lys Arg Leu Trp Val His Gly Leu
            20                  25                  30
Ser Lys Glu Gln Ala Ser Ala Ser Gly Ala Gln Lys Gly Arg Thr Gln
            35                  40                  45
Gly Gly Ser Gln Ser Ser Tyr Val Leu Gln Thr Glu Glu Leu Val Val
        50                  55                  60
Asn Lys Gln Gln Arg Glu Thr Lys Asn Ser His Gln Asn Lys Gly His
65                  70                  75                  80
```

```
        Tyr Gln Asn Val Val Asp Val Arg Glu Glu His Ser Ser Lys Leu Gln
                        85                  90                  95
        Thr Ser Leu His Pro Ala His Gln Asp Arg Leu Gln His Gly Pro Lys
                    100                 105                 110
        Asp Ile Phe Thr Thr Gln Asp Glu Leu Leu Val Tyr Asn Lys Asn Gln
                    115                 120                 125
        His Gln Thr Lys Asn Leu Ser Gln Asp Gln Glu His Gly Arg
            130                 135                 140


        <210>   91
        <211>   116
        <212>   PRT
        <213>   Artificial Sequence

        <220>
        <221>   UNSURE
        <222>   (1)..(116)
        <223>   SEMG2-P3


        <400>   91
        Lys Ala His Lys Ile Ser Tyr Pro Ser Ser Arg Thr Glu Glu Arg Gln
        1                   5                   10                  15
        Leu His His Gly Glu Lys Ser Val Gln Lys Asp Val Ser Lys Gly Ser
                        20                  25                  30
        Ile Ser Ile Gln Thr Glu Glu Lys Ile His Gly Lys Ser Gln Asn Gln
                    35                  40                  45
        Val Thr Ile His Ser Gln Asp Gln Glu His Gly His Lys Glu Asn Lys
                50                  55                  60
        Ile Ser Tyr Gln Ser Ser Ser Thr Glu Glu Arg His Leu Asn Cys Gly
        65                  70                  75                  80
        Glu Lys Gly Ile Gln Lys Gly Val Ser Lys Gly Ser Ile Ser Ile Gln
                        85                  90                  95
        Thr Glu Glu Gln Ile His Gly Lys Ser Gln Asn Gln Val Arg Ile Pro
                    100                 105                 110
        Ser Gln Ala Gln
                115


        <210>   92
        <211>   98
        <212>   PRT
        <213>   Artificial Sequence

        <220>
        <221>   UNSURE
        <222>   (1)..(98)
        <223>   SEMG2-P4


        <400>   92
        Glu Tyr Gly His Lys Glu Asn Lys Ile Ser Tyr Gln Ser Ser Ser Thr
        1                   5                   10                  15
        Glu Glu Arg Arg Leu Asn Ser Gly Glu Lys Asp Val Gln Lys Gly Val
                        20                  25                  30
        Ser Lys Gly Ser Ile Ser Ile Gln Thr Glu Glu Lys Ile His Gly Lys
                    35                  40                  45
        Ser Gln Asn Gln Val Thr Ile Pro Ser Gln Asp Gln Glu His Gly His
                50                  55                  60
        Lys Glu Asn Lys Met Ser Tyr Gln Ser Ser Ser Thr Glu Glu Arg Arg
        65                  70                  75                  80
        Leu Asn Tyr Gly Gly Lys Ser Thr Gln Lys Asp Val Ser Gln Ser Ser
                        85                  90                  95
        Ile Ser
```

```
<210>    93
<211>    45
<212>    PRT
<213>    Artificial Sequence

<220>
<221>    UNSURE
<222>    (1)..(45)
<223>    SEMG2-P5


<400>    93
Phe Gln Ile Glu Lys Leu Val Glu Gly Lys Ser Gln Ile Gln Thr Pro
1               5                   10                  15
Asn Pro Asn Gln Asp Gln Trp Ser Gly Gln Asn Ala Lys Gly Lys Ser
            20                  25                  30
Gly Gln Ser Ala Asp Ser Lys Gln Asp Leu Leu Ser His
        35                  40                  45


<210>    94
<211>    42
<212>    PRT
<213>    Artificial Sequence

<220>
<221>    UNSURE
<222>    (1)..(42)
<223>    SEMG2-P6


<400>    94
Glu Gln Lys Gly Arg Tyr Lys Gln Glu Ser Ser Glu Ser His Asn Ile
1               5                   10                  15
Val Ile Thr Glu His Glu Val Ala Gln Asp Asp His Leu Thr Gln Gln
            20                  25                  30
Tyr Asn Glu Asp Arg Asn Pro Ile Ser Thr
        35                  40


<210>    95
<211>    9
<212>    PRT
<213>    Artificial Sequence

<220>
<221>    UNSURE
<222>    (1)..(9)
<223>    VL CDR3


<400>    95
Gln Gln Ser Tyr Ser Leu Pro Trp Thr
1               5


<210>    96
<211>    118
<212>    PRT
<213>    Artificial Sequence

<220>
<221>    UNSURE
<222>    (1)..(118)
```

<223>   VH


<400>   96
Gln Val Gln Leu Leu Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Ala Met His Trp Val Arg Gln Ala Ser Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Met Asp Asn His Gly Ser Pro Asp Tyr Trp Gly Gln Gly Thr
            100                 105                 110
Leu Val Thr Val Ser Ser
            115


<210>   97
<211>   118
<212>   PRT
<213>   Artificial Sequence


<220>
<221>   UNSURE
<222>   (1)..(118)
<223>   VH


<400>   97
Gln Val Gln Leu Leu Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Ala Met His Trp Val Arg Gln Ala Ser Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Met Asp Ser Gly Gly Ser Pro Asp Tyr Trp Gly Gln Gly Thr
            100                 105                 110
Leu Val Thr Val Ser Ser
            115

<210>   98
<211>   118
<212>   PRT
<213>   Artificial Sequence


<220>
<221>   UNSURE
<222>   (1)..(118)
<223>   VH


<400>   98
Gln Val Gln Leu Leu Glu Ser Gly Gly Gly Val Val Gln Pro Gly Arg

```
                 1                   5                            10                            15
                 Ser Leu Arg Leu Ser Cys Ala Ala Ser Gly Phe Thr Phe Ser Ser Tyr
                         20                            25                            30
                 Ala Met His Trp Val Arg Gln Ala Ser Gly Lys Gly Leu Glu Trp Val
                         35                            40                            45
                 Ala Val Ile Ser Tyr Asp Gly Ser Asn Lys Tyr Tyr Ala Asp Ser Val
                     50                            55                            60
                 Lys Gly Arg Phe Thr Ile Ser Arg Asp Asn Ser Lys Asn Thr Leu Tyr
                 65                            70                            75                            80
                 Leu Gln Met Asn Ser Leu Arg Ala Glu Asp Thr Ala Val Tyr Tyr Cys
                                 85                            90                            95
                 Ala Arg Met Asp Gly His Gly Ser Pro Asp Tyr Trp Gly Gln Gly Thr
                         100                           105                           110
                 Leu Val Thr Val Ser Ser
                         115


                 <210>   99
                 <211>   124
                 <212>   PRT
                 <213>   Artificial Sequence


                 <220>
                 <221>   UNSURE
                 <222>   (1)..(124)
                 <223>   VH



                 <400>   99
                 Gln Val Gln Leu Leu Glu Ser Gly Gly Gly Leu Val Gln Pro Gly Gly
                 1                   5                            10                            15
                 Ser Leu Arg Leu Ser Cys Ser Ala Ser Gly Phe Thr Phe Ser Ser Arg
                         20                            25                            30
                 Ala Met His Trp Val Arg Gln Ala Pro Gly Lys Gly Leu Glu Tyr Val
                         35                            40                            45
                 Ser Ala Ile Ser Ser Asn Gly Gly Ser Thr Tyr Tyr Ala Asp Ser Val
                     50                            55                            60
                 Lys Gly Arg Phe Thr Ile Ser Arg Asp Asn Ser Lys Asn Thr Leu Tyr
                 65                            70                            75                            80
                 Leu Gln Met Ser Ser Leu Arg Ala Glu Asp Thr Ala Val Tyr Tyr Cys
                                 85                            90                            95
                 Val Ile Glu Gly Gly Ser Thr Gly Ser Thr Thr Ser Gly Ala Phe Asp
                         100                           105                           110
                 Ile Trp Gly Gln Gly Thr Met Val Thr Val Ser Ser
                         115                           120


                 <210>   100
                 <211>   124
                 <212>   PRT
                 <213>   Artificial Sequence


                 <220>
                 <221>   UNSURE
                 <222>   (1)..(124)
                 <223>   VH



                 <400>   100
                 Gln Val Gln Leu Leu Glu Ser Gly Gly Gly Leu Val Gln Pro Gly Gly
                 1                   5                            10                            15
                 Ser Leu Arg Leu Ser Cys Ser Ala Ser Gly Phe Thr Phe Ser Ser Tyr
                         20                            25                            30
                 Ala Met His Trp Val Arg Gln Ala Pro Gly Lys Gly Leu Glu Tyr Val
                         35                            40                            45
```

```
Ser Ala Ile Ser Ser Asn Gly Gly Ser Thr Tyr Tyr Ala Asp Ser Val
    50                      55                      60
Lys Gly Arg Phe Thr Ile Ser Arg Asp Asn Ser Lys Asn Thr Leu Tyr
65                      70                      75                      80
Leu Gln Met Ser Ser Leu Arg Ala Glu Asp Thr Ala Val Tyr Tyr Cys
                85                      90                      95
Val Ile Glu Gly Gly Ser Thr Gly Ser Thr Val Ser Gly Ala Phe Asp
                100                     105                     110
Ile Trp Gly Gln Gly Thr Met Val Thr Val Ser Ser
            115                     120
```

```
<210>   101
<211>   112
<212>   PRT
<213>   Artificial Sequence

<220>
<221>   UNSURE
<222>   (1)..(112)
<223>   VL
```

```
<400>   101
Asp Ile Val Met Thr Gln Ser Pro Leu Ser Leu Pro Val Thr Leu Gly
1               5                       10                      15
Gln Pro Ala Ser Ile Ser Cys Arg Ser Ser Gln Ser Leu Val Tyr Ser
            20                      25                      30
Asp Gly Asn Thr Tyr Leu Asn Trp Phe Gln Gln Arg Pro Gly Gln Ser
            35                      40                      45
Pro Arg Arg Leu Ile Tyr Glu Val Ser Asn Arg Asp Ser Gly Val Pro
    50                      55                      60
Asp Arg Phe Ser Gly Ser Gly Ser Gly Thr Asp Phe Thr Leu Lys Ile
65                      70                      75                      80
Ser Arg Val Glu Ala Glu Asp Val Gly Val Tyr Tyr Cys Met Gln Gly
                85                      90                      95
Thr His Trp Pro Pro Ala Phe Gly Gln Gly Thr Lys Val Glu Ile Lys
            100                     105                     110
```

```
<210>   102
<211>   112
<212>   PRT
<213>   Artificial Sequence

<220>
<221>   UNSURE
<222>   (1)..(112)
<223>   VL
```

```
<400>   102
Asp Ile Val Met Thr Gln Ser Pro Leu Ser Leu Pro Val Thr Leu Gly
1               5                       10                      15
Gln Pro Ala Ser Ile Ser Cys Arg Ser Ser Gln Ser Leu Val Tyr Ser
            20                      25                      30
Asp Gly Asn Thr Tyr Leu Asn Trp Phe Gln Gln Arg Pro Gly Gln Ser
            35                      40                      45
Pro Arg Arg Leu Ile Tyr Gly Val Ser Asn Arg Asp Ser Gly Val Pro
    50                      55                      60
Asp Arg Phe Ser Gly Ser Gly Ser Gly Thr Asp Phe Thr Leu Lys Ile
65                      70                      75                      80
Ser Arg Val Glu Ala Glu Asp Val Gly Val Tyr Tyr Cys Met Gln Gly
                85                      90                      95
Thr His Trp Pro Pro Ala Phe Gly Gln Gly Thr Lys Val Glu Ile Lys
```

100                    105                    110

```
<210>  103
<211>  112
<212>  PRT
<213>  Artificial Sequence

<220>
<221>  UNSURE
<222>  (1)..(112)
<223>  VL


<400>  103
Asp Ile Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Lys
            20                  25                  30
Asp Gly Asn Thr Tyr Leu Asn Trp Phe Gln Gln Arg Pro Gly Gln Ser
            35                  40                  45
Pro Arg Arg Leu Ile Tyr Lys Val Ser Asn Arg Asp Ser Gly Val Pro
        50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Pro Arg Phe Gly Gln Gly Thr Lys Val Glu Ile Lys
                100                 105                 110
```

**Description**

**Technical Field**

The invention relates to the field of biomedicine, specifically to a SEMG2 antigenic epitope peptide and the use thereof.

**Background of the Invention**

The CD27 molecule belongs to the tumor necrosis factor receptor (TNFR) superfamily and is a type I membrane protein with a molecular weight of about 55 kDa, and exists as a dimer of two monomers linked by a disulfide bond. CD27 is mainly expressed in lymphocytes. Recent studies based on CD27 knockout mice have shown that activation of the CD27 signaling pathway can increase the infiltration of suppressor T cells (Treg) in solid tumors and reduce anti-tumor immunity (Claus C, Riether C, Schürch C, Matter MS, Hilmenyuk T, Ochsenbein AF. Cancer Res. 2012 Jul 15;72(14):3664-76). Consistently, the study also found that Treg cells in skin tissue fail to perform normal immune regulation functions after losing CD27 expression *(Remedios KA, Zirak B, Sandoval PM, Lowe MM, Boda D, Henley E et al., Sci Immunol. 2018. Dec 21;3(30)pii:eaau2042)*. Furthermore, activation of CD27 increases Treg numbers and reduces atherosclerosis in hyperlipidemic mice (Winkels H, Meiler S, Lievens D, Engel D, Spitz C, Bürger C, et al., Eur Heart J. 2017; 38(48):3590-3599). The recent studies consistently demonstrate that CD27 plays an important role in the functional activation of specific Treg cells (including tumor-infiltrating Treg), and therefore avoiding the activation of CD27 expressed by tumor-infiltrating Treg cells is a potential cancer treatment strategy.

Binding to ligands activates the downstream signal transduction of CD27, and the currently known CD27 ligand molecule is CD70. CD70 is a 193 amino acid polypeptide with a hydrophilic N-terminal domain of 20 amino acids and a C-terminal domain containing 2 potential N-linked glycosylation sites, belonging to the TNF family (Goodwin, R . G . et al. (1993) Cell 73:447-56; Bowman et al. (1994) Immunol 152:1756 - 61). These properties suggest that CD70 is a type II transmembrane protein with an extracellular C-terminal portion. CD70 is transiently present on activated T and B lymphocytes and dendritic cells (Hintzen et al., (1994) J. Immunol. 152:1762-1773; Oshima et al., (1998) Int. Immunol. 10:517-26; Tesselaar et al., (2003) J. Immunol. 170:33-40). In addition to normal cells, CD70 expression has been reported in different types of cancer, including renal cell carcinoma, metastatic breast cancer, brain tumor, leukemia, lymphoma, and nasopharyngeal carcinoma *(Junker et al., J. Urol. 2005; 173: 2150-3; Sloan et al., Am J Pathol. 2004; 164:315-23; Held-Feindt and Mentlein et al., Int J Cancer 2002; 98:352-6)*. Currently, blocking the binding between CD70 and CD27 is a strategy being investigated for tumor immunotherapy.

Previous studies have not suggested that CD27 has other ligands than CD70. However, novel ligands of immune checkpoint pathway receptors (especially novel ligands expressed by tumor cells with relatively high specificity) are of great significance for the development of more effective anti-tumor treatments. The present invention aims to develop new anti-tumor treatments and drugs.

**Summary of the Invention**

In one aspect, the invention discloses a compound agonizing or antagonizing an interaction between SEMG2 and CD27. Wherein, the interaction between SEMG2 and CD27 is located on the amino acid site at positions 497, 498, 499, 500, 501, 502, 503, 504, 505, 506, and 508 of SEMG2, and the amino acid sequence of the SEMG2 protein is shown in SEQ ID NO:1. Wherein, the compound is a small molecule inhibitor, polypeptide, antibody, or antigen-binding fragment.

In one embodiment, the invention discloses a polypeptide, the polypeptide comprises an amino acid sequence of SEQ ID NO:2 (QIEKLVEGKS), SEQ ID NO:86 (QIEKLVEGKS(x)I(x)), SEQ ID NO:87 (QIEKLVEGKS(x)I), or SEQ ID NO:88 (QIEKLVEGKS(x)); preferably the polypeptide comprises an amino acid sequence of SEQ ID NO:3 (QIEKLVEGKSQIQ), SEQ ID NO:4 (QIEKLVEGKSQ), or SEQ ID NO:5 (QIEKLVEGKSQI), or an amino acid sequence at least 90%identity to an amino acid sequence as provided in SEQ ID NOs: 2-5. Wherein, the polypeptide agonizes the interaction between SEMG2 and CD27. Wherein, the amino acid site of the interaction between SEMG2 and CD27 is located at positions 497, 498, 499, 500, 501, 502, 503, 504, 505, 506 and 508 of SEMG2, and the amino acid sequence of the SEMG2 protein is shown in SEQ ID NO:1.

In one embodiment, the invention discloses an antibody specifically binding to native or mutant SEMG2 protein, the antibody binds to an antigenic epitope peptide derived from SEMG2 protein, and the antigenic epitope peptide comprises an amino acid sequence of SEQ ID NO:2 (QIEKLVEGKS), SEQ ID NO:3 (QIEKLVEGKSQIQ), SEQ ID NO:4 (QIEKLVEGKSQ), or SEQ ID NO:5 (QIEKLVEGKSQI). Wherein, the antibody antagonizes the interaction between SEMG2 and CD27.

In one embodiment, the invention discloses an antibody specifically binding to native or mutant SEMG2 protein, the antibody recognizes at least one amino acid residue at positions 497, 498, 499, 500, 501, 502, 503, 504, 505, 506 and 508 of the native SEMG2 protein or recognizes an amino acid residue in the corresponding position of the mutant SEMG2 protein, the amino acid sequence of the native SEMG2 protein is shown in SEQ ID NO:1. Wherein, the antibody antagonizes the interaction between SEMG2 and CD27.

In one embodiment, the invention discloses an antibody specifically binding native or mutant SEMG2 protein, wherein the antibody comprises a heavy chain variable region and a light chain variable region, the heavy chain variable region comprises HCDR1, HCDR2 and HCDR3 defined by IMGT; and the light chain variable region comprises LCDR1, LCDR2 and LCDR3 defined by IMGT,

the HCDR1 consists of or comprises an amino acid sequence selected from the group consisting of SEQ ID NOs:6-11, SEQ ID NOs:60-61 and SEQ ID NO:76;
the HCDR2 consists of or comprises an amino acid sequence selected from the group consisting of SEQ ID NOs:12-16 and SEQ ID NOs:62-64;
the HCDR3 consists of or comprises an amino acid sequence selected from the group consisting of SEQ ID NOs:17-20, SEQ ID NOs:65-67 and SEQ ID NOs:77-81;
the LCDR1 consists of or comprises an amino acid sequence selected from the group consisting of SEQ ID NOs:21-25, SEQ ID NOs:68-70 and SEQ ID NO:82;
the LCDR2 consists of or comprises an amino acid sequence selected from the group consisting of SEQ ID NOs:26-29, SEQ ID NOs:71-72, SEQ ID NOs:83-84 and SEQ ID NO:28;
the LCDR3 consists of or comprises an amino acid sequence selected from the group consisting of SEQ ID NOs:30-34, SEQ ID NOs:73-75, SEQ ID NO:85 and SEQ ID NO:99.

In one specific embodiment, the CDR sequence of the antibody is selected from any one of the combinations in (a)-(k):

(a) the HCDR1 comprises the amino acid sequence of SEQ ID NO:6; the HCDR2 comprises the amino acid sequence of SEQ ID NO:12; the HCDR3 comprises the amino acid sequence of SEQ ID NO:17; the LCDR1 comprises the amino acid sequence of SEQ ID NO:21; the LCDR2 comprises the amino acid sequence of SEQ ID NO:26; the LCDR3 comprises the amino acid sequence of SEQ ID NO:30;

(b) the HCDR1 comprises the amino acid sequence of SEQ ID NO:7; the HCDR2 comprises the amino acid sequence of SEQ ID NO:13; the HCDR3 comprises the amino acid sequence of SEQ ID NO:18; the LCDR1 comprises the amino acid sequence of SEQ ID NO:22; the amino acid sequence of LCDR2 comprises the amino acid sequence of SEQ ID NO:27; the LCDR3 comprises the amino acid sequence of SEQ ID NO:31 or SEQ ID NO:99;

(c) the HCDR1 comprises the amino acid sequence of SEQ ID NO:6; the HCDR2 comprises the amino acid sequence of SEQ ID NO:16; the HCDR3 comprises the amino acid sequence of SEQ ID NO:17; the LCDR1 comprises the amino acid sequence of SEQ ID NO:21; the LCDR2 comprises the amino acid sequence of SEQ ID NO:26; the LCDR3 comprises the amino acid sequence of SEQ ID NO:30;

(d) the HCDR1 comprises the amino acid sequence of SEQ ID NO:8; the HCDR2 comprises the amino acid sequence of SEQ ID NO:13; the HCDR3 comprises the amino acid sequence of SEQ ID NO:18; the LCDR1 comprises the amino acid sequence of SEQ ID NO:23; the LCDR2 comprises the amino acid sequence of SEQ ID NO:27; the LCDR3 comprises the amino acid sequence of SEQ ID NO:32;

(e) the HCDR1 comprises the amino acid sequence of SEQ ID NO:9; the HCDR2 comprises the amino acid sequence of SEQ ID NO:14; the HCDR3 comprises the amino acid sequence of SEQ ID NO:19; the LCDR1 comprises the amino acid sequence of SEQ ID NO:24; the LCDR2 comprises the amino acid sequence of SEQ ID NO:28; the LCDR3 comprises the amino acid sequence of SEQ ID NO:33;

(f) the HCDR1 comprises the amino acid sequence of SEQ ID NO:10; the HCDR2 comprises the amino acid sequence of SEQ ID NO:15; the HCDR3 comprises the amino acid sequence of SEQ ID NO:20; the LCDR1 comprises the amino acid sequence of SEQ ID NO:25; the LCDR2 comprises the amino acid sequence of SEQ ID NO:29; the LCDR3 comprises the amino acid sequence of SEQ ID NO:34;

(g) the HCDR1 comprises the amino acid sequence of SEQ ID NO:11; the HCDR2 comprises the amino acid sequence of SEQ ID NO:15; the HCDR3 comprises the amino acid sequence of SEQ ID NO:20; the LCDR1 comprises the amino acid sequence of SEQ ID NO:25; the LCDR2 comprises the amino acid sequence of SEQ ID NO:29; the LCDR3 comprises the amino acid sequence of SEQ ID NO:34;

(h) the HCDR1 comprises the amino acid sequence of SEQ ID NO:60; the HCDR2 comprises the amino acid sequence of SEQ ID NO:62; the HCDR3 comprises the amino acid sequence of SEQ ID NO:65; the LCDR1 comprises the amino acid sequence of SEQ ID NO:68; the LCDR2 comprises the amino acid sequence of SEQ ID NO:71; the LCDR3 comprises the amino acid sequence of SEQ ID NO:73;

(i) the HCDR1 comprises the amino acid sequence of SEQ ID NO:61; the HCDR2 comprises the amino acid sequence of SEQ ID NO:63; the HCDR3 comprises the amino acid sequence of SEQ ID NO:66; the LCDR1 comprises the amino acid sequence of SEQ ID NO:69; the LCDR2 comprises the amino acid sequence of SEQ ID NO:72; the LCDR3 comprises the amino acid sequence of SEQ ID NO:74;

(j) the HCDR1 comprises the amino acid sequence of SEQ ID NO:60; the HCDR2 comprises the amino acid sequence of SEQ ID NO:64; the HCDR3 comprises the amino acid sequence of SEQ ID NO:67; the LCDR1 comprises the amino acid sequence of SEQ ID NO:70; the LCDR2 comprises the amino acid sequence of SEQ ID NO:28; the LCDR3 comprises the amino acid sequence of SEQ ID NO:75;

(k) the HCDR1 comprises the amino acid sequence of SEQ ID NO:60 or 76; the HCDR2 comprises the amino acid sequence of SEQ ID NO:64 or 62; the HCDR3 comprises the amino acid sequence of SEQ ID NO:77, 78 or 79; and/or

the LCDR1 comprises the amino acid sequence of SEQ ID NO:70 or 82; the LCDR2 comprises the amino acid sequence of SEQ ID NO:28, 83 or 84; the LCDR3 comprises the amino acid sequence of SEQ ID NO:75 or 85.

In one embodiment, the invention discloses an antibody binding to native or mutant SEMG2 protein specifically, wherein the antibody comprises a heavy chain variable region and a light chain variable region,

the heavy chain variable region comprises an amino acid sequence selected from the group consisting of SEQ ID NOs:35-41, 48-51, 54-56 and 96-100, or an amino acid sequence at least 70%, 80%, 90%, 95% or 99% identity to any sequences of SEQ ID NOs:35-41, 48-51, 54-56 and 96-100;

the light chain variable region comprises the amino acid sequence selected from the group consisting of SEQ ID NOs:42-47, 52-53, 57-69 and 101-103, or an amino acid sequence at least 70%, 80%, 90%, 95% or 99% identity to any sequences of SEQ ID NOs:42-47, 52-53, 57-69 and 101-103.

In one specific embodiment, the heavy chain variable region and the light chain variable region are selected from any one of the combinations in (a)-(o):

(a) the heavy chain variable region comprises SEQ ID NO:35 or an amino acid sequence at least 70%, 80%, 90%, 95% or 99% identity to SEQ ID NO:35; the light chain variable region comprises SEQ ID NO:42 or an amino acid sequence at least 70%, 80%, 90%, 95% or 99% identity to SEQ ID NO:42;

(b) the heavy chain variable region comprises SEQ ID NO:36 or an amino acid sequence at least 70%, 80%, 90%, 95% or 99% identity to SEQ ID NO:36; the light chain variable region comprises SEQ ID NO:43 or an amino acid sequence at least 70%, 80%, 90%, 95% or 99% identity to SEQ ID NO:43;

(c) the heavy chain variable region comprises SEQ ID NO:37 or an amino acid sequence at least 70%, 80%, 90%, 95% or 99% identity to SEQ ID NO:37; the light chain variable region comprises SEQ ID NO:44 or an amino acid sequence at least 70%, 80%, 90%, 95% or 99% identity to SEQ ID NO:44;

(d) the heavy chain variable region comprises SEQ ID NO:38 or an amino acid sequence at least 70%, 80%, 90%, 95% or 99% identity to SEQ ID NO:38; the light chain variable region comprises SEQ ID NO:45 or an amino acid sequence at least 70%, 80%, 90%, 95% or 99% identity to SEQ ID NO:45;

(e) the heavy chain variable region comprises SEQ ID NO:39 or an amino acid sequence at least 70%, 80%, 90%, 95% or 99% identity to SEQ ID NO:39; the light chain variable region comprises SEQ ID NO:46 or an amino acid sequence at least 70%, 80%, 90%, 95% or 99% identity to SEQ ID NO:46;

(f) the heavy chain variable region comprises SEQ ID NO:40 or an amino acid sequence at least 70%, 80%, 90%, 95% or 99% identity to SEQ ID NO:40; the light chain variable region comprises SEQ ID NO:47 or an amino acid sequence at least 70%, 80%, 90%, 95% or 99% identity to SEQ ID NO:47;

(g) the heavy chain variable region comprises SEQ ID NO:41 or an amino acid sequence at least 70%, 80%, 90%, 95% or 99% identity to SEQ ID NO:41; the light chain variable region comprises SEQ ID NO:47 or an amino acid sequence at least 70%, 80%, 90%, 95% or 99% identity to SEQ ID NO:47;

(h) the heavy chain variable region comprises SEQ ID NO:48, 49, 50, 51 or an amino acid sequence at least 70%, 80%, 90%, 95% or 99% identity to SEQ ID NO:48, 49, 50 or 51; the light chain variable region comprises SEQ ID NO:52, 53 or an amino acid sequence at least 70%, 80%, 90%, 95% or 99% identity to SEQ ID NO:52 or 53;

(i) the heavy chain variable region comprises SEQ ID NO:54 or an amino acid sequence at least 70%, 80%, 90%, 95% or 99%identity to SEQ ID NO:54; the light chain variable region comprises SEQ ID NO:57 or an amino acid sequence at least 70%, 80%, 90%, 95% or 99% identity to SEQ ID NO:57;

(j) the heavy chain variable region comprises SEQ ID NO:55 or an amino acid sequence at least 70%, 80%, 90%, 95% or 99% identity to SEQ ID NO:55; the light chain variable region comprises SEQ ID NO:58 or an amino acid sequence at least 70%, 80%, 90%, 95% or 99% identity to SEQ ID NO:58;

(k) the heavy chain variable region comprises SEQ ID NO:56 or an amino acid sequence at least 70%, 80%, 90%, 95% or 99% identity to SEQ ID NO:56; the light chain variable region comprises SEQ ID NO:59 or an amino acid sequence at least 70%, 80%, 90%, 95% or 99% identity to SEQ ID NO:59;

(l) the heavy chain variable region comprises SEQ ID NO:96 or an amino acid sequence at least 70%, 80%, 90%, 95% or 99% identity to SEQ ID NO:96; the light chain variable region comprises SEQ ID NO:59, 101, 102, 103 or an amino acid sequence at least 70%, 80%, 90%, 95% or 99% identity to SEQ ID NO:59, 101, 102 or 103;

(m) the heavy chain variable region comprises SEQ ID NO:97 or an amino acid sequence at least 70%, 80%, 90%, 95% or 99% identity to SEQ ID NO:97; the light chain variable region comprises SEQ ID NO:59 or an amino acid sequence at least 70%, 80%, 90%, 95% or 99% identity to SEQ ID NO:59;

(n) the heavy chain variable region comprises SEQ ID NO:98 or an amino acid sequence at least 70%, 80%, 90%, 95% or 99% identity to SEQ ID NO:98; the light chain variable region comprises SEQ ID NO:103 or an amino acid sequence at least 70%, 80%, 90%, 95% or 99% identity to SEQ ID NO:103;

(o) the heavy chain variable region comprises SEQ ID NO:99, 100 or an amino acid sequence at least 70%, 80%, 90%, 95% or 99% identity to SEQ ID NO:99 or 100; the light chain variable region comprises SEQ ID NO:57 or an amino acid sequence at least 70%, 80%, 90%, 95% or 99% identity to SEQ ID NO:57.

The antibody of the invention further comprises a coupling moiety linked to the polypeptide, the coupling moiety is selected from the group consisting one or more of radionuclides, drugs, toxins, cytokines, enzymes, fluorescein, carrier proteins, lipids, and biotin, wherein the polypeptide or antibody is selectively linked to the coupling moiety by a linker, preferably the linker is a peptide or polypeptide.

Wherein the antibody is selected from monoclonal antibodies, polyclonal antibodies, antisera, chimeric antibodies, humanized antibodies, and human antibodies.

Wherein the antibody is selected from multi-specific antibodies, single-chain variable fragments (scFvs), single-chain antibodies, anti-idiotype (anti-Id) antibodies, diabodies, minibodies, nanobodies, single domain antibodies, Fab fragments, F(ab') Fragments, disulfide-linked bispecific Fv (sdFv) and intracellular antibodies.

In another aspect, the invention discloses an antigenic epitope peptide, wherein the antigenic epitope peptide is derived from SEMG2 protein, and the amino acid of the antigenic epitope peptide comprises an amino acid sequence selected from the group consisting of SEQ ID NO:2 (QIEKLVEGKS), SEQ ID NO:3 (QIEKLVEGKSQIQ), SEQ ID NO:4 (QIEKLVEGKSQ), and SEQ ID NO:5 (QIEKLVEGKSQI).

The invention discloses a protein, wherein the protein comprises an amino acid sequence as the amino acid sequences shown in SEQ ID NO:2 (QIEKLVEGKS), SEQ ID NO:86 (QIEKLVEGKS(x)I(x)), SEQ ID NO:87 (QIEKLVEGKS(x))I), or SEQ ID NO:88 (QIEKLVEGKS(x)), preferably the polypeptide comprises an amino acid sequence of SEQ ID NO:3 (QIEKLVEGKSQIQ), SEQ ID NO:4 (QIEKLVEGKSQ), or SEQ ID NO:5 (QIEKLVEGKSQI) or an amino acid sequence at least 90% identity to any one of SEQ ID NOs:2-5, more preferably SEQ ID NOs:89-94 and SEQ ID NO:3 (corresponding to P1-P6, and P7 respectively); and a tag sequence which can selectively be linked at the N-terminus or C-terminus. Those skilled in the art should understand that the addition of a protein tag will not affect the prepared antibody's participation in binding between SEMG2 and CD27, the protein tag includes, but is not limited to, C-Myc, His, GST (glutathione S-transferase), HA, MBP (maltose-binding protein), Flag, SUMO, eGFP/eCFP/eYFP/mCherry, etc.

In one specific embodiment, the polypeptide has amino acid sequence of SEQ ID NO:3 (P7: QIEKLVEGKSQIQ) or SEQ ID NO:93 (P5).

The invention also discloses a method of preparing an antibody or an antigen-binding fragment thereof, wherein the protein is used as an immunogen to inject a subject such as a mouse or screening natural library to prepare the antibody, and the amino acid sequence of the antibody comprises an amino acid sequence of SEQ ID NO:2 (QIEKLVEGKS), SEQ ID NO: 86 (QIEKLVEGKS(x)I(x)), SEQ ID NO:87 (QIEKLVEGKS(x)I), or SEQ ID NO:88(QIEKLVEGKS(x)), preferably the polypeptide comprises an amino acid sequence of SEQ ID NO:3 (QIEKLVEGKSQIQ), SEQ ID NO:4 (QIEKLVEGKSQ), or SEQ ID NO:5 (QIEKLVEGKSQI) or an amino acid sequence at least 90% identity to any one of SEQ ID NOs:2-5, more preferably SEQ ID NO:93 (P5) or SEQ ID NO:3 (P7).

In a preferred embodiment, a method of obtaining isolated antibodies is disclosed, which uses a key epitope polypeptide of the binding between SEMG2 and CD27 as immunogen and screens with murine hybridoma and phage display in human and camel natural library.

In another aspect, the invention discloses an isolated polynucleotide encoding the compound, antigenic peptide, or protein.

The invention discloses a recombinant vector comprising the polynucleotide and optional regulatory sequences; preferably, the recombinant vector is a cloning vector or an expression vector.

Wherein, the regulatory sequence is selected from a leading sequence, a polyadenylation sequence, a polypeptide sequence, a promoter, a signal sequence, a transcription terminator, or any combination thereof.

The invention discloses a host cell comprising the recombinant vector.

Wherein, the host cell is a prokaryotic cell or a eukaryotic cell.

The invention discloses a pharmaceutical composition comprising the compound, the antigenic peptide, the protein, the polynucleotide, the recombinant vector, and one or more types of the host cells as previously described.

Wherein, the composition further comprises a pharmaceutically acceptable carrier or adjuvant.

The invention also discloses the use of the compounds, the antigenic peptide, the protein, the polynucleotide, the recombinant vector, or the host cell in preparation of products for agonizing or antagonizing the interaction between SEMG2 and CD27, preferably SEMG2 is expressed in tumor cells and CD27 is expressed in immune cells.

The invention also discloses the use of the compound, the antigenic peptide, the protein, the polynucleotide, the recombinant vector, or the host cell in preparation of a drug for preventing or treating tumors or a drug for modulating an immune response elicited against tumors.

In one specific embodiment, wherein the tumor is selected from one or more of colorectal cancer, lung cancer, melanoma, lymphoma, liver cancer, head and neck cancer, stomach cancer, kidney cancer, bladder cancer, prostate cancer, testicular cancer, endometrial cancer, breast cancer and ovarian cancer.

The invention also discloses a method of screening drugs or reagents preventing or treating tumors, comprising obtaining candidate drugs or reagents by screening inhibitors or antibodies which inhibit the interaction between SEMG2 and CD27.

The invention also discloses a method of preventing or treating tumors, comprising:

contacting immune cells such as lymphocytes (T lymphocytes) or tumor cells of the subject with an effective dose of any one of the compound; wherein the expression of SEMG2 in tumor cells can be selectively detected before contacting immune cells such as lymphocytes and/or tumor cells of the subject with an effective dose of the compound.

Wherein the subject has received or is receiving or will receive additional anti-cancer therapy.

Wherein the additional anti-cancer therapy comprises surgery, radiotherapy, chemotherapy, immunotherapy, or hormone therapy.

The invention also discloses a kit comprising one or more of the compounds, the antigenic peptides, the proteins, the polynucleotides, the recombinant vectors, and one or more types of the host cells, and the above components are accommodated in a suitable container.

The invention also discloses a method of detecting the presence or absence of SEMG2 in a biological sample *in vitro,* comprising: contacting the biological sample with the compound.

A method of inhibiting tumor cell growth of tumor cells, comprising the following steps: A) analyzing the expression of SEMG2 in tumor cells; B) contacting the tumor cells with an antibody recognizing SEMG2, the binding of the antibody to SEMG2 is KD $<2\times10^{-8}$; C) contacting T lymphocytes, with the antibody and tumor cells. Wherein the KD$<2\times10^{-8}$, $<1\times10^{-8}$, $<9\times10^{-9}$, $<8\times10^{-9}$, $<7\times10^{-9}$, $<6\times10^{-9}$, $<5\times10^{-9}$, $<4\times10^{-9}$, $<3\times10^{-9}$, $<2\times10^{-9}$, $<1\times10^{-9}$, $<1\times10^{-10}$.

**Description of the Drawings**

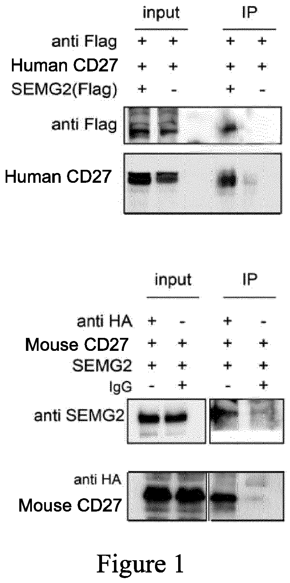

**FIGURE 1** depicts the result of co-immunoprecipitation assay, divided into upper panel and lower panel. The upper panel demonstrates the existence of physical interaction between human CD27 and SEMG2 (Flag). The lower panel demonstrates the existence of physical interaction between mouse CD27 and SEMG2 (Flag).

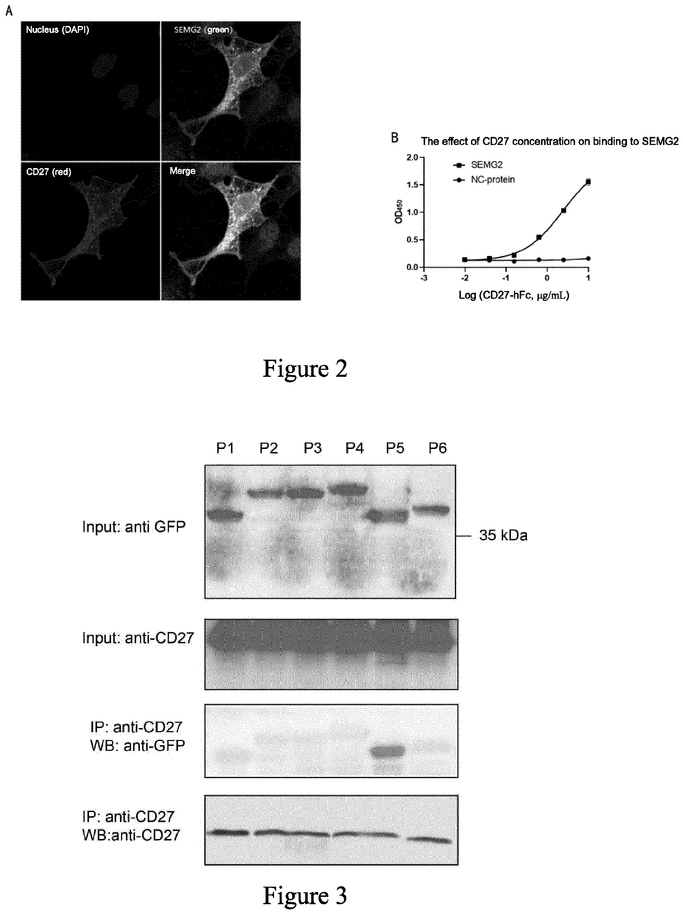

**FIGURE 2** depicts the results of immunofluorescent staining and ELISA. Figure 2(A) demonstrates significant colocalization between CD27 and SEMG2 after overexpression in tumor cells. Figure 2(B) shows the result of ELISA, demonstrating the effect of CD27 concentration-dependent on binding SEMG2 in microplate, while CD27 does not bind to the negative control protein and there is no concentration effect.

**FIGURE 3** depicts the result of co-immunoprecipitation assay for examining whether SEMG2 fragment (i.e., P1 to P6) binds to CD27. Wherein the P5 fragment has been detected with significant binding to CD27.

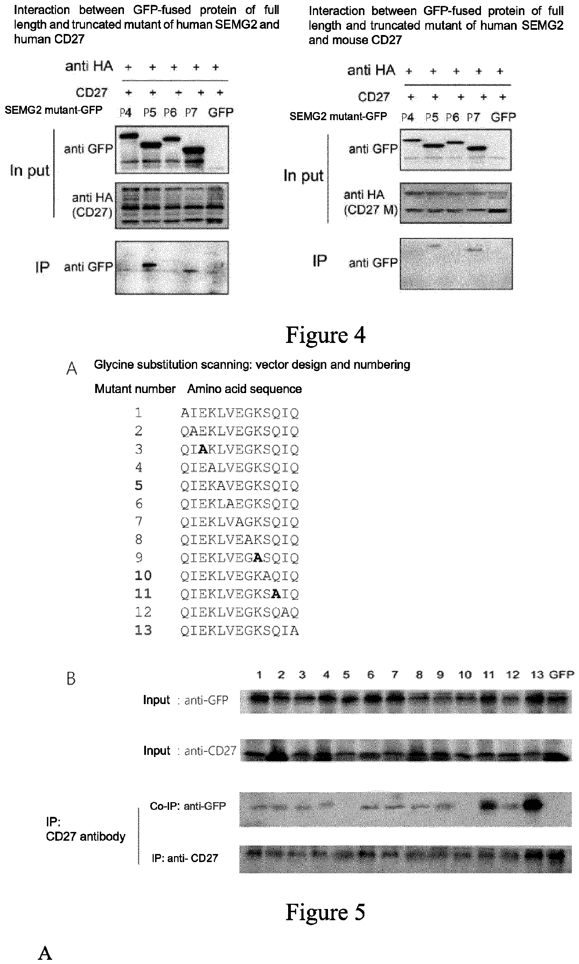

**FIGURE 4** depicts the result of co-immunoprecipitation assay for examining whether SEMG2 fragment (i.e., P4, P5, P6, P7) binds to CD27. Wherein the P7 sequence is derived from a part of P5, which is "QIEKLVEGKSQIQ". The result includes left panel and right panel. The left panel shows the binding between the SEMG2 fragment and human CD27, and the right panel shows the binding between the SEMG2 fragment and mouse CD27. The results show that both human and mouse CD27 could bind to P5 and P7 fragments.

**FIGURE 5** depicts the contribution of each amino acid of P7 to binding CD27 protein, accurately demonstrated by Alanine Scanning method, including panel A and panel B. Panel A shows the sequence produced by substitution of each amino acid of P7 for glycine one by one, i.e., the mutated amino acid sequences numbered 1-13. Panel B is the result of co-immunoprecipitation experiment, indicating the extent to which the GFP fusion protein of mutant 1-13 polypeptide binds to CD27; wherein mutants 5 and 9 completely lost the binding to CD27; mutants 11 and 13 did not affect the binding between SEMG2 (497 -509) and CD27; mutants at other sites (1, 2, 3, 4, 6, 7, 8, 10, 12) somewhat attenuated the binding between SEMG2 (497-509) and CD27. The amino acids located at positions 497, 498, 499, 500, 501, 502, 503, 504, 505, 506 and 508 of SEMG2 have obvious effects on the binding to CD27.

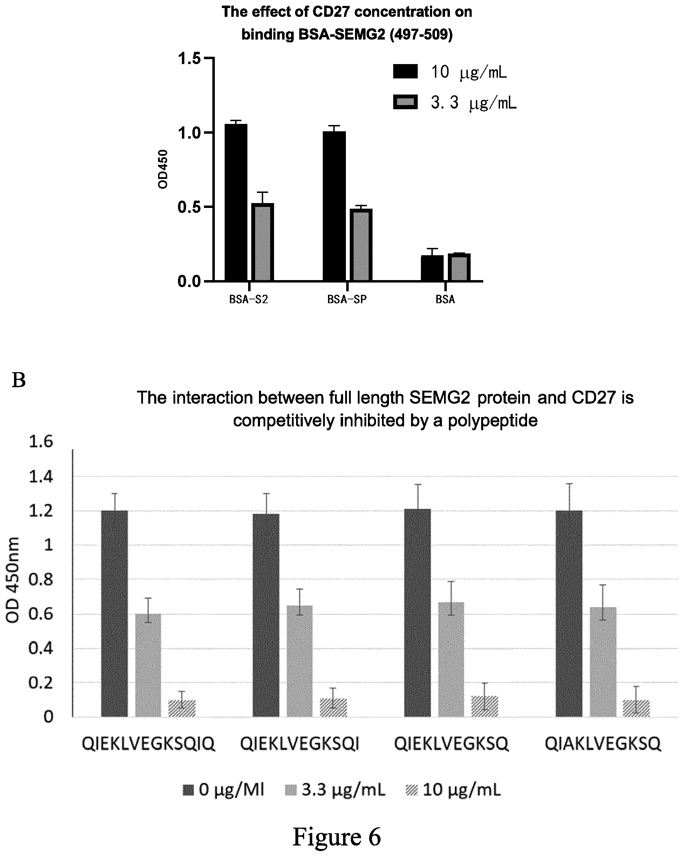

**FIGURE 6** depicts the binding between SEMG2 epitope polypeptides and CD27, and its competitive inhibition of full-length SEMG2 binding to CD27. (A) **FIGURE 6A** shows BSA-conjugated human SEMG2 (497-509) polypeptide and monkey SEMG2 polypeptide can bind to CD27 protein on the microplate respectively, which are significantly higher than that of the negative BSA control, therefore indicating that CD27 can bind to both human and monkey SEMG2 (497-509) fragment. (B) **FIGURE 6B** shows the inhibitory effect of SEMG2-derived polypeptides and derivatives QIEKLVEGKSQIQ, QIEKLVEGKSQI, QIEKLVEGKSQ and QIAKLVEGKSQ on the binding between full-length SEMG2 and CD27. As shown in the figure, different concentrations of peptides are firstly co-incubated with CD27-Fc for binding, and then added into the micro-reaction plate pre-coated with SEMG2 protein. After co-incubation, the unconjugated molecules are washed off, and anti-Fc secondary antibody-HRP are then used for detecting and developing. The results show that the polypeptide molecule can inhibit the binding between full-length SEMG2 and CD27.

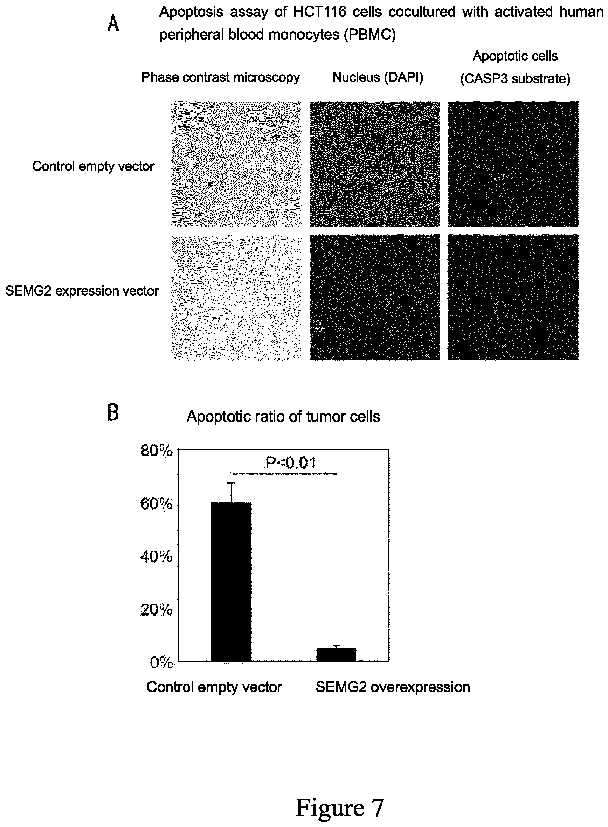

**FIGURE 7** depicts apoptosis assay of HCT116 cells stably transfected with SEMG2 or control empty vector and co-cultured with activated human peripheral blood mononuclear cells. (A) **FIGURE 7A** is a representative image of apoptosis analysis, green field shows apoptotic cells. (B) **FIGURE 7B** is based on the statistics of three independent biological experiments (error bars represent standard deviation).

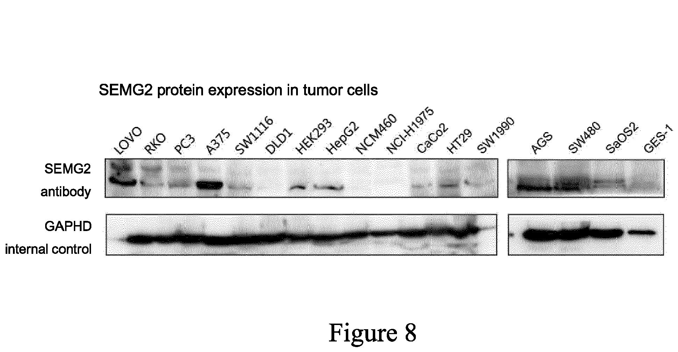

**FIGURE 8** depicts immunoblotting assay to show the expression of SEMG2 protein in different tumor cells. The names of the tumor cells are indicated above (the font is tilted 45 degrees). About half of the tested cell lines have detectable SEMG2 protein expression.

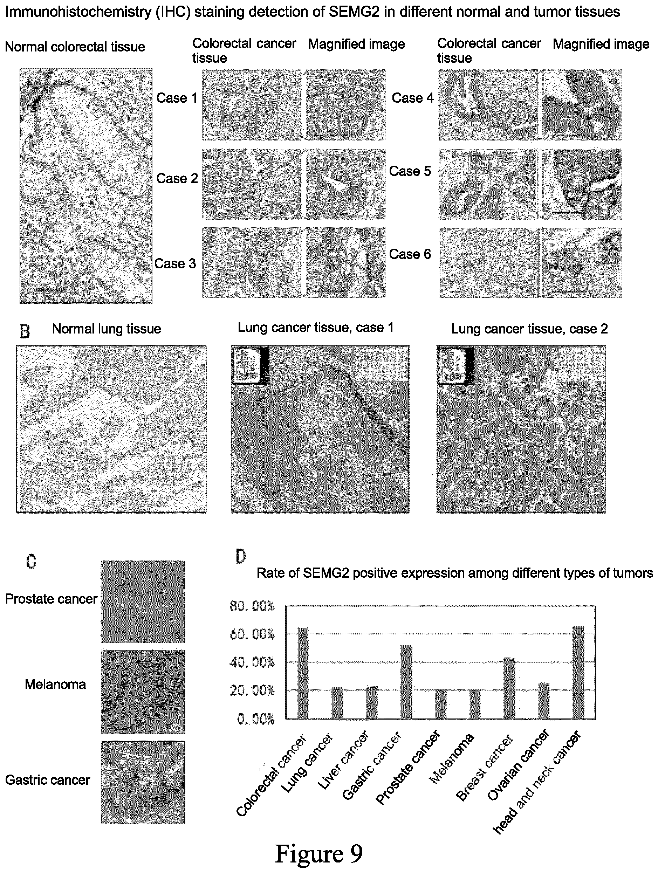

**FIGURE 9** depicts the result of immunohistochemistry (IHC) assay revealing the expression of SEMG2 protein in different tumor tissues. (A) **FIGURE 9A** shows the expression of SEMG2 in different colorectal cancer tumor tissues, with normal colorectal tissue as control; (B) **FIGURE 9B** shows the expression of SEMG2 in different lung cancer tissues, with normal lung tissue as control; (C) **FIGURE 9C** shows representative images of SEMG2 positive expression in prostate cancer, melanoma, and gastric cancer. Due to space limitation, the detection results of all tumor types are not listed here in detail; (D) **FIGURE 9D** is the rate of SEMG2 positive expression among different types of tumors. Positive expression is defined as moderate or strong positive expression by immunohistochemical staining. Statistical results based on tissue chips (each chip included more than 50 tissue samples) are plotted as percentages to show the positive expression ratio of SEMG2.

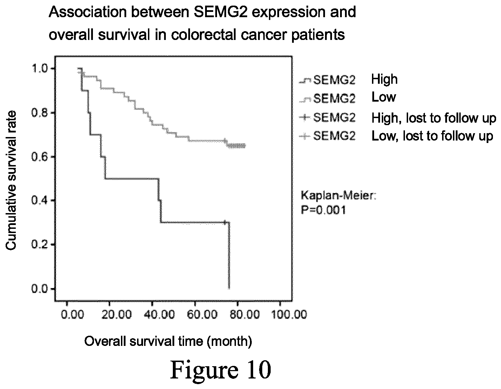

**FIGURE 10** depicts the statistical result of the Kaplan-Meier factor survival analysis, which suggests that high expression of SEMG2 (defined as moderate, strong positive staining by SEMG2 immunohistochemical staining) is significantly associated with shortened overall survival in colorectal cancer patients. P value below 0.001 indicates a highly significant association.

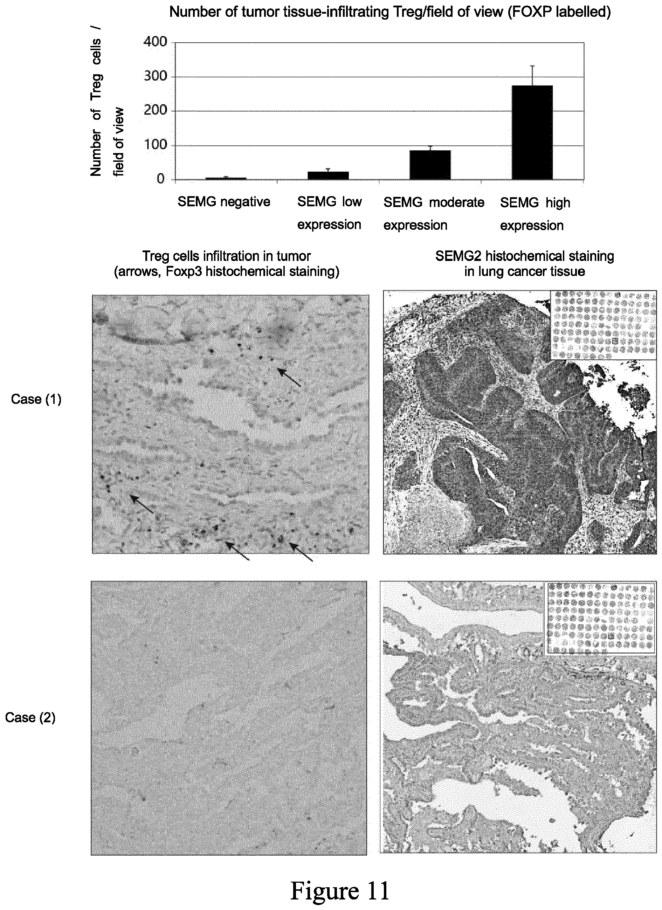

**FIGURE 11** depicts the result of the immunohistochemical assay. The upper panel shows the statistical result of the correlation between the staining of regulatory T lymphocytes, namely Treg and SEMG2 in lung cancer. The intensity of SEMG2 immunohistochemical staining is divided into different levels, and the number of Treg (labeled with Foxp3 antibody) in each field of view is counted separately and compared. The bottom panels are Treg marked representative images of SEMG2 positive and negative expression, respectively.

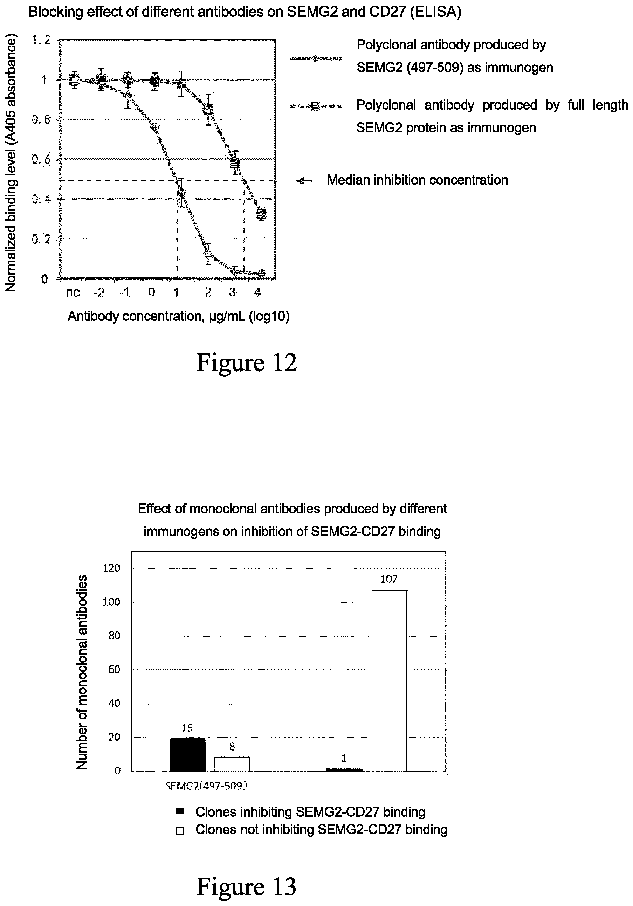

**FIGURE 12** depicts the result of ELISA. The ordinate shows the normalized A405 absorbance value as the reading of ELISA, showing the degree of binding between SEMG2 and CD27; the abscissa shows the concentration of antibody added. The solid line represents the blocking effect of the polyclonal antibody generated by SEMG2(497-509) as an antigen; the dotted line represents the blocking effect of the polyclonal antibody generated by the full-length protein of SEMG2 as an immunogen. The polyclonal antibody generated by SEMG2(497-509) requires a lower concentration to exert the blocking effect, that is, the blocking titer of the antibody generated by SEMG2(497-509) is higher than that of the SEMG2 full-length protein. It suggests that the recognition of the key role epitope, SEMG2(497-509), makes the development of blocking antibodies much easier.

**FIGURE 13** depicts the number of blocking monoclonal antibodies and the total number of antibodies obtained after injecting mice with SEMG2 (497-509) epitope peptide and full-length SEMG2 protein as immunogens. Among the mouse monoclonal antibodies obtained by hybridoma fusion, the antibodies confirmed by ELISA that can inhibit the binding between SEMG2 and CD27 are counted and displayed as black bars. Most of the antibodies prepared from SEMG2 (497-509) epitope fragment as immunogen can block the binding between SEMG2 and CD27, and the positive rate is significantly higher than that of antibodies prepared using full-length SEMG2 as immunogen.

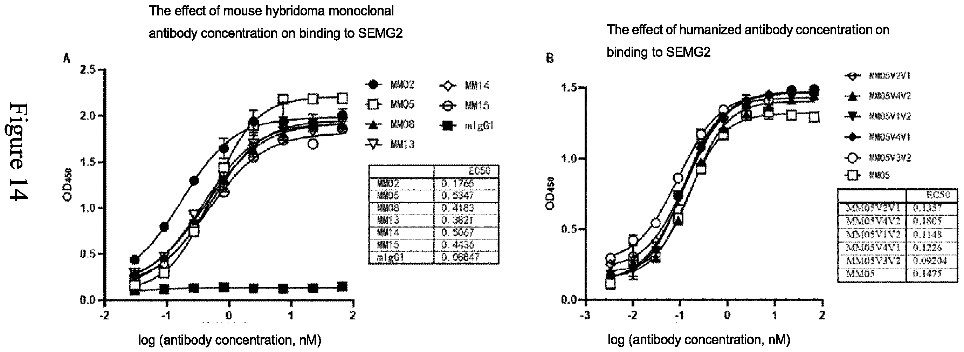

**FIGURE 14** depicts the binding ability of the mouse monoclonal antibody and the humanized mouse monoclonal antibody to SEMG2 protein. The reading of the ELISA, the $OD_{450}$ absorbance, is used as the ordinate, and the abscissa shows the different concentrations of antibody added. As the concentration of the antibody in the ELISA system increases, the $OD_{450}$ value gradually increases, indicating that the binding of SEMG2 to the murine monoclonal antibody (Figure 14A) or the humanized monoclonal antibody (Figure 14B) increases gradually. The fitting curve is a representative result based on statistics from three independent biological experiments.

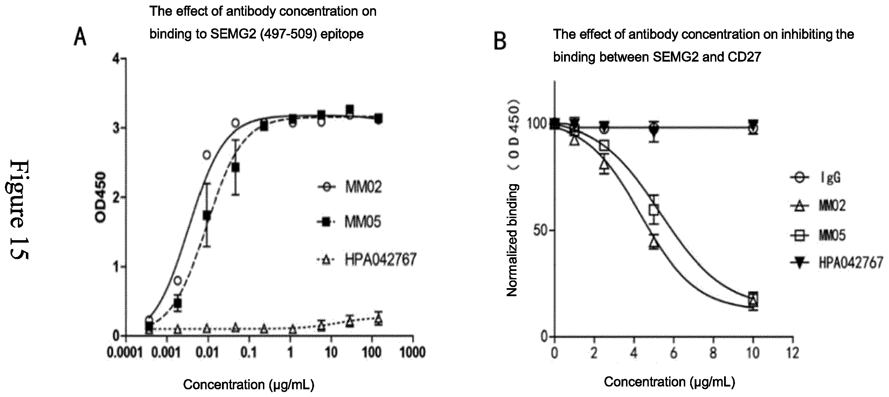

**FIGURE 15** depicts the ability of mouse monoclonal antibody in binding to BSA-SEMG2 (497-509) and blocking the binding between SEMG2 and receptor protein. (A) Panel A shows the reading of the ELISA, the $OD_{450}$ absorbance, is used as the ordinate, and the abscissa shows the added antibody with different concentrations, which indicates a gradually increased binding between SEMG2 (497-509) and the mouse monoclonal antibody. (B) Panel B shows that mouse monoclonal antibody blocks the binding between SEMG2 and CD27, and the blocking effect increases with the increasing concentration. The control mouse IgG antibody does not show blocking function. The ordinate is the blocking ratio which is the normalized blocking ratio; the abscissa shows the different concentrations of antibody added. The binding between SEMG2 and CD27 gradually decreased as the antibody concentration increased in the ELISA system. The fitting curve is based on statistics of three independent biological experiments (error bars represent standard deviation).

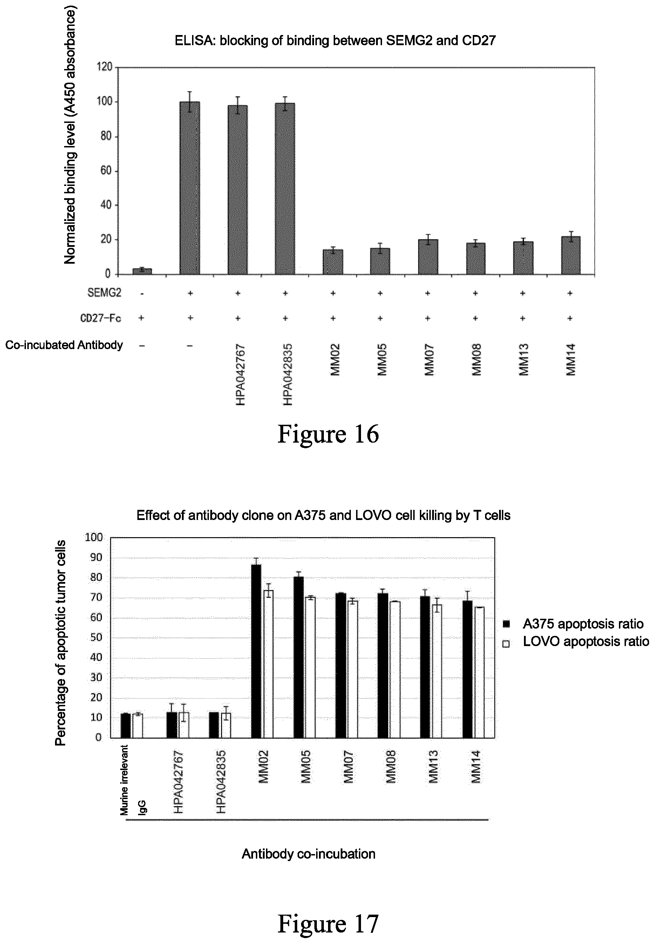

**FIGURE 16** depicts the result of ELISA. The ordinate shows the normalized $OD_{450}$ absorbance as reading of the ELISA, which indicates the extent of binding between SEMG2 (fixed on the surface of ELISA plate) and CD27-Fc added; the abscissa shows different experimental conditions, i.e., different antibodies co-incubated (the concentration is 10 μg/mL): HPA042767 and HPA042835 are rabbit polyclonal antibodies against SEMG2(354-403) and SEMG2(563-574) respectively; MM02, MM05, MM07, MM08, MM13, MM14 are mouse monoclonal antibodies against SEMG2(497-509) epitope. The results show that mouse monoclonal antibodies against SEMG2(497-509) epitope, but not antibodies against other epitopes, block the binding between SEMG2 and CD27. This experiment demonstrates that the mouse monoclonal antibodies against SEMG2(497-509) epitope functionally belong to the same type of antibodies.

**FIGURE 17** depicts the effect of different types of antibodies on tumor cell killing effect by T cells. Activated human peripheral blood monocytes (PBMC) are co-cultured with human melanoma cell A375 highly expressing SEMG2 or colorectal cancer cell LOVO respectively, and meanwhile different antibodies are added: irrelevant mouse IgG, HPA042767, HPA042835, MM02, MM05, MM07, MM08, MM13, or MM14. The ordinate shows the percentage of apoptotic tumor cells; the abscissa shows different treatment conditions in experiment, i.e., the different antibodies added. Mouse monoclonal antibodies (MM02, MM05, MM07, MM08, MM13, or MM14) against SEMG2(497-509) epitope significantly promote the tumor killing effect by T cells, while the control irrelevant IgG or HPA042767 and HPA042835 antibodies against SEMG2(354-403) and SEMG2(563-574) antigenic epitopes do not show such a function. It demonstrates that SEMG2(497-509) epitope are key sites of SEMG2 expressed by tumor cells in immune escape function, and the antibodies directed against this epitope belong to the same type in terms of anti-tumor immunomodulatory function.

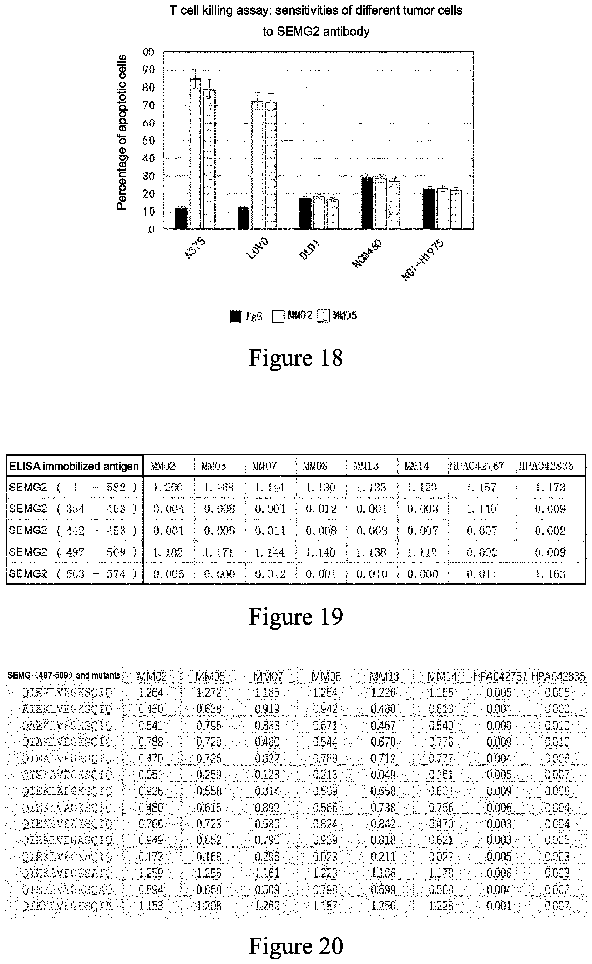

**FIGURE 18** depicts the result of T cell killing experiment to different tumor cells in presence of SEMG2 blocking antibodies. Wherein A375 and LOVO are tumor cells highly expressing SEMG2 protein, while DLD1, NCM460 and NCI-H1975 are SEMG2-negative cells. During the T cell killing experiment on the tumor cells, different antibodies are added, i.e.: irrelevant murine IgG antibody, MM02 or MM05 mouse monoclonal antibody. The abscissa represents the different tumor cell lines, while the ordinate represents the percentage of apoptotic tumor cells. Tumor cells with higher expression of SEMG2 (A375 and LOVO) can be more effectively killed by T cells after antibody treatment, while there is no obvious increase in apoptosis level of tumor cells without SEMG2 expression (DLD1, NCM460 and NCI-H1975) after administration of SEMG2 blocking antibodies MM02 and MM05. This demonstrates that positive expression of SEMG2 can be used as a selective marker for administration of SEMG2-blocking antibodies. When SEMG2 blocking antibody is used as an anti-tumor immune drug, the expression of SEMG2 has guiding significance for the selection of suitable patients.

**FIGURE 19** depicts the A450 absorbance as a reading in ELISA to detect the extent of SEMG2 binding to different antibodies. Different antigens from SEMG2 (shown on the left) were coated on the ELISA plates and conjugated with HPA04276, HPA042835, MM02, MM05, MM07, MM08, MM13, MM14, followed by bound antibody detection using anti-mouse secondary antibody (against HPA04276, HPA042835, MM02, MM05, MM07 and MM08) or anti-rabbit secondary antibody (against HPA04276, HPA042835). MM02, MM05, MM07, MM08, MM13, and MM14 all bind to the SEMG2 (497-509) epitope and belong to the same type; HPA04276 binds to the SEMG2 (354-403) epitope, and HPA042835 binds to the SEMG2 (563-574) epitope.

**FIGURE 20** depicts the value detected by ELISA, i.e., $OD_{450}$ absorbance. In this experiment, the SEMG2 (497-509) epitope peptide and its glycine scan mutant (i.e., amino acid substitution to glycine one by one) polypeptides are immobilized on an ELISA plate, and further bind to different antibodies as shown in figure. This experiment is used to determine the precise amino acid epitopes that different monoclonal antibodies bind to, and the relative importance of each amino acid to the binding antibody. The control antibodies HPA04276 and HPA042835 do not bind to the epitopes and mutants; the amino acids at different sites contribute differently to the binding of the antibodies; and important amino acids bound by each antibody (MM02, MM05, MM07, MM08, MM13, MM14) which blocks the binding between SEMG2 and CD27 are similar. This demonstrates that antibodies with blocking function belong to the same type in terms of binding epitopes.

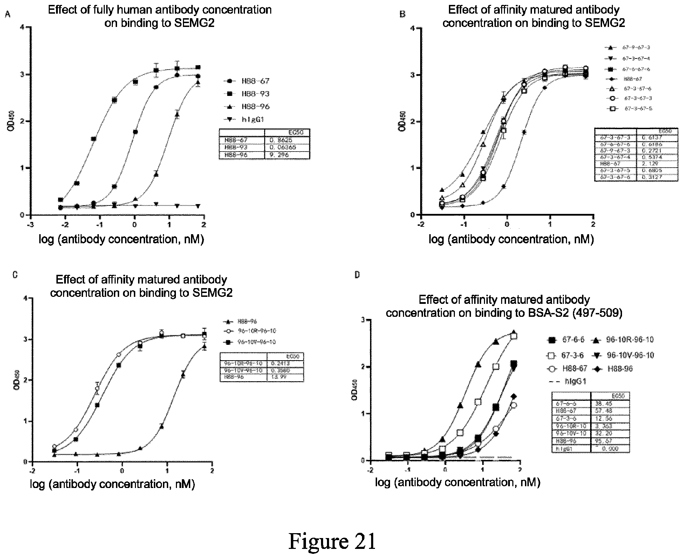

**FIGURE 21** depicts the result of ELISA, which shows effects of fully human antibodies H88-67, H88-93, H88-96 and affinity mature fully human antibodies concentration-dependent on binding to SEMG2 and BSA-SEMG2(497-509) polypeptide. The reading of the ELISA, the $OD_{450}$ absorbance, is used as the ordinate, and the abscissa shows the different concentrations of antibody added.

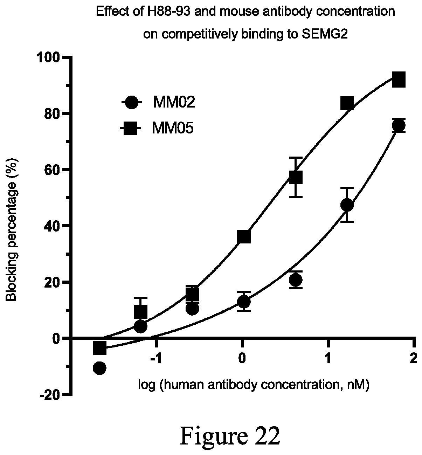

**FIGURE 22** depicts the result of ELISA shows effects of the fully human antibody and mouse monoclonal antibodies concentration-dependent on competitively binding to SEMG2. The ordinate shows the ratio of fully human antibody blocking the binding between SEMG2's and mouse antibodies. As the concentration of fully human antibody increases, the detected signal of mouse antibodies binding to SEMG2 gradually decreases.

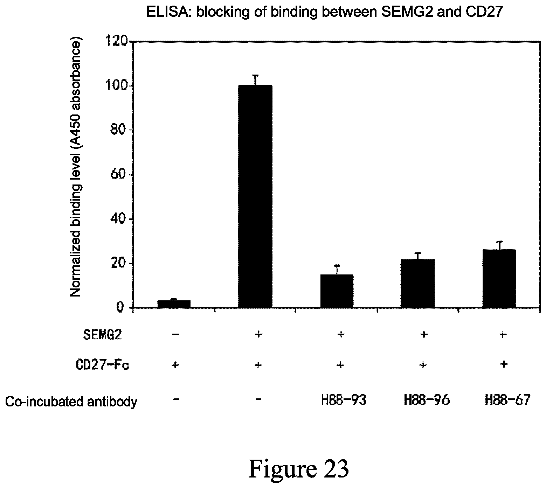

**FIGURE 23** depicts the result of ELISA, which shows the effect of different human antibodies H88-93, H88-96 and H88-67 blocking the binding between SEMG2 and CD27. All antibody concentrations are 10 μg/mL. Antibody clones H88-93, H88-96 and H88-67 are all fully human antibodies screened in the natural phage library using the SEMG2 (497-509) epitope.

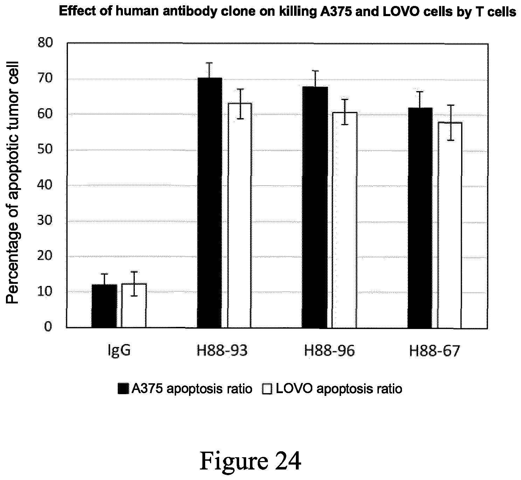

**FIGURE 24** depicts the degree of killing of co-cultured A375 and LOVO tumor cells by T cells, and the influence of human antibodies H88-93, H88-96, and H88-67 on the killing effect. The result demonstrates that the three antibodies against SEMG2 (497-509) epitope significantly promote killing SEMG2-expressing tumor cells by T cells.

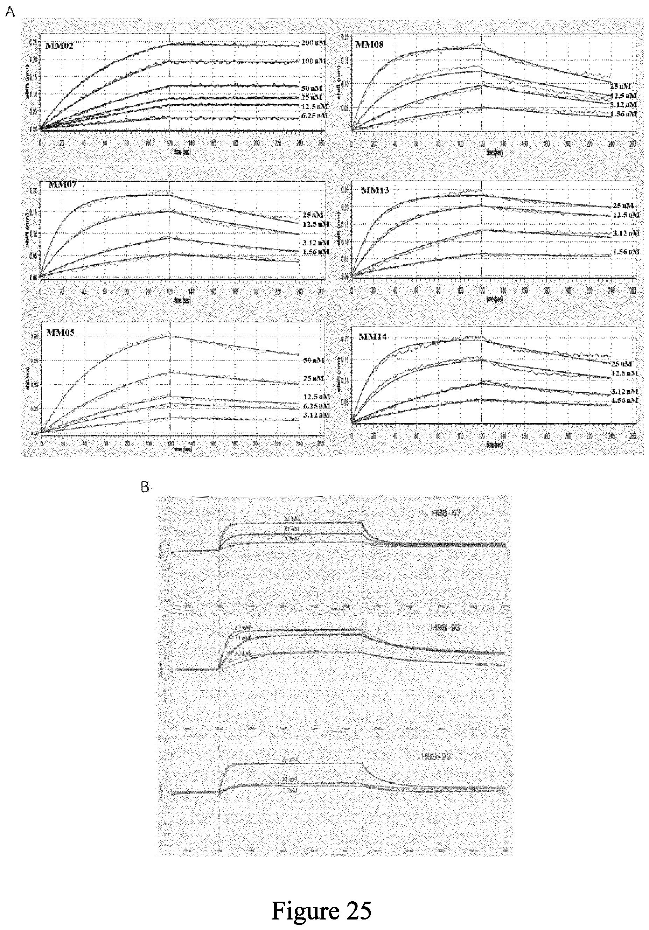

**FIGURE 25** depicts the binding between SEMG2 and fully human antibody molecules determined by Bio-Layer Interferometry shows the changes in the binding and dissociation of fully human antibodies in solution to the SEMG2 protein molecules immobilized on the biosensor, based on which the affinity constant between the fully human antibody and SEMG2 is calculated.

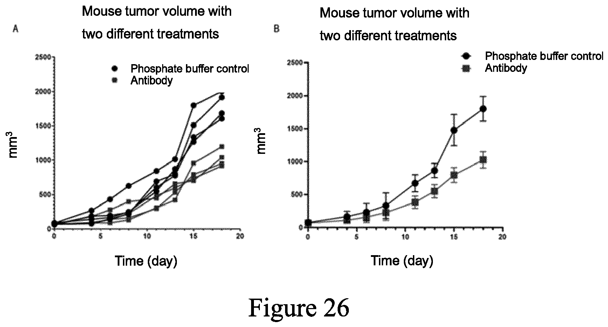

**FIGURE 26** depicts the SEMG2 antibody significantly inhibits tumor growth in the A375 melanoma mouse *in vivo* model.

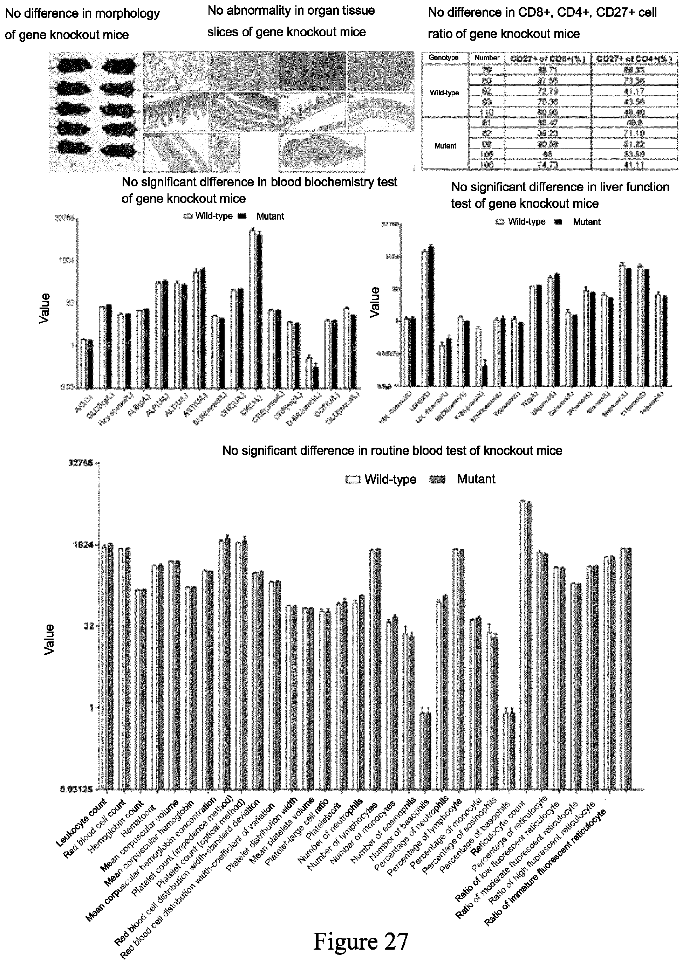

**FIGURE 27** depicts the phenotypic analysis results of the homozygous knockout of the mouse gene Svs3a corresponding to human SEMG2 compared to wild-type mice, including specific results of gross morphology, biopsy examination of various tissues and organs, ratio analysis of different subtypes of T lymphocytes, blood biochemistry, liver function and routine blood tests.

**Detailed Description**

The following makes further explanations to the invention by detailed description.

Unless defined otherwise, all technical and scientific terms used herein have the same meaning as commonly understood by one of ordinary skill in the art to which the invention pertains.

In this application, the singular forms "a", "an" and "the" include plural reference, unless the context clearly dictates otherwise.

As used herein, the term "subject" includes any human or nonhuman animals. The term "nonhuman primate" includes all vertebrates, such as mammals or nonmammals, for example nonhuman primates, sheep, canines, felines, equines, bovines, chickens, rats, mice, amphibians, reptiles, and the like. Unless otherwise specified, the terms "patient" and "subject" can be used interchangeably. In the present invention, a subject is preferably human.

As used herein, the term "SEMG2" is human semenogelin 2, one of the major components in human semen, secreted by seminal gland, and forms colloidal material to coat sperm cells and restrict their movement. The proteolytic enzymes and fibrinolytic enzymes secreted by the prostate gland in semen can break down the semenogelin and promote semen liquefaction, allowing sperm to move more freely. See Yoshida K, Karzai ZT, Krishna Z, Yoshika M, Kawano N, Yoshida M, et al., Cell Motil Cytoskeleton. 2009;66(2):99-108. The "SgII A" polypeptide isolated from SEMG2 protein has antibacterial activity, and the sequence is H-KQEGRDHDKSKGHFHMIVIHHKGGQAHHG-OH. It should be noted that different from the key amino acid sequence for binding between SEMG2 and CD27 described in the present invention, the antimicrobial peptide sequence is located in a completely different region of SEMG2. See Edström AM, Malm J, Frohm B, Martellini JA, Giwercman A, Mörgelin M, et al., J Immunol. 2008;181(5):3413-21. In addition, SEMG2 has also been reported to bind to zinc ions and affect the activity of prostatic proteolytic enzyme PSA. See Jonsson M, Linse S, Frohm B, Lundwall A, Malm J. Biochem J. 2005;387(Pt 2):447-53.

As used herein, the term "antibody" includes intact antibody and any antigen-binding fragment (i.e., "antigen-binding part") or the single chain thereof. "Antibody" refers to a protein containing at least two heavy (H) chains and two light (L) chains connected by disulfide bond, or its antigen-binding part. Each heavy chain consists of a heavy chain variable region (short for VH herein) and a heavy chain constant region. The heavy chain constant region consists of three domains, CH1, CH2 and CH3. Each light chain consists of a light chain variable region (short for VL herein) and a light chain constant region. The light chain constant region consists of a CL domain. The VH and VL regions can be further subdivided into high-variable regions, known as complementary decision area (CDR), scattered over more conservative regions known as framework region (FR). Each VH and VL consists of three CDRs and four FRs arranged in the following order from the amino terminus to the carboxy terminus: FR1, CDR1, FR2, CDR2, FR3, CDR3, FR4. The variable regions of heavy and light chains contain binding domains for antigen interaction.

The term "antibody" refers to immunoglobulins or their fragments or derivatives thereof, and includes any polypeptides that contain antigen binding sites, whether they are produced *in vitro* or *in vivo.* The term includes, but is not limited to, multi-clone, monoclonal, monospecific, multispecific, nonspecific, humanized, single-chain, chimeric, synthetic, recombinant, hybridized, mutation, and graft antibodies. The term "antibody" also includes antibody fragments such as Fab, F(ab') 2, Fv, scFv, Fd, dAb, and other antibody fragments that retain antigen binding function, i.e., can specifically bind to PD-1. Generally, such fragments will contain antigen binding fragments.

The terms "antigen-binding fragment", "antigen-binding domain" and "binding fragment" refer to an antibody molecule, which contains amino acids responsible for the binding between specific antibodies and antigens. For example, where the antigen is large and the antigen-binding fragment binds only a portion of the antigen. That is, the part of the antigen molecule responsible for the specific interaction with the antigen binding fragment is called "epitope" or "antigenic determinant".

An antigen-binding fragment typically comprises an antibody light chain variable region (VL) and an antibody heavy chain variable region (VH), however, it does not necessarily have to comprise both. For example, a so-called Fd antibody fragment consists only of a VH domain, but still retains some of the antigen binding functions of the intact antibody.

The term "epitope" is defined as an antigenic determinant which specifically binds/recognizes a binding fragment. Binding fragments can specifically bind/react with a conformation that is unique to the target structure or a contiguous epitope, the conformation or discontinuous epitope is characterized by that the polypeptide antigen being two or more separated discrete amino acid residues in the primary sequence, but the polypeptides are aggregated together on the surface of the molecule when they are folded into native proteins/antigens. Two or more discrete amino acid residues of an epitope exist in separate parts of one or more polypeptide chains. When the polypeptide chain folds into a three-dimensional structure, these residues gather on the surface of the molecule to form an epitope. In contrast, contiguous or linear epitopes, consisting of two or more discrete amino acid residues, are present in a single linear segment of a polypeptide chain.

The terms "treating" or "treatment" refer to both therapeutic treatment and prophylactic/preventing measures. Those in need of treatment include individuals who already have a particular medical condition, as well as those who may eventually acquire the condition.

The term "vector" as used herein refers to a molecular tool for the transport, transduction, and expression in a target cell of a contained exogenous gene of interest (for example, a polynucleotide according to the present invention). The tool provides a suitable nucleotide sequence that initiates transcription, i.e., the promoter.

The terms "tag protein" and "protein tag" in the present invention are interchangeable, and refer to a polypeptide or protein fused and expressed with the target protein by using DNA *in vitro* recombination technology, to facilitate protein expression, detection, tracking and purification. Tag proteins include, but not limited to, His6, Flag, GST, MBP, HA, GFP and Myc.

Examples

Unless otherwise specifically explained, the implementation methods in the following examples are all conventional methods. The invention will be further understood with reference to the following non-limiting experimental examples.

**Example 1: detection of binding betweenSEMG2 and CD27.**

Human HEK293 cells were co-transfected in a 10 cm diameter culture dish. 48 hours after co-transfection with complex including pcDNA3-Flag-SEMG2 plasmid and pcDNA3-HA-CD2 plasmid, cells were collected and lysed, CD27 in lysate was enriched by standard immunoprecipation procedure. The antibody used for immunoprecipitation was Flag antibody, and IgG nonspecific antibody was used for the control group. Immunoblotting (western blot) experiment was carried out later using HA antibody to detect the amount of co-immunoprecipitated CD27 and using Flag antibody to detect the amount of immunoprecipitated SEMG2. In immunoblotting assay, cells were lysed by Roche Complete protease inhibitor in 1%Triton X-100 (TBS pH7.6) for 30 minutes on ice, and insoluble material was pelleted by centrifugation. Lysate in SDS sample buffer with 50 mM DTT was heated to 100°C for 10 minutes, separated by SDS-PAGE and transferred to PVDF membrane (Millipore). The cell membrane was blocked in TBS with 5% bovine serum albumin (BSA) and probed with the indicated antibodies. The bands were visualized with West Pico (Thermo Fisher Scientific).

In co-immunoprecipitation experiment, cells were lysed in IP buffer (Thermo Scientific) and Roche complete protease inhibitor for 10 minutes, followed by the addition of benzonase (sigma) for 25 minutes at room temperature. The lysate was then centrifuged at 15,000 rpm at 4°C to remove the precipitate. The supernatant was then incubated with primary antibody slowly rotated overnight at 4°C, followed by the addition of protein A or protein G dynabeads and incubated at 4°C for 2 hours, washed for 4 times in PBST (PBS with 0.01% Tween 20), eluted with 50 mM DTT in SDS sample buffer for 10 minutes at 100°C, separated with SDS, and immunoblotted as previously described.

The result showed that precipitation with Flag antibody made the precipitated complex contain both SEMG2 and CD27, while the control group did not contain SEMG2 or CD27. See **FIGURE 1** for the experimental result. This experiment indicates a physical interaction between SEMG2 and human CD27. In addition, the interaction between SEMG2 and mouse CD27 was detected using the same method described above. In this experiment, mouse CD27 was used instead of human CD27 for HEK293 cell transfection, and the other experimental conditions remained unchanged. The experimental result is shown in **FIGURE 1.** This experiment indicates a physical interaction between SEMG2 and mouse CD27.

Under the above co-transfection experimental conditions, the pre-placed cell slides in a 10 cm dish were fixed, permeabilized, and blocked, and further immunolabeled with antibodies containing HA tag (mouse anti) and Flag tag (rabbit anti) simultaneously for CD27 and SEMG2, and then labeled with secondary antibodies to show red and green colors, respectively. The co-localization of SEMG2 and CD27 in cells was observed under fluorescence confocal microscopy. The results are shown in **FIGURE 2.** The co-expressed SEMG2 and CD27 proteins showed obvious co-localization in cells, and the localization patterns were even nearly identical. This is consistent with the finding that the binding between SEMG2 and CD27 proteins.

**Example 2: Binding between SEMG2(497-509) fragment and CD27protein**

To further confirm which part of SEMG2 binds to CD27, fragments of SEMG2 protein were designed. The amino acid sequence of full-length SEMG2 protein (SEQ ID NO:1) was divided into 6 segments of sequences, fused with GFP and named as SEMG2-P1, SEMG2-P2, SEMG2-P3, SEMG2-P4, SEMG2-P5 and SEMG2-P6 (See Table 1 for specific sequences, with corresponding abbreviation as P1-P6 respectively). Plasmids expressing these amino acid sequences were co-transfected with CD27 into HEK293 cells, and co-immunoprecipitation experiments were performed to identify the main fragment of SEMG2 that binds to CD27. The co-immunoprecipitation results are shown in **FIGURE 3.** Only SEMG2-P5 had significant binding to CD27, while SEMG2-P1, SEMG2-P2, SEMG2-P3, SEMG2-P4 and SEMG2-P6 did not bind to CD27. The above results indicated that the SEMG2-P5 fragment is the major part that binds to CD27.

## SEQ ID NO:1 (human SEMG2):

MKSIILFVLSLLLILEKQAAVMGQKGGSKGQLPSGSSQFPHGQKGQHYFGQKDQQHTKSKGS
FSIQHTYHVDINDHDWTRKSQQYDLNALHKATKSKQHLGGSQQLLNYKQEGRDHDKSKGHFHMI
VIHHKGGQAHHGTQNPSQDQGNSPSGKGLSSQCSNTEKRLWVHGLSKEQASASGAQKGRTQGGS
QSSYVLQTEELVVNKQQRETKNSHQNKGHYQNVVDVREEHSSKLQTSLHPAHQDRLQHGPKDIF
TTQDELLVYNKNQHQTKNLSQDQEHGRKAHKISYPSSRTEERQLHHGEKSVQKDVSKGSISIQTEE
KIHGKSQNQVTIHSQDQEHGHKENKISYQSSSTEERHLNCGEKGIQKGVSKGSISIQTEEQIHGKSQ
NQVRIPSQAQEYGHKENKISYQSSSTEERRLNSGEKDVQKGVSKGSISIQTEEKIHGKSQNQVTIPS
QDQEHGHKENKMSYQSSSTEERRLNYGGKSTQKDVSQSSISFQIEKLVEGKSQIQTPNPNQDQWS
GQNAKGKSGQSADSKQDLLSHEQKGRYKQESSESHNIVITEHEVAQDDHLTQQYNEDRNPIST

**Table 1: Corresponding amino acid sequences for construction of SEMG2 expression fragments**

| | | |
|---|---|---|
| SEMG2-P1 | GSFSIQHTYHVDINDHDWTRKSQQYDLNALHKATKSKQHLGGSQQLLNYKQEGR DHDKSKGHFHMIVIHHKGGQAHHGT | SEQ ID NO:89 |
| SEMG2-P2 | QNPSQDQGNSPSGKGLSSQCSNTEKRLWVHGLSKEQASASGAQKGRTQGGSQS SYVLQTEELVVNKQQRETKNSHQNKGHYQNVVDVREEHSSKLQTSLHPAHQDRL QHGPKDIFTTQDELLVYNKNQHQTKNLSQDQEHGR | SEQ ID NO:90 |
| SEMG2-P3 | KAHKISYPSSRTEERQLHHGEKSVQKDVSKGSISIQTEEKIHGKSQNQVTIHSQDQE HGHKENKISYQSSSTEERHLNCGEKGIQKGVSKGSISIQTEEQIHGKSQNQVRIPSQ AQ | SEQ ID NO:91 |
| SEMG2-P4 | EYGHKENKISYQSSSTEERRLNSGEKDVQKGVSKGSISIQTEEKIHGKSQNQVTIPSQ DQEHGHKENKMSYQSSSTEERRLNY GGKSTQKDVSQSSIS | SEQ ID NO:92 |
| SEMG2-P5 | FQIEKLVEGKSQIQTPNPNQDQWSGQNAKGKSGQSADSKQDLLSH | SEQ ID NO:93 |
| SEMG2-P6 | EQKGRYKQESSESHNIVITEHEVAQDDHLTQQYNEDRNPIST | SEQ ID NO:94 |
| SEMG2-P7 | QIEKLVEGKSQIQ | SEQ ID NO:3 |

To further confirm the key amino acids in the binding between SEMG2-P5 sequence and CD27, SEMG2(497-509) fragment was selected and named as SEMG2-P7 (the specific sequence is QIEKLVEGKSQIQ, abbreviated as P7 or SP7). SEMG2-P7(497-509), SEMG2-P5(positive control), SEMG2-P4 (negative control) or SEMG2-P6 (negative control) was co-transfected with CD27 into HEK293 cells respectively, including human CD27 and mouse CD27. The result of co-immunoprecipitation experiment performed later showed that both SEMG2-P7 and SEMG2-P5 bind to CD27, and the results of human CD27 and mouse CD27 were the same. The experimental results are shown in **FIGURE 4.** This co-immunoprecipitation experiment confirmed that SEMG2(497-509) is the main structure that binds to human and mouse CD27.

**Example 3: Precise characterization of the key amino acids of SEMG2 binding to CD27 using glycine scanning method**

To characterize the epitope of SEMG2 binding to CD27 with higher resolution, and to demonstrate the contribution of each amino acid of SEMG2(497-509) to binding CD27 protein more accurately, each amino acid of SEMG2(497-509) was replaced one by one with glycine, and the resulting sequences are mutant amino acid sequences numbered 1-13 (see **FIGURE 5**). These mutant plasmids and CD27 expression vector were co-transfected into HEK293 cells, and the degree of GFP-fused 1-13 polypeptide variants binding to CD27 was detected by co-immunoprecipitation assay. The experimental results are shown in **FIGURE 5,** wherein mutants 5 and 9 completely lost the binding to CD27; mutants 11 and 13 did not affect the binding of SEMG2 (497-509) to CD27; mutants at other sites (1, 2, 3, 4, 6, 7, 8, 10, 12) weakened the binding between SEMG2(497-509) and CD27 to some extent. Therefore, it can be seen that the amino acids at positions 497, 498, 499, 500, 501, 502, 503, 504, 505, 506 and 508 of SEMG2 have obvious effects on the binding to CD27. The peptide sequence 497-509 was coupled to BSA, and coated onto a 96-well microplate. CD27-hFc at different concentrations was used as the primary antibody to detect the ability of CD27 to bind to the peptide sequence. The experimental result is shown in **FIGURE 6.** The result demonstrates that the effect of CD27 concentration on binding to SEMG2 does exist.

**Example 4: SEMG2 expressed by tumor cells inhibits effect of killing tumors by immune cells**

T cell-mediated killing assay of tumor cells. HCT116 human colorectal cancer cells were stably transfected with SEMG2 expression vector or control empty vector, and the proportion of apoptotic cells after co-culturing with activated PBMCs was determined by caspase3/7 lysis assay (green fluorescence assay). Specifically, HCT116 cells stably expressing SEMG2 were seeded in 96-well plate. Human peripheral blood mononuclear cells (PBMC; #70025, Stem Cell) were activated with 100 ng/mL of CD3 antibody, 100 ng/mL of CD28 antibody, and 10 ng/mL of IL2 (#317303; #302913; #589102, BioLegend) respectively, and co-cultured with the colorectal cancer cells (#4440, Essen Bioscience) at a ratio of 10:1 in presence of fluorescent caspase-3/7 substrate. After 10 hours, cells were observed under a fluorescence microscope. The result is shown in **FIGURE 7.** Compared to control cells, tumor cells overexpressing SEMG2 exhibited significantly reduced apoptosis after co-culture with activated PBMCs. The result of this experiment supports that SEMG2 has a role in suppressing immune cell function.

**Example 5: Detection of SEMG2 expression in different tumor cells**

Different types of human tumor cells, including LOVO colorectal cancer, RKO colorectal cancer, PC3 prostate cancer, A375 malignant melanoma, SW1116 colorectal cancer, DLD1 colorectal cancer, HEK293 human renal epithelial cell line, HepG2 hepatocellular carcinoma, NCM460 human normal colonic epithelial cells, NCI-H1975 human non-small cell lung adenocarcinoma, CaCo2 colonic adenocarcinoma, HT29 colorectal adenocarcinoma, SW1990 human pancreatic adenocarcinoma, AGS human gastric adenocarcinoma, SW480 colorectal cancer, SaOS2 osteosarcoma, GES-1 human gastric mucosal cells, and so like, were incubated with DMEM medium containing 10% calf serum in cell incubator with 5% carbon dioxide at 37°C.

In immunoblotting assay, cells were lysed by Roche complete protease inhibitor in 1%Triton X-100 (TBS pH7.6) for 30 minutes on ice, and insoluble material was pelleted by centrifugation. Lysate in SDS sample buffer with 50 mM DTT was heated to 100°C for 10 minutes, separated by SDS-PAGE and transferred to PVDF membrane (Millipore). The cell membrane was blocked in TBS with 5% bovine serum albumin (BSA) and probed with the primary antibodies specifically against SEMG2 and internal control GAPDH, respectively. The primary antibodies were labeled with HRP-conjugated secondary antibodies. The bands were visualized with West Pico (Thermo Fisher Scientific). The results are shown in **FIGURE 8,** indicating that SEMG2 was not expressed in GES-1 human gastric mucosal cells and NCM460 human normal colonic epithelial cells, but observably expressed in multiple types of malignant tumor cells, including LOVO colorectal cancer, RKO colorectal cancer, PC3 prostate cancer, A375 malignant melanoma, SW1116 colorectal cancer, HEK293 human renal epithelial cell line, HepG2 hepatocellular carcinoma, CaCo2 colonic adenocarcinoma, HT29 colorectal adenocarcinoma, AGS human gastric adenocarcinoma, SW480 colorectal cancer and SaOS2 osteosarcoma. This result demonstrates that SEMG2 is a protein ubiquitously expressed in tumors.

**Example 6: Detection of SEMG2 expression in different tumor cells using immunohistochemistry (IHC)**

For immunohistochemical staining, we obtained tissue chips of various tumors from Shanghai Xinchao Biotechnology Company. Briefly, tissue specimens were incubated with anti-SEMG2 antibody (HPA042767, purchased from Sigma Aldrich, 1:100 dilution) and a biotin-conjugated secondary antibody, followed by incubation with an anti-biotin-biotin-peroxidase complex, and observed with chromophoric reagent aminoethylcarbazole. As the histological score, staining intensities were divided into four groups: high (3), moderate (2), low (1), and negative (0).

First, the expression of SEMG2 in tissue chips of colorectal cancer tumor was stained, and normal colorectal tissue was used as control; it was found that there was extensively high expression of SEMG2 in colorectal cancer tissue. The result is shown in **FIGURE 9.**

Next, the expression of SEMG2 in different lung cancer tissues was stained, and normal lung tissue was used as control; it was found that there was extensively high expression of SEMG2 in lung cancer tissues. The results are shown in **FIGURE 9.**

Again, the positive expression of SEMG2 in prostate cancer, melanoma, and gastric cancer was stained, and the results are shown in **FIGURE 9.**

Finally, based on the tissue chip staining, the positive rate of SEMG2 expression in the different tumor types indicated was calculated. Positive expression was defined as moderate or strong positive expression in immunohistochemical staining. Statistical results based on tissue chips (each chip comprises more than 50 tissue samples) are shown as a percentage in **FIGURE 9.**

**Example 7: Demonstration of the association between high SEMG2 expression and poor tumor prognosis**

In immunohistochemical staining, we obtained tissue chips of various tumors from Shanghai Xinchao Biotechnology Co., Ltd., all with follow-up data of survival time information. Immunohistochemical detection was performed by the method described in Example 6. Taking colorectal cancer as an example, the patients were classified according to the expression of SEMG2, and divided into two groups: high SEMG2 (immunohistochemical score of 2, 3) and low SEMG2 (immunohistochemical score of 0, 1). The Kaplan-Meier method was used to compare the overall survival of the two patient groups. The results are shown in **FIGURE 10.** It was found that the survival of patients with high SEMG2 expression was significantly shorter than that of tumor patients with low SEMG2 expression. There was also such significant correlation for other tumors such as lung cancer (P<0.05) and gastric cancer (P<0.05). The above results suggest that SEMG2 is a key molecule for tumor immune evasion and may serve as a new anti-tumor target.

**Example 8: Confirmation of the correlation between high SEMG2 expression and infiltration of Regulatory T cells (Treg) with immunosuppressive function**

To analyze the correlation between SEMG2 expression in tumor tissues and infiltration of Regulatory T cells (Treg) with immunosuppressive function, we examined a variety of tumor tissue chips purchased from Shanghai Xinchao Biotechnology Co., Ltd using immunohistochemistry assay. Take lung cancer for example, the infiltration of Treg in tumor tissues (labeled by Foxp3 antibody) was compared according to the expression of SEMG2. It was found that the higher the expression of SEMG2 was, the more Treg infiltrated (there was a statistically significant difference among the tissues, P<0.05), see **FIGURE 11.** The result demonstrates that the expression of SEMG2 is significantly correlated with the local immune microenvironment of the tumor, and the expression of SEMG2 can be used as a biomarker of immunosuppression status and a companion diagnostic marker for tumor immunomodulators.

**Example 9: Preparation of antibody using SEMG2 (497-509) fragment as immunogen**

Specifically, the following steps are included: (1) antigen preparation, synthesize polypeptide according to SEMG2 (497-509), i.e., the "QIEKLVEGKSQIQ" sequence, and couple to VLP carrier for immunization; use full-length SEMG2 protein (purchased from Cusabio, Cat. No. CSB-YP021002HU) as immunogen for another group. (2) The first immunization: remove part of the rabbit hair on both hind paws of the rabbit using a pair of scissors, and disinfect the skin with alcohol and iodine. Aspirate 1 mL of antigen solution emulsify by Freund's complete adjuvant (FCA) using a 2 mL syringe, and inject 0.5 mL of which into each sole of the feet subcutaneously. (3) The second immunization: After an interval of 10-14 days, inject the antigen solution into the swollen lymph nodes on bilateral fossa and groin, 0.1 mL for each lymph node and 1 mL for the rest under the skin near the lymph nodes. If the lymph nodes are not swollen or the swelling is not obvious, directly inject it into bilateral fossa and subcutaneous of groin. (4) After an interval of 7-10 days, collect 0.5-1.0 mL of blood from the ear vein, separate the serum, and determine the serum titer using indirect ELISA which coated with 10 $\mu$g/mL of antigen. Collect the blood if the titer is 1:64,000 or more. (5) If the titer does not meet the requirements, inject the antigen liquid without adjuvant into the ear vein for immunization. Which is, inject for 3 times within 1 week, 0.1, 0.3 and 0.5 mL for each time, respectively. Repeat the blood test after an interval of 1 week. If the titer meets the requirement, take the blood immediately, and collect all the antiserum.

The specific experimental steps for polyclonal antibody purification comprise: (1) Preparation of protein A sepharose CL-4B affinity column. To prepare 10 mL of protein A sepharose CL-4B packing, mix equal volume of packing and TBS buffer solution in a vacuum flask, stir and vacuum for 15 minutes to remove air bubbles in the packing. Slowly add Protein A sepharose CL-4B packing into the glass column using the pump to control the filling speed at 1 mL/min-2 mL/min, avoid column dryness, and use 10 times the bed volume of pre-cooled TBS buffer solution to equilibrate the column. (2) Preparation of antiserum. Slowly thaw the antiserum in ice water or in a 4°C freezer to avoid protein aggregation. Aggregates appeared during protein thawing process can be dissolved by preheating at 37°C. Add solid sodium azide to a concentration of 0.05%, centrifuge at 15,000 $\times$ g for 5 minutes at 4°C, remove the clarified antiserum and filter through a filter to remove excess lipids. (3) Affinity chromatography. Dilute the antibody with TBS buffer solution at 1:5 and filtered through a filter. Load the antiserum onto the column at a speed of 0.5 mL/min. To ensure the binding of the antiserum to the packing, the column should be loaded continuously for 2 times and the loading effluent should be kept. Wash the column with TBS buffer solution until A$\lambda$280nm<0.008, add pH 2.7 elution buffer solution, and elute at a speed of 0.5mL/min until all proteins flow down. Use a 1.5mL EP tube with 100$\mu$L of neutralizing buffer solution added to collect the eluate in separate tubes. After mixing, check the pH of the eluate with pH test paper. If the pH is lower than 7, use the neutralization buffer to adjust to about pH 7.4 to prevent antibodies denaturation. Add 10 mL of elution buffer solution, pH 1.9, into the column, and collect the eluate until A$\lambda$280nm<0.008 according to the method. The protein content in each tube was determined using a spectrophotometer.

**Example 10: Blocking effect comparison between SEMG2 (497-509) and full-length SEMG2 as immunogens in antibody preparation**

Because SEMG2 (497-509) sequence fragment is the key epitope of SEMG2 binding to CD27, and has a relatively short sequence, so SEMG2 (497-509) was used as an immunogen to prepare antibodies, which is theoretically easier to obtain functional antibody molecules with the function of blocking the binding between SEMG2 and CD27 than using full-length SEMG2 to prepare antibodies. For direct comparison, the differences in effective concentration of producing antibody by the two methods were verified using ELISA in the examples. Antibodies produced with SEMG2 (497-509) as immunogen and antibodies produced with full-length SEMG2 were added into the enzyme-linked immunosorbent assay (ELISA) reaction system at different concentrations (10^-2, 10^-1, 10^0, 10^1, 10^2, 10^3, 10^4 ng/mL), and the ELISA binding valueswere measured. The specific steps of enzyme-linked immunosorbent assay are as follows: (1) Dissolve SEMG2 protein antigen with 50 mM carbonate coating buffer (pH 9.6) to make the antigen concentration 10 $\mu$g/mL, and add into 96-well ELISA plate (purchased from Corning) at 100 $\mu$L/well, place at 4°C overnight. (2) After discarding the coating solution on the next day, wash with PBST for three times, add 150 $\mu$L of 1% BSA to each well, and block for 2 hours at 37°C. (3) After washing for 3 times with PBST, add the indicated antibodies (polyclonal antibody produced with SEMG2 (497-509) as immunogen, polyclonal antibody produced with full-length SEMG2 as immunogen) to each well to make different final concentrations as shown in **FIGURE 12,** add 10 $\mu$g/mL of CD27-Fc fusion protein (i.e., the extracellular region of human CD27 protein fused to the Fc fragment of human antibody), and incubate at 37° C for 2 hours. (4) After washing for 5 times with PBST, add 100 $\mu$l of diluted HRP-labeled anti-human Fc secondary antibody, and incubate at 37° C for 1 hour. (5) After washing with PBST for 5 times, 20 min after developing with chromogenic agent, the A450 absorption value was read using the microplate reader..
The experimental results are shown in **FIGURE 10.** The antibody produced by SEMG2 (497-509) as an antigen reduced the binding between SEMG2 and CD27 detected by ELISA by 50% at a lower concentration, while the polyclonal antibody produced by full-length protein of SEMG2 as immunogen only exerted such an effect at higher concentration (the required dose is more than 300 times the former). That is, the blocking titer of the antibody produced by SEMG2 (497-509) was more than 300 times higher than that of the antibody produced by the full-length SEMG2 protein. This indicates that recognition of the key epitope SEMG2 (497-509) makes the development of blocking antibodies easier, and enables those skilled in the art to obtain antibodies that can block the binding between SEMG2 and CD27 more easily.

**Example 11: Preparation of mouse monoclonal antibody using SEMG2 (497-509) epitope peptide and SEMG2 full-length protein**

The SEMG2 (497-509) sequence was used to synthesize a polypeptide and coupled to a VLP carrier for immunization; HEK293 cells were used to express the full-length SEMG2 protein, and the purity was tested to reach 92%, and the binding activity of SEMG2 protein to CD27 was verified by ELISA. The protein and polypeptide antigens were used to immunize 10 mice respectively, and multiple immunizations were performed to enhance the effect: (1) The first immunization, 50 $\mu$g/mice of antigen, multiple subcutaneous injections together with Freund's complete adjuvant, with an interval of 3 weeks; (2) the second immunization; the same dosage and route as above, with incomplete Freund's adjuvant, and with an interval of 3 weeks; (3) the third immunization, the same dosage as above, without adjuvant, intraperitoneal injection with an interval of 3 weeks; (4) the booster immunization, the dose is 50 $\mu$g, intraperitoneal injection. 3 days after the last injection, blood was collected to measure its titer and the immune effect, and mice with higher titers were selected for hybridoma fusion screening. After subcloning, the binding of the monoclonal antibodies to the target antigens was detected by ELISA, and the function of different monoclonal antibodies in blocking the binding between SEMG2 and CD27 was measured by ELISA.

The monoclonal antibodies produced by hybridomas were screened by ELISA. Among the monoclonal antibodies prepared with SEMG2(497-509) as immunogen, 19 strains had blocking function (inhibiting the binding between SEMG2 and CD27) in the first batch of 27 stains of antibodies, as shown in **FIGURE 13**. Among the monoclonal antibodies prepared with the full-length protein of SEMG2 as immunogen, only 1 stain of antibody with blocking function was obtained in a total of 108 stains of antibodies after verification in batches, as shown in **FIGURE 13**.

Therefore, preparing monoclonal antibodies using SEMG2 (497-509) epitope peptide as immunogen significantly improved the efficiency of finding blocking antibodies. The subtypes (Table 2) and sequences (Table 3) of murine monoclonal antibodies are shown in following tables.

**Table 2. Subtypes of murine monoclonal antibodies**

| Antibody clone ID | Subtype | Light chain |
|---|---|---|
| MM02 | mIgG2b | kappa |
| MM05 | mIgG1 | kappa |
| MM07 | mIgG2b | kappa |
| MM08 | mIgG1 | kappa |
| MM13 | mIgG1 | kappa |
| MM14 | mIgG2b | kappa |
| MM15 | mIgG2a | kappa |

**Table 3. Heavy and light chain variable region sequences of murine monoclonal antibodies**

| VH amino acid sequences are as follows: | |
|---|---|
| MM02 | QIQLVQSGPEVKKPGETVRISCKASGYTLTTAGIQWVQKMPGKGLKWIGWINTHSGVPEYAEDFKGRFAFF LETSASTAYLQISNLKNEDTATYFCARLGLLGYWGQGTTLTVSS (SEQ ID NO: 35) |
| MM05 | QVQLQQPGAELVRPGASVKLSCEASGYTFTSYWMNWVKQRPGQGLEWIGMIDPSDSETHYNQMFKDK ATLTVDKSSSTAYMQLSSLTSEDSAVYYCARYLGGKEGSFDYWGQGTTLTVSS (SEQ ID NO: 36) |
| MM07 | MDWLWTLLFLMAAAQSIQAQIQLVQSGPELKKPGETVRISCKASGYTLTTAGMQWVQKIPGKGLKWIGW INTHSGVAEFAEDFKGRFAFSLETSANTAYLQIRNLKNEDTATYFCARLGLLGYWGQGTTLTVSS (SEQ ID NO: 37) |
| MM08 | QVQLQQPGAELVRPGASVKLSCKSSDYTFTRYWMNWVKQRPGQGLEWIGMIDPSDSETHHNQMFKDK ATLTVDKSSSTAYMQLSSLTSEDSAVYFCARYLGGKEGSFDYWGQGTTLTVSS (SEQ ID NO: 38) |
| MM13 | EVQLQQSGAELVRSGASVKLSCTASGFNIKDYYMHWMKQRPEQGLEWIGWIDPENGDNEYAPKFQGKAT MTADTSSNTAYLQLSSLTSEDTAVYYCNVGGAHYWGQGTTLTVSS (SEQ ID NO: 39) |
| MM14 | QVQLKESGPGLVAPSQSLSITCTVSGFSLTSYAVSWVRQPPGKGLEWLGIIWGDGSTNYHSALISRLSISKDN SKSQVFLKLNSLQTDDTATYYCAKQERFSDGYYDGFAYWGQGTLVTVSA (SEQ ID NO: 40) |
| MM15 | QVQLKESGPGLVAPSQSLSITCTVSGFSLTRYGVSWVRQTPGKGLEWLGIIWGDGSTNYHSALISRLSISKDN SKSQVFLKLNSLQTDDTATYYCAKQERFSDGYYDGFAYWGQGTLVTVSA (SEQ ID NO: 41) |
| VL amino acid sequences are as follows: | |
| MM02 | DILLTQSPAILSVSPGERVSFSCRASQSIGTTIHWYQQRTNGSPRLLIKYASESISGIPSRFSGSGSGTDFTLSINS VESEDIADYYCQQSNSWPWTFGGGTKLEIKRA (SEQ ID NO: 42) |

(continued)

| VL amino acid sequences are as follows: | |
|---|---|
| MM05 | DIVMSQSPSSLAVSAGEKVTMSCKSSQSLLNSRTRKNYLAWYQQKPGQSPKLLIYWASTRESGVPDRFTGS GSGTDFTLTISSVQAEDLAVYYCKQSYSLPWTFGGGTKLEIKRA (SEQ ID NO: 43) |
| MM07 | MVSSAQFLVFLLFWIPASRGDILLTQSPAILSVSPGERVSFSCRASQSIGTTIHWYQQRTNGSPRLLIKYASESI SGIPSRFSGSGSGTDFTLSINSVESEDIADYYCQQSNSWPWTFGGGTKLEIKRA {SEQ ID NO: 44} |
| MM08 | DIVLTQSPSSLAVSAGERVTMSCKSSQSLFNSRTRKNYLAWYQQKPGQSPKLLLYWASTRESGVPDRFTGSG SGTDFTLTISSVKTEDLAVYYCKQSYELPWTFGGGTKLEMKRA (SEQ ID NO: 45) |
| MM13 | DVVMTQTPLSLPVSLGDQASISCRSSQSLVHSNGNTYLHWYLQKPGQSPKLLIYKVSNRFSGVPDRFSGSGS GTDFTLKISRVEAEDLGVYFCSQSTHVPYTFGGGTKLEIKRA (SEQ ID NO: 46) |
| MM14/MM 15 | QIVLTQSPAIMSASPGEKVTITCSASSSVSYMHWFQQKPGTSPKLWIYSTSNLASGVPARFSGSGSGTSYSLTI SRMEAEDAATYYCQQRSSYPFTFGSGTKLEIKRA {SEQ ID NO: 47} |

Table 4. CDR amino acid sequences of mouse antibodies

| VH CDR sequence -IMGT analysis | | | |
|---|---|---|---|
| Antibody | CDR1 | CDR2 | CDR3 |
| MM02 | GYTLTTAG (SEQ ID NO: 6) | INTHSGVP (SEQ ID NO: 12) | ARLGLLGY (SEQ ID NO: 17) |
| MM05 | GYTFTSYW (SEQ ID NO: 7) | IDPSDSET (SEQ ID NO: 13) | ARYLGGKEGSFDY (SEQ ID NO: 18) |
| MM07 | GYTLTTAG (SEQ ID NO: 6) | INTHSGVA (SEQ ID NO: 16) | ARLGLLGY (SEQ ID NO: 17) |
| MM08 | DYTFTRYW (SEQ ID NO: 8) | IDPSDSET (SEQ ID NO: 13) | ARYLGGKEGSFDY (SEQ ID NO: 18) |
| MM13 | GFNIKDYY (SEQ ID NO: 9) | IDPENGDN (SEQ ID NO: 14) | NVGGAHY (SEQ ID NO: 19) |
| MM14 | GFSLTSYA (SEQ ID NO: 10) | IWGDGST (SEQ ID NO: 15) | AKQERFSDGYYDGFAY (SEQ ID NO: 20) |
| MM15 | GFSLTRYG (SEQ ID NO: 11) | IWGDGST (SEQ ID NO: 15) | AKQERFSDGYYDGFAY (SEQ ID NO: 20) |
| VL CDR sequence -IMGT analysis | | | |
| Antibody | CDR1 | CDR2 | CDR3 |
| MM02 | QSIGTT (SEQ ID NO: 21) | YA (SEQ ID NO: 26) | QQSNSWPWT (SEQ ID NO: 30) |
| MM05 | QSLLNSRTRKNY (SEQ ID NO: 22) | WA (SEQ ID NO: 27) | KQSYSLPWT (SEQ ID NO: 31) |
| MM05-2 | QSLLNSRTRKNY (SEQ ID NO: 22) | WA (SEQ ID NO: 27) | QQSYSLPWT (SEQ ID NO: 95) |
| MM07 | QSIGTT (SEQ ID NO: 21) | YA (SEQ ID NO: 26) | QQSNSWPWT (SEQ ID NO: 30) |
| MM08 | QSLFNSRTRKNY (SEQ ID NO: 23) | WA (SEQ ID NO: 27) | KQSYELPWT (SEQ ID NO: 32) |

(continued)

| VL CDR sequence -IMGT analysis | | | |
|---|---|---|---|
| Antibody | CDR1 | CDR2 | CDR3 |
| MM13 | QSLVHSNGNTY (SEQ ID NO: 24) | KV (SEQ ID NO: 28) | SQSTHVPYT (SEQ ID NO: 33) |
| MM14 | SSVSY (SEQ ID NO: 25) | ST (SEQ ID NO: 29) | QQRSSYPFT (SEQ ID NO: 34) |
| MM15 | SSVSY (SEQ ID NO: 25) | ST (SEQ ID NO: 29) | QQRSSYPFT (SEQ ID NO: 34) |

The ELISA plate was coated with SEMG2 protein, and the serially diluted murine monoclonal antibody was used as the primary antibody, and the anti-mouse secondary antibody was used to detect the binding abilities of the murine monoclonal antibodies to SEMG2. The results are shown in **FIGURE 14A.** It is shown that the murine monoclonal antibodies have fine affinities for SEMG2 protein.

**Example 12: Humanization of anti-SEMG2 mAb**

The mouse anti-SEMG2 monoclonal antibody MM05 was humanized to reduce immunogenicity when used in human patients. The sequences of the heavy and light chain variable regions (VH and VL) were compared to human antibody sequences in Protein Data Bank (PDB) and homology models were established. The CDRs in the heavy and light chains of mouse mAbs were transplanted to human frame regions that most likely maintain the proper structure required for antigen binding. Reverse mutations or other mutations from human residues to mouse residues were designed when necessary, for example: the amino acid at position 95 of the humanized light chain VL-V2 was mutated from K to Q, and the corresponding CDR3 sequence of the light chain was converted to QQSYSLPWT (SEQ ID NO:95) according to IMGT analysis. Humanized VH and VL regions were fused to the constant regions of heavy chain and κ light chain of human IgGl, respectively. Transient transfections were performed in 293E cells using the construction vectors corresponding to mAb sequences, and the binding abilities of the purified mAbs to SEMG2 protein were analyzed using ELISA. Results are shown in absorbance, where higher absorbance indicates a higher level of interaction between the humanized antibody and SEMG2. The amino acid sequences of CDRs, light chain variable regions and heavy chain variable regions, light chains and heavy chains of the 8 humanized antibodies obtained in the present invention are shown in **Table 4** and **Table 5** below. **FIGURE 14B** shows the fitting curves of the binding of serially diluted humanized monoclonal antibody to SEMG2 protein, and the result show that the humanized antibody maintains the binding ability of murine monoclonal antibody to SEMG2 protein.

**Table 5. VH and VL amino acid sequences of MM05 humanized antibody**

| VH amino acid sequences are as follows: | |
|---|---|
| VH_V1 | QVQLVQSGAEVKKPGASVKVSCKASGYTFTSYWMNWVRQAPGKGLEWVGMIDPSDSETHYNQMFKD RVTITADKSTSTAYMELSSLRSEDTAVYYCARYLGGKEGSFDYWGQGTLVTVSS (SEQ ID NO: 48) |
| VH_V2 | QVQLVQSGAEVKKPGASVKVSCKASGYTFTSYWMNWVRQAPGKGLEWVGMIDPSDSETHYNQMFKD RVTITVDKSTSTAYMELSSLRSEDTAVYYCARYLGGKEGSFDYWGQGTLVTVSS (SEQ ID NO: 49) |
| VH_V3 | QVQLVQSGAEVKKPGASVKVSCKASGYTFTSYWMNWVRQAPGQGLEWVGMIDPSDSETHYAQKFQG RVTITVDKSTSTVYMELSSLRSEDTAVYYCARYLGGKEGSFDYWGQGTLVTVSS (SEQ ID NO: 50) |
| VH_V4 | QVQLVQSGAEVKKPGASVKVSCKASGYTFTSYWMNWVRQAPGQGLEWVGMIDPSDSETHYAQKFQG RVTITADKSTSTVYMELSSLRSEDTAVYYCARYLGGKEGSFDYWGQGTLVTVSS (SEQ ID NO: 51) |
| VL amino acid sequences are as follows: | |
| VL_V1 | DIVMTQSPDSLAVSLGERATINCKSSQSLLNSRTRKNYLAWYQQKPGQPPKLLIYWASTRESGVPDRFSGS GSGTDFTLTISSLQAEDVAVYYCKQSYSLPWTFGGGTKVEIK (SEQ ID NO: 52) |
| VL_V2 | DIVMTQSPDSLAVSLGERATINCKSSQSLLNSRTRKNYLAWYQQKPGQPPKLLIYWASTRESGVPDRFSGS GSGTDFTLTISSLQAEDVAVYYCQQSYSLPWTFGGGTKVEIK (SEQ ID NO: 53) |

**Example 13: Functional comparison between SEMG2 (497-509) epitope-specific antibodies and other epitope-specific antibodies in blocking SEMG2 and CD27 binding**

To demonstrate the importance of the SEMG2 (497-509) epitope for the preparation of blocking antibodies, the functions of antibodies against different SEMG2 epitopes in blocking binding between SEMG2 and CD27 were further compared. It is known that the existing commercial antibodies of HPA042767 and HPA042835 (both purchased from Sigma Aldrich Company) are rabbit polyclonal antibodies against epitopes of SEMG2 (354-403) and SEMG2 (563-574), respectively.

First, SEMG2(497-509) epitope-specific antibodies (for example, MM02, MM05) were compared with other epitope-specific antibodies (for example, HPA042767) for their function in blocking binding between SEMG2 and CD27 at different concentration ranges. The binding of the above antibodies to SEMG2 (497-509) epitope was confirmed by ELISA: MM02 and MM05 were able to bind to SEMG2(497-509), while HPA042767 could not bind to this epitope in a wide range of concentration, as shown in **FIGURE 15A.** The blocking function of different antibodies (irrelative mouse IgG, MM02, MM05, HPA042767) on binding between SEMG2 and CD27 was analyzed by the ELISA experiment as described in Example 11. As shown in **FIGURE 15B,** as the concentrations of MM02 and MM05 increased, the binding of SEMG2 and CD27 gradually decreased, and this phenomenon was observed for both the irrelative mouse IgG and HPA042767 antibodies, indicating that the latter does not possess the function of blocking the binding betweenSEMG2 and CD27 in a wide range of concentration. These results support the importance of the SEMG2(497-509) epitope in preparing blocking antibodies.

Further, the effects of different antibodies on the binding between SEMG2 and CD27 were compared under the condition of the same antibody concentration. In the ELISA, the same concentration (10 $\mu$g/mL) of antibody was used, and the strength of the binding between SEMG2 and CD27 was measured. The results are shown in **FIGURE 16.** MM02, MM05, MM07, MM08, MM13 and MM14 antibodies against SEMG2(497-509) epitope significantly reduced the binding between SEMG2 and CD27; while none of the HPA042767 and HPA042835 antibodies against other epitopes of SEMG2 reduced the binding between SEMG2 and CD27.

**Example 14: Effective comparison between SEMG2 (497-509) epitope-specific antibodies and other epitope-specific antibodies in tumor cell killing by activated PBMC**

In the examples, SEMG2 exerts function of inhibiting activated PBMC from killing tumor cells. Since SEMG2 may play the above role by binding to CD27, and SEMG2 (497-509) epitope is a key site for CD27 binding, SEMG2 (497-509) epitope-specific antibody may neutralize the influence of SEMG2 on tumor cell killing by PBMC.

To test the above hypothesis, the effects of different epitope-specific antibodies on tumor cell killing by activated PBMC were compared. Specifically, A375 human melanoma and LOVO human colorectal cancer cells highly expressing SEMG2 were seeded in 96-well plate. Human peripheral blood mononuclear cells (PBMC; #70025, Stem Cell) were activated with 100 ng/mL of CD3 antibody, 100 ng/mL of CD28 antibody, and 10 ng/mL of IL2 (#317303; #302913; #589102, BioLegend), respectively, and co-cultured with the above tumor cells at a ratio of 10:1 in presence of fluorescent caspase-3/7 substrate (#4440, Essen Bioscience). After 10 hours, cells were observed under fluorescence microscope. The results are shown in **FIGURE 17.** Neither HPA042767 nor HPA042835 antibody could affect activated PBMC to kill tumor cells; while MM02, MM05, MM07, MM08, MM13 and MM14 antibodies against SEMG2 (497-509) epitope significantly increased apoptosis tumor cell ratio. The above results indicate that SEMG2 (497-509) epitope-specific antibody can neutralize the activity of SEMG2 (i.e., eliminating the inhibitory effect of SEMG2 on tumor cell killing by PBMC).

**Example 15: Verifying the correlation between the expression level of SEMG2 and the promotive function of blocking antibodies in tumor cell killing by PBMC**

Since the expression of SEMG2 is a prerequisite for its inhibition of tumor-specific immunity, the expression of SEMG2 is also a potential condition for the suitability of SEMG2-blocking antibody administration. In theory, tumor cells with high SEMG2 expression will have a relative increase in the tumor cell killing by PBMC after neutralizing of SEMG2 activity; tumor cells that do not express SEMG2 may not rely on SEMG2 to play immune escape function, therefore the tumor cell killing by PBMC may not produce a significant change after neutralizing of SEMG2 activity.

To verify the above hypothesis, tumor cells with high SEMG2 expression (A375, LOVO) and SEMG2-negative tumor cells (DLD1, NCM460 and NCI-H1975) were selected. Different antibodies (irrelevant mouse IgG antibodies, MM02 or MM05 antibodies) were added during the PBMC killing experiments for the tumor cells. Results are shown in **FIGURE 18.** MM02 and MM05 antibodies significantly increased the killing of SEMG2-positive tumor cells (A375, LOVO) by activated PBMC, but has no obvious impact to the killing of SEMG2-negative tumor cells (DLD1, NCM460 and NCI-H1975). Therefore, the above experimental results indicate that the positive expression of SEMG2 is a screening condition for administration of SEMG2 and CD27 blocking antibodies, i.e., a corresponding biomarker.

**Example 16: Accurate Definition of Associated Epitopes of Antibody for Blocking the Binding Between SEMG2 and CD27**

To clearly distinguish the binding epitopes of blocking antibodies (i.e., antibodies that can inhibit the binding between SEMG2 and CD27) and non-blocking antibodies, a corresponding ELISA analysis method was established. Specifically, SEMG2 full-length protein (1-582), SEMG2(354-403) fragment, SEMG2(442-453) fragment, SEMG2(497-509) fragment and SEMG2(563-574) fragment were immobilized on the ELISA plate, and the same concentration of antibodies (MM02, MM05, MM07, MM08, MM13, MM14, HPA042767 and HPA042835) were added. Anti-mouse or anti-rabbit secondary antibodies were then used to detect the corresponding bound antibodies. The results are shown in **FIGURE 19.** MM02, MM05, MM07, MM08, MM13, and MM14 all bound to SEMG2(497-509) epitope, HPA042767 bound to SEMG2 (354-403) epitope, and HPA042835 bound to SEMG2 (497-509) epitope, while none of the antibodies bound to SEMG2 (442-453) control fragment. These results support the labelling specificity of the antibodies.

To further precisely define the exact epitopes (specific to the level of single amino acid) to which blocking antibodies MM02, MM05, MM07, MM08, MM13, MM14 bind, corresponding ELISA analysis method was established. As shown in **FIGURE 20,** SEMG2(497-509) polypeptide and a group of polypeptide sequences substituted by glycine one by one (glycine mutation scanning sequence group) were immobilized on the ELISA plate, and the same concentration of antibodies (MM02, MM05, MM07, MM08, MM13, MM14, HPA042767 and HPA042835) were added respectively. Anti-mouse or anti-rabbit secondary antibodies were then used to detect the corresponding bound antibodies. The results are shown in **FIGURE 20.** The HPA042767 and HPA042835 antibodies did not bind to the sequences, indicating the specificity of the experiment and the different epitope classes of the two types of antibodies. Meanwhile, the different amino acids in the SEMG2(497-509) sequence had different degrees of influence on the binding of similar blocking antibodies (MM02, MM05, MM07, MM08, MM13, MM14) after substitution by glycine. For example: the substitution of amino acids at positions 507 and 509 did not significantly affect the binding of MM02 and similar antibodies; the substitution of amino acids at positions 501 and 506 significantly affected the binding of MM02 and similar antibodies (a decrease of more than 70%); amino acids at other sites affected the binding of MM02 and similar antibodies to a certain extent after substitution by glycine. The results precisely define the epitope amino acids associated with MM02 and similar antibodies (i.e., antibodies that block the binding between SEMG2 and CD27), and the contribution of each amino acid to the binding. In addition, the key amino acids of SEMG2 participating in binding to blocking antibodies are highly consistent with those participating in binding to CD27, which indicates that MM02 and its similar antibodies compete with CD27 for binding to SEMG2, which verifies the molecular mechanism of antibody function.

**Example 17: Preparation and screening of fully human antibodies using SEMG2(497-509) epitope to block the binding between SEMG2 and CD27 and to promote the tumor cell killing by PBMC**

Results of the examples showed the importance of SEMG2(497-509) epitope in the preparation of blocking antibodies, and this epitope was applied to the screening of fully human antibodies. Specifically, the preparation of polypeptide antigens and the screening of human natural antibody library were firstly performed. The SEMG2(497-509) polypeptide was synthesized and coupled to BSA and KLH, respectively, and screened in a fully human phage display antibody library. ELISA was used to select clones that bind to antigenic epitopes for preliminary screening. Different unique sequences were obtained after sequencing single colonies, sorted according to affinity sorting, and full-length antibodies were constructed from antigen-binding fragments (Fab) with relatively high affinity. The binding ability and blocking function tests were performed after purification, that is, the effect of the antibody on binding between SEMG2 and CD27 was determined by the ELISA experiment.

In the same batch of screening, a total of 3 unique sequences of antibodies that bind to SEMG2 (497-509) epitope and inhibit binding of SEMG2 and CD27 were obtained. The three clones were named respectively: H88-93, H88-96 and H88-67. The effect of the corresponding full-length antibody concentration on binding SEMG2 is shown in **FIGURE 22A.** The amino acid sequences of VH and VL corresponding to the three fully human antibodies and the corresponding CDR sequences are shown in **Table 6** and **Table 7.**

**Table 6. Variable region sequences of fully human antibodies**

| VH amino acid sequences are as follows: | |
|---|---|
| H88-96 | QVQLLESGGGLVQPGGSLRLSCSASGFTFSSYAMHWVRQAPGKGLEYVSAISSNGGSTYYADSVK GRFTISRDNSKNTLYLQMSSLRAEDTAVYYCVIEGGSTTGTTSGAFDIWGQGTMVTVSS (SEQ ID NO: 54) |

(continued)

| VH amino acid sequences are as follows: | |
|---|---|
| H88-93 | QITLKESGPTLVKPTQTLTLTCNFSGFSLTTSGVGVAWIRQPPGKALEWLALIYWDDDQRYSPSLKSR LSVTKHTSKDQVVLTMTNVGPVDTATYYCAHLSYGPGWGYYMDVWGNGTMVTVSS (SEQ ID NO: 55) |
| H88-67 | QVQLLESGGGVVQPGRSLRLSCAASGFTFSSYAMHWVRQAPGKGLEWVAVISYDGSNKYYADSV KGRFTISRDNSKNTLYLQMNSLRAEDTAVYYCARMDGSGSPDYWGQGTLVTVSS (SEQ ID NO: 56) |
| VL amino acid sequences are as follows: | |
| H88-96 | DIQMIQSPPSVSASVGDTVTIACRANQGIDSWLAWYQQKPGRAPKLLIYSASRLQSGVPSRFSGGG SGTDFALTISNLQPEDFATYYCQQALSLPITFGQGTRLEIK (SEQ ID NO: 57) |
| H88-93 | EIVLTQSPGTLSLSPGERASLSCRASQSVRNNYLAWYQQKPGQAPRLLIFGASNRATGIPDTFSGSGS GTDFTLTISRLEPEDFAVYYCQQYGHSPITFGQGTRLEIK (SEQ ID NO: 58) |
| H88-67 | DIVMTQSPLSLPVTLGQPASISCRSSQSLVYSDGNTYLNWFQQRPGQSPRRLIYKVSNRDSGVPDRF SGSGSGTDFTLKISRVEAEDVGVYYCMQGTHWPPAFGQGTKVEIK (SEQ ID NO: 59) |

**Table 7. CDR amino acid sequences of human antibodies**

| VH CDR sequence -IMGT analysis | | | |
|---|---|---|---|
| Antibody | CDR1 | CDR2 | CDR3 |
| H88-96 | GFTFSSYA (SEQ ID NO: 60) | ISSNGGST (SEQ ID NO: 62) | VIEGGSTTGTTSGAFD (SEQ ID NO: 65) |
| H88-93 | GFSLTTSGVG (SEQ ID NO: 61) | IYWDDDQ (SEQ ID NO: 63) | AHLSYGPGWGYYMDV (SEQ ID NO: 66) |
| H88-67 | GFTFSSYA | ISYDGSNK | ARMDGSGSPDY |
| | (SEQ ID NO: 60) | (SEQ ID NO: 64) | (SEQ ID NO: 67) |
| VL CDR sequence -IMGT analysis | | | |
| Antibody | CDR1 | CDR2 | CDR3 |
| H88-96 | QGIDSW (SEQ ID NO: 68) | SA (SEQ ID NO: 71) | QQALSLPIT (SEQ ID NO: 73) |
| H88-93 | SQSVRNNY (SEQ ID NO: 69) | GA (SEQ ID NO: 72) | QQYGHSPIT (SEQ ID NO: 74) |
| H88-67 | QSLVYSDGNTY (SEQ ID NO: 70) | KV (SEQ ID NO: 28) | MQGTHWPPA (SEQ ID NO: 75) |

The binding abilities of fully human antibodies and murine antibodies to SEMG2 were tested, that is, murine antibodies MM02 and MM05 and concentration gradient diluted fully human antibodies H88-93 were mixed and added as primary antibodies in a 96-well microplate coated with SEMG2. Murine monoclonal antibody bound to SEMG2 was measured using anti-mouse HRP secondary antibody. The blocking percentage is calculated according to the following formula:

Blocking percentage = [1-(A450 of experimental antibody group-Blank control)/( A450 of positive control antibody - A450 of empty control)]×100%

The result shows that H88-93 competes with MM02 and MM05 for binding to SEMG2, as shown in **FIGURE 22.** It shows that fully human antibodies and murine monoclonal antibodies are the same type of antibodies binding to SEMG2, and because MM02, MM05 and H88-93 all bind to the short peptide SEMG2 (497-509), this type of antibodies can be defined as a class of SEMG2 (497-509) binding antibodies.

Furthermore, the effect of human antibodies H88-93, H88-96 and H88-67 on blocking the binding between SEMG2 and CD27 was detected by ELISA. All antibodies at a concentration of 10 μg/mL inhibited the binding between SEMG2 and CD27 in varying degrees, as shown in **FIGURE 23.**

To verify the effect of the human antibodies on the function of activated PBMC on tumor cell killing, A375 and LOVO cells were co-cultured with activated PBMC, and H88-93, H88-96, or H88-67 antibody was added at the same time, and the apoptosis ratio of tumor cells were detected. The result is shown in **FIGURE 24,** which indicates that the three fully human antibodies against SEMG2 (497-509) epitope can significantly promote the killing of SEMG2-expressing tumor cells by PBMC cells.

## Example 17: Binding kinetic determination of the monoclonal antibodies of the present invention to antigens by bio-optical interferometry

The equilibrium dissociation constant (KD) of the antibody of the present invention binding to human SEMG2 was determined by biolayer interferometry (ForteBio Bltz or Gator instrument). For example, the ForteBio affinity assay was performed according to the existing method, that is, half an hour before start, an appropriate amount of AMQ (Pall, 1506091) (for sample detection) or AHQ (Pall, 1502051) (for positive control detection) sensors were taken and soaked in SD buffer (PBS 1×, BSA 0.1%, Tween-20 0.05%). 100 μl of SD buffer, antibody and SEMG2 were added to a 96-well black polystyrene half area microplate, respectively. Select the sensor location based on the sample location layout. KD valueswere analyzed using molecular interaction analysis software. In the experiments of the assays, the affinity constants of murine monoclonal antibodies and human antibodies H88-67, H88-93 and H88-96 are shown in **Table 8,** and the affinity and dissociation curves of SEMG2 and corresponding proteins are shown in **FIGURE 25.**

**Table 8. Affinity constants (equilibrium dissociation constants) for the detection of antigen-antibody binding by biolayer optical interferometry**

| Antibody | KD (M) |
|---|---|
| MM02 | $1.33×10^{-9}$ |
| MM05 | $5.28×10^{-9}$ |
| MM07 | $1.82×10^{-9}$ |
| MM08 | $2.34×10^{-9}$ |
| MM13 | $6.93×10^{-10}$ |
| MM14 | $1.44×10^{-9}$ |
| H88-67 | $2.84×10^{-8}$ |
| H88-93 | $4.60×10^{-9}$ |
| H88-96 | $1.40×10^{-8}$ |

## Example 18: Affinity maturation of fully human monoclonal antibodies

Using the plasmids constructed from the VH and VL coding sequences of fully human antibodies H88-96 and H88-67 as templates, the plasmids were obtained by gene synthesis, and then made single-point and double-point saturation mutation. *In vitro* ligation method was then performed to recombine antibody genes. Finally, the Fab gene sequence of recombinant antibody was inserted into the vector, and then transformed to obtain 4 phage affinity-matured antibody libraries with titer higher than $10^8$CFU. The antibody mutant library was screened by the immunotube gradient screening assay, and the mutants with finely improved affinity compared to the wild type were obtained. The full-length affinity matured human antibody was then constructed according to the detected Fab sequence or the recombination of VH and VL sequences in the Fab sequence. The VH and VL sequences derived from H88-67 and the CDR regions of the VH sequence of H88-96 after affinity maturation are shown in **Table 9,** and CDR regions of the light and heavy chain of the antibody are shown in **Table 9.**

**Table 9. CDR sequences corresponding to affinity matured fully human antibodies**

| VH CDR sequence -IMGT analysis | | | |
|---|---|---|---|
| **VH ID** | CDR1 | CDR2 | CDR3 |
| **67-3** | GFTFSSYA (SEQ ID NO: 60) | ISYDGSNK (SEQ ID NO: 64) | ARMDNHGSPDY (SEQ ID NO: 77) |
| **67-6** | GFTFSSYA (SEQ ID NO: 60) | ISYDGSNK (SEQ ID NO: 64) | ARMDGHGSPDY (SEQ ID NO: 78) |
| **67-9** | GFTFSSYA (SEQ ID NO: 60) | ISYDGSNK (SEQ ID NO: 64) | ARMDSGGSPDY (SEQ ID NO: 79) |
| **96-10R** | GFTFSSRA (SEQ ID NO: 76) | ISSNGGST (SEQ ID NO: 62) | VIEGGSTGSTTSGAFDI (SEQ ID NO: 80) |
| **96-10V** | GFTFSSYA (SEQ ID NO: 60) | ISSNGGST (SEQ ID NO: 62) | VIEGGSTGSTVSGAFDI (SEQ ID NO: 81) |
| VL CDR sequence -IMGT analysis | | | |
| **VL ID** | CDR1 | CDR2 | CDR3 |
| **67-3** | QSLVYSDGNTY (SEQ ID NO: 70) | KV (SEQ ID NO: 28) | MQGTHWPPA (SEQ ID NO: 75) |
| **67-4** | QSLVYSDGNTY (SEQ ID NO: 70) | EV (SEQ ID NO: 83) | MQGTHWPPA (SEQ ID NO: 75) |
| **67-5** | QSLVYSDGNTY (SEQ ID NO: 70) | GV (SEQ ID NO: 84) | MQGTHWPPA (SEQ ID NO: 75) |
| **67-6** | QSLVYKDGNTY (SEQ ID NO: 82) | KV (SEQ ID NO: 28) | MQGTHWPPR (SEQ ID NO: 85) |

**Table 10. VH and VL sequences corresponding to affinity matured fully human antibodies**

| Antibody Number | Heavy chain variable region VH | Light chain variable region VL |
|---|---|---|
| **67-3-67-3** | QVQLLESGGGVVQPGRSLRLSCAASGFTFS SYAMHWVRQASGKGLEWVAVISYDGSNK YYADSVKGRFTISRDNSKNTLYLQMNSLRA EDTAVYYCARMDNHGSPDYWGQGTLVTV SS ((SEQ ID NO: 96) | DIVMTQSPLSLPVTLGQPASISCRSSQSLVYSDGNTYLN WFQQRPGQSPRRLIYKVSNRDSGVPDRFSGSGSGTDF TLKISRVEAEDVGVYYCMQGTHWPPAFGQGTKVEIK (SEQ ID NO: 59) |
| **67-3-67-4** | QVQLLESGGGVVQPGRSLRLSCAASGFTFS SYAMHWVRQASGKGLEWVAVISYDGSNK YYADSVKGRFTISRDNSKNTLYLQMNSLRA EDTAVYYCARMDNHGSPDYWGQGTLVTV SS ((SEQ ID NO:96) | DIVMTQSPLSLPVTLGQPASISCRSSQSLVYSDGNTYLN WFQQRPGQSPRRLIYEVSNRDSGVPDRFSGSGSGTDF TLKISRVEAEDVGVYYCMQGTHWPPAFGQGTKVEIK (SEQ ID NO: 101) |
| **67-3-67-5** | QVQLLESGGGVVQPGRSLRLSCAASGFTFS SYAMHWVRQASGKGLEWVAVISYDGSNK YYADSVKGRFTISRDNSKNTLYLQMNSLRA EDTAVYYCARMDNHGSPDYWGQGTLVTV SS ((SEQ ID NO: 96) | DIVMTQSPLSLPVTLGQPASISCRSSQSLVYSDGNTYLN WFQQRPGQSPRRLIYGVSNRDSGVPDRFSGSGSGTDF TLKISRVEAEDVGVYYCMQGTHWPPAFGQGTKVEIK (SEQ ID NO: 102) |
| **67-3-67-6** | QVQLLESGGGVVQPGRSLRLSCAASGFTFS SYAMHWVRQASGKGLEWVAVISYDGSNK YYADSVKGRFTISRDNSKNTLYLQMNSLRA EDTAVYYCARMDNHGSPDYWGQGTLVTV SS ((SEQ ID NO: 96) | DIVMTQSPLSLPVTLGQPASISCRSSQSLVYKDGNTYLN WFQQRPGQSPRRLIYKVSNRDSGVPDRFSGSGSGTDF TLKISRVEAEDVGVYYCMQGTHWPPRFGQGTKVEIK (SEQ ID NO: 103) |
| **67-9-67-3** | QVQLLESGGGVVQPGRSLRLSCAASGFTFS | DIVMTQSPLSLPVTLGQPASISCRSSQSLVYSDGNTYLN |

(continued)

| Antibody Number | Heavy chain variable region VH | Light chain variable region VL |
|---|---|---|
|  | SYAMHWVRQASGKGLEWVAVISYDGSNK YYADSVKGRFTISRDNSKNTLYLQMNSLRA EDTAVYYCARMDSGGSPDYWGQGTLVTV SS ((SEQ ID NO: 97) | WFQQRPGQSPRRLIYKVSNRDSGVPDRFSGSGSGTDF TLKISRVEAEDVGVYYCMQGTHWPPAFGQGTKVEIK (SEQ ID NO: 59) |
| 67-6-67-6 | QVQLLESGGGVVQPGRSLRLSCAASGFTFS SYAMHWVRQASGKGLEWVAVISYDGSNK YYADSVKGRFTISRDNSKNTLYLQMNSLRA EDTAVYYCARMDGHGSPDYWGQGTLVTV SS ((SEQ ID NO:98) | DIVMTQSPLSLPVTLGQPASISCRSSQSLVYKDGNTYLN WFQQRPGQSPRRLIYKVSNRDSGVPDRFSGSGSGTDF TLKISRVEAEDVGVYYCMQGTHWPPRFGQGTKVEIK (SEQ ID NO: 103) |
| 96-10R-10 | QVQLLESGGGLVQPGGSLRLSCSASGFTFS SRAMHWVRQAPGKGLEYVSAISSNGGSTY YADSVKGRFTISRDNSKNTLYLQMSSLRAE DTAVYYCVIEGGSTGSTTSGAFDIWGQGT MVTVSS ((SEQ ID NO: 99) | DIQMIQSPPSVSASVGDTVTIACRANQGIDSWLAWYQ QKPGRAPKLLIYSASRLQSGVPSRFSGGGSGTDFALTIS NLQPEDFATYYCQQALSLPITFGQGTRLEIK (SEQ ID NO: 57) |
| 96-10V-10 | QVQLLESGGGLVQPGGSLRLSCSASGFTFS SYAMHWVRQAPGKGLEYVSAISSNGGSTY YADSVKGRFTISRDNSKNTLYLQMSSLRAE DTAVYYCVIEGGSTGSTVSGAFDIWGQGT MVTVSS ((SEQ ID NO: 100) | DIQMIQSPPSVSASVGDTVTIACRANQGIDSWLAWYQ QKPGRAPKLLIYSASRLQSGVPSRFSGGGSGTDFALTIS NLQPEDFATYYCQQALSLPITFGQGTRLEIK (SEQ ID NO: 57) |

Through the above affinity maturation process, we obtained anti-human SEMG2 monoclonal antibodies with improved affinity, such as 67-3-67-3, 67-3-67-4, 67-3-67-5 and 67-3-67-6 which consist of the combination of the affinity-matured heavy chain numbered 67-3 and the affinity-matured light chain sequence numbered 67-3, 67-4, 67-5 and 67-6, antibody 67-9-67-3 consists of the combination of the heavy chain numbered 67-9 and the light chain numbered 67-3, antibody 67-6-67-6 consists of the combination of the light chain and heavy chain numbered 67-6, and antibodies 96-10R-10 and 96-10V- 10 reconstituted by the heavy chains numbered 96-10R and 96-10V and the light chain of H88-96L. The recombinant monoclonal antibodies consist of these light and heavy chains have an affinity more than 10-fold higher for SEMG2 and BSA-S2 (497-509) (i.e., BSA-SP7) than that of the parent antibodies (see **FIGURE 21B-D).**

**Example 19: Validation of the anti-tumor effect of SEMG2 antibody in xenograft model of PBMC-HIS model in human malignant melanoma A375 cells**

30 male NPSG mouse models aged 6-8 week were weighed. A375 cells (with confirmed endogenous expression of SEMG2) were cultured *in vitro* to obtain $1.8 \times 10^8$ cells. After 30 mice were inoculated with PBMC, A375 tumor cells were inoculated on the 3rd day. After that, the proportion of hCD45+ cells in mouse blood and the body weight were measured once a week. After inoculation, tumor volume was measured once a week, and the proportion of hCD45+ cells in mouse blood was measured when the average tumor volume reached about 40-80mm$^3$. Mice were grouped randomly based on tumor volume and the proportion of hCD45+ cells in mouse blood, and the administration was started immediately. The date began the administration was considered day 0. Dosing regimen: SEMG2 antibody (MM05 clone) was injected intraperitoneally at 5 mg/kg three times a week. After the start of administration, the tumor growth status of the mice was observed every week. After the tumor growth, the body weight and tumor volume were measured 3 times a week, and the relative count of hCD45+ cells in mouse blood was monitored by flow cytometry 3 times a week. When the tumor volume reached the end point, blood was collected and the same indexed were detected, and the experiment was ended. The observation of mice includes: daily observation, observation of animal morbidity and death every working day after inoculation. Measurement of tumor volume: after inoculation and before grouping, when tumors were visible, the tumor volume of experimental animals was measured once a week. After inoculation and grouping, the tumor volume of animals in the experiment was measured twice a week. The tumor volume was measured by a bidirectional measurement method. First, the long and short diameters of the tumor were measured with a vernier caliper, and the tumor volume was then calculated using the formula TV=0.5*a*b2, where a is the long diameter of the tumor and b is the short diameter of the tumor. The experimental results are shown in **Figure 26.** SEMG2 antibody significantly inhibited the growth of tumor in mice. This result indicates that SEMG2 is an effective anti-tumor target.

**Example 20: Knockout of the corresponding gene Svs3a in mice proves no significant side effect after function blockade of SEMG2**

To prove the possible toxic and side effects after functional blockade of SEMG2 as a drug target, the corresponding gene Svs3a in mice was knocked out systemically. The specific scheme was as follows: CRISPR/cas9 technology was adopted in the project, and non-homologous recombination was used to introduce mutation, resulting in a shift in the reading frame and loss of function of Svs3a gene. The brief process is as follows: Cas9 mRNA and gRNA were obtained by *in vitro* transcription; Cas9 mRNA and gRNA were microinjected into the fertilized eggs of C57BL/6J mice to obtain F0 generation mice. The positive F0 mice verified by PCR amplification and sequencing were mated with C57BL/6J mice to obtain positive F1 mice.
gRNAs sequence (5'-3'):

gRNA1, CAGCCGCAGAGAGGCACTCAGGG ;
gRNA2, ATGCACCACCAAGAAACACTGGG.

Sequence alignment before and after knockout:

Wild-type:

TGAGTTCAGGGAGCAGCCGCAGAGAGGCACTCAGGGAGAATGTCCATAAG
GATGCCATGGCAGTGAGAG……AGTGTCTTAGCAAACGGGAGAGCTGTCTG
CCCCAGTGTTTCTTGGTGGTGCATGGTGGGCTCCCTGTGCCCGCAGTGC ;

Mutant:

TGAGTTCAGGGAGCAGCCGCAAGAGAGG…(-
1006bp)…GAGGTGCATGGTGGGCTCCCTGTGCCCGCAGTGC。

Subsequent reproduction: the obtained gene knockout heterozygous mice (gene+/-) were divided into two parts: a part of heterozygous mice was mated with wild-type mice for expansion of more heterozygous mice; a part of heterozygous mice self-bred to obtain gene knockout homozygous mice (gene-/-) for gene knockout effect verification and subsequent phenotype analysis.

Phenotype analysis: Anticoagulated whole blood was taken from mice for flow cytometry, and the proportion of CD8+, CD4+, CD3+, CD27+ positive cells in blood was analyzed. After the mice rested for 2 days, the anticoagulated whole blood was collected from the inner canthus, and the molecule department would perform the blood routine test. After the mice rested for 3 days, the mice were weighed and anesthetized, and the mouse gross bodies were imaged; the eyeballs of the mice were removed, the blood was collected, and the serum was separated. The molecule department would measure the serum biochemical parameters. After the eyeball was removed and the blood was collected, the mice were euthanized for material collection: brain: the whole brain was removed and divided by the sagittal plane, and the left side was fixed, and the right side was quick-frozen; liver: the whole liver was removed and divided in two, the left lobe was fixed, and the rest were quick-frozen; spleen: the whole spleen was removed and divided in two, half fixed, half quick-frozen; kidney: the left kidney was removed for fixation, the right kidney was removed and quick-frozen; stomach: the whole stomach was removed and divided sagittal, the greater curvature was fixed, and the lesser curvature was quick-frozen; large intestine: the intact large intestine was removed for Swiss roll fixation; small intestine: the whole small intestine was removed and divided into three sections (duodenum, ileum, jejunum) for Swiss roll fixation; lung: the left lung was removed for fixation, and the right lung for removed for quick freezing; heart: the entire heart was removed for fixation after dilation. All fixed samples were sent to pathology for paraffin embedding, wherein 11 organs (brain, heart, lung, kidney, spleen, liver, stomach, duodenum, jejunum, ileum, and colon) of one KO mouse (#98) were sectioned, HE stained, and read for analysis.

The phenotype analysis results of wild-type (WT) and homozygous knockout mice (KO) are shown in **FIGURE 27**. The results show that no offspring was born after mating the Svs3a homozygous knockout mice, while there was no effect on reproductive function for the heterozygous knockout situation. No abnormality was found in other analyses. Therefore, complete loss or blockade of Svs3a function may affect fertility without significant toxic effects on other systems. This suggests that the possible toxicity or side effects of SEMG2 target blockade are limited and have high safety.

The embodiments of the present invention have been described above by the inventors, but the present invention is not limited thereto, and those skilled in the art can understand that modifications and changes can be made within the scope of the purpose of the present invention. The manner of modifications and changes should fall within the scope of protection of the present invention.

## Claims

1. A compound agonizing or antagonizing an interaction between SEMG2 and CD27.

2. The compound of claim 1, wherein the interaction between SEMG2 and CD27 is located on the amino acid site at positions 497, 498, 499, 500, 501, 502, 503, 504, 505, 506, and 508 of SEMG2, and the amino acid sequence of the SEMG2 protein is shown in SEQ ID NO:1.

3. The compound of claim 1, wherein the compound is a small molecule inhibitor, polypeptide, antibody, or antigen binding fragment.

4. The compound of claim 3, wherein the polypeptide comprises an amino acid sequence of SEQ ID NO:2 (QIEKLVEGKS), SEQ ID NO:86 (QIEKLVEGKS(x)I(x)), SEQ ID NO:87 (QIEKLVEGKS(x)I), or SEQ ID NO:88 (QIEKLVEGKS(x)); preferably the polypeptide comprises an amino acid sequence of SEQ ID NO:2, SEQ ID NO:3 (QIEKLVEGKSQIQ), SEQ ID NO:4 (QIEKLVEGKSQ), or SEQ ID NO:5 (QIEKLVEGKSQI), or an amino acid sequence at least 90% identity to an amino acid sequence as provided in SEQ ID NO: 2-5, wherein the x is selected from any amino acid.

5. The compound of claim 3, wherein the antibody specifically binds to native or mutant SEMG2 protein, the antibody binds to an antigenic epitope peptide derived from SEMG2 protein, the antigenic epitope peptide comprises an amino acid sequence of SEQ ID NO:2 (QIEKLVEGKS), SEQ ID NO:3 (QIEKLVEGKSQIQ), SEQ ID NO:4 (QIEKLVEGKSQ), or SEQ ID NO:5 (QIEKLVEGKSQI).

6. The compound of claim 3, wherein the antibody specifically binds to native or mutant SEMG2 protein, the antibody recognizes at least one amino acid residue at positions 497, 498, 499, 500, 501, 502, 503, 504, 505, 506 and 508 of the native SEMG2 protein, or recognizes an amino acid residue in the corresponding position of the mutant SEMG2 protein, the amino acid sequence of the native SEMG2 protein is shown in SEQ ID NO:1.

7. The compound of claim 3, wherein the antibody comprises a heavy chain variable region and a light chain variable region, the heavy chain variable region comprises HCDR1, HCDR2 and HCDR3 defined by IMGT; and the light chain variable region comprises LCDR1, LCDR2 and LCDR3 defined by IMGT,

the HCDR1 consists of or comprises an amino acid sequence selected from the group consisting of SEQ ID NOs:6-11, SEQ ID NOs:60-61 and SEQ ID NO:76;
the HCDR2 consists of or comprises an amino acid sequence selected from the group consisting of SEQ ID NOs:12-16 and SEQ ID NOs:62-64;
the HCDR3 consists of or comprises an amino acid sequence selected from the group consisting of SEQ ID NOs:17-20, SEQ ID NOs:65-67 and SEQ ID NOs:77-81;
the LCDR1 consists of or comprises an amino acid sequence selected from the group consisting of SEQ ID NOs:21-25, SEQ ID NOs:68-70 and SEQ ID NO:82;
the LCDR2 consists of or comprises an amino acid sequence selected from the group consisting of SEQ ID NOs:26-29, SEQ ID NOs:71-72, SEQ ID NOs:83-84 and SEQ ID NO:28;
the LCDR3 consists of or comprises an amino acid sequence selected from the group consisting of SEQ ID NOs:30-34, SEQ ID NOs:73-75, SEQ ID NO:85 and SEQ ID NO:95.

8. The compound of claim 7, wherein the antibody comprises a heavy chain variable region and a light chain variable region, the heavy chain variable region comprises HCDR1, HCDR2 and HCDR3 defined by IMGT; and the light chain variable region comprises LCDR1, LCDR2 and LCDR3 defined by IMGT, the CDR sequence of the antibody is selected from any one of the combinations in (a)-(k):

(a) the HCDR1 comprises the amino acid sequence of SEQ ID NO:6; the HCDR2 comprises the amino acid sequence of SEQ ID NO:12; the HCDR3 comprises the amino acid sequence of SEQ ID NO:17; the LCDR1 comprises the amino acid sequence of SEQ ID NO:21; the LCDR2 comprises the amino acid sequence of SEQ ID NO:26; the LCDR3 comprises the amino acid sequence of SEQ ID NO:30;
(b) the HCDR1 comprises the amino acid sequence of SEQ ID NO:7; the HCDR2 comprises the amino acid sequence of SEQ ID NO:13; the HCDR3 comprises the amino acid sequence of SEQ ID NO:18; the LCDR1 comprises the amino acid sequence of SEQ ID NO:22; the LCDR2 comprises the amino acid sequence of SEQ ID NO:27; the LCDR3 comprises the amino acid sequence of SEQ ID NO:31 or SEQ ID NO:95;
(c) the HCDR1 comprises the amino acid sequence of SEQ ID NO:6; the HCDR2 comprises the amino acid sequence of SEQ ID NO:16; the HCDR3 comprises the amino acid sequence of SEQ ID NO:17; the LCDR1 comprises the amino acid sequence of SEQ ID NO:21; the LCDR2 comprises the amino acid sequence of SEQ ID NO:26; the LCDR3 comprises the amino acid sequence of SEQ ID NO:30;
(d) the HCDR1 comprises the amino acid sequence of SEQ ID NO:8; the HCDR2 comprises the amino acid sequence of SEQ ID NO:13; the HCDR3 comprises the amino acid sequence of SEQ ID NO:18; the LCDR1 comprises the amino acid sequence of SEQ ID NO:23; the LCDR2 comprises the amino acid sequence of SEQ ID NO:27; the LCDR3 comprises the amino acid sequence of SEQ ID NO:32;
(e) the HCDR1 comprises the amino acid sequence of SEQ ID NO:9; the HCDR2 comprises the amino acid sequence of SEQ ID NO:14; the HCDR3 comprises the amino acid sequence of SEQ ID NO:19; the LCDR1 comprises the amino acid sequence of SEQ ID NO:24; the LCDR2 comprises the amino acid sequence of SEQ ID NO:28; the LCDR3 comprises the amino acid sequence of SEQ ID NO:33;
(f) the HCDR1 comprises the amino acid sequence of SEQ ID NO:10; the HCDR2 comprises the amino acid sequence of SEQ ID NO:15; the HCDR3 comprises the amino acid sequence of SEQ ID NO:20; the LCDR1 comprises the amino acid sequence of SEQ ID NO:25; the LCDR2 comprises the amino acid sequence of SEQ ID NO:29; the LCDR3 comprises the amino acid sequence of SEQ ID NO:34;
(g) the HCDR1 comprises the amino acid sequence of SEQ ID NO:11; the HCDR2 comprises the amino acid sequence of SEQ ID NO:15; the HCDR3 comprises the amino acid sequence of SEQ ID NO:20; the LCDR1 comprises the amino acid sequence of SEQ ID NO:25; the LCDR2 comprises the amino acid sequence of SEQ ID NO:29; the LCDR3 comprises the amino acid sequence of SEQ ID NO:34;
(h) the HCDR1 comprises the amino acid sequence of SEQ ID NO:60; the HCDR2 comprises the amino acid sequence of SEQ ID NO:62; the HCDR3 comprises the amino acid sequence of SEQ ID NO:65; the LCDR1 comprises the amino acid sequence of SEQ ID NO:68; the LCDR2 comprises the amino acid sequence of SEQ ID NO:71; the LCDR3 comprises the amino acid sequence of SEQ ID NO:73;
(i) the HCDR1 comprises the amino acid sequence of SEQ ID NO:61; the HCDR2 comprises the amino acid sequence of SEQ ID NO:63; the HCDR3 comprises the amino acid sequence of SEQ ID NO:66; the LCDR1 comprises the amino acid sequence of SEQ ID NO:69; the LCDR2 comprises the amino acid sequence of SEQ ID NO:72; the LCDR3 comprises the amino acid sequence of SEQ ID NO:74;
(j) the HCDR1 comprises the amino acid sequence of SEQ ID NO:60; the HCDR2 comprises the amino acid sequence of SEQ ID NO:64; the HCDR3 comprises the amino acid sequence of SEQ ID NO:67; the LCDR1 comprises the amino acid sequence of SEQ ID NO:70; the LCDR2 comprises the amino acid sequence of SEQ ID NO:28; the LCDR3 comprises the amino acid sequence of SEQ ID NO:75;

(k) the HCDR1 comprises the amino acid sequence of SEQ ID NO:60 or 76; the HCDR2 comprises the amino acid sequence of SEQ ID NO:64 or 62; the HCDR3 comprises the amino acid sequence of SEQ ID NO:77, 78 or 79; and/or

the LCDR1 comprises the amino acid sequence of SEQ ID NO:70 or 82; the LCDR2 comprises the amino acid sequence of SEQ ID NO:28, 83 or 84; the LCDR3 comprises the amino acid sequence of SEQ ID NO:75 or 85.

9. The compound of claim 3, wherein the antibody comprises a heavy chain variable region and a light chain variable region,

the heavy chain variable region comprises an amino acid sequence selected from the group consisting of SEQ ID NOs:35-41, 48-51, 54-56 and 96-100, or an amino acid sequence at least 70%, 80%, 90%, 95% or 99% identity to any sequence of SEQ ID NOs:35-41, 48-51, 54-56 and 96-100;
the light chain variable region comprises an amino acid sequence selected from the group consisting of SEQ ID NOs:42-47, 52-53, 57-69 and 101-103, or an amino acid sequence at least 70%, 80%, 90%, 95% or 99% identity to any sequences of SEQ ID NOs:42-47, 52-53, 57-69 and 101-103.

10. The compound of claim 9, wherein the antibody comprises a heavy chain variable region and a light chain variable region, the heavy chain variable region and the light chain variable region are selected from any one of the combinations in (a)-(o):

(a) the heavy chain variable region comprises SEQ ID NO:35 or an amino acid sequence at least 70%, 80%, 90%, 95% or 99% identity to SEQ ID NO:35; the light chain variable region comprises SEQ ID NO:42 or an amino acid sequence at least 70%, 80%, 90%, 95% or 99% identity to SEQ ID NO:42;
(b) the heavy chain variable region comprises SEQ ID NO:36 or an amino acid sequence at least 70%, 80%, 90%, 95% or 99% identity to SEQ ID NO:36; the light chain variable region comprises SEQ ID NO:43 or an amino acid sequence at least 70%, 80%, 90%, 95% or 99% identity to SEQ ID NO:43;
(c) the heavy chain variable region comprises SEQ ID NO:37 or an amino acid sequence at least 70%, 80%, 90%, 95% or 99% identity to SEQ ID NO:37; the light chain variable region comprises SEQ ID NO:44 or an amino acid sequence at least 70%, 80%, 90%, 95% or 99% identity to SEQ ID NO:44;
(d) the heavy chain variable region comprises SEQ ID NO:38 or an amino acid sequence at least 70%, 80%, 90%, 95% or 99% identity to SEQ ID NO:38; the light chain variable region comprises SEQ ID NO:45 or an amino acid sequence at least 70%, 80%, 90%, 95% or 99% identity to SEQ ID NO:45;
(e) the heavy chain variable region comprises SEQ ID NO:39 or an amino acid sequence at least 70%, 80%, 90%, 95% or 99% identity to SEQ ID NO:39; the light chain variable region comprises SEQ ID NO:46 or an amino acid sequence at least 70%, 80%, 90%, 95% or 99% identity to SEQ ID NO:46;
(f) the heavy chain variable region comprises SEQ ID NO:40 or an amino acid sequence at least 70%, 80%, 90%, 95% or 99% identity to SEQ ID NO:40; the light chain variable region comprises SEQ ID NO:47 or an amino acid sequence at least 70%, 80%, 90%, 95% or 99% identity to SEQ ID NO:47;
(g) the heavy chain variable region comprises SEQ ID NO:41 or an amino acid sequence at least 70%, 80%, 90%, 95% or 99% identity to SEQ ID NO:41; the light chain variable region comprises SEQ ID NO:47 or an amino acid sequence at least 70%, 80%, 90%, 95% or 99% identity to SEQ ID NO:47;
(h) the heavy chain variable region comprises SEQ ID NO:48, 49, 50, 51 or an amino acid sequence at least 70%, 80%, 90%, 95% or 99% identity to SEQ ID NO:48, 49, 50 or 51; the light chain variable region comprises SEQ ID NO:52 or 53 or an amino acid sequence at least 70%, 80%, 90%, 95% or 99% to SEQ ID NO:52 or 53;
(i) the heavy chain variable region comprises SEQ ID NO:54 or an amino acid sequence at least 70%, 80%, 90%, 95% or 99% identity to SEQ ID NO:54; the light chain variable region comprises SEQ ID NO:57 or an amino acid sequence at least 70%, 80%, 90%, 95% or 99% identity to SEQ ID NO:57;
(j) the heavy chain variable region comprises SEQ ID NO:55 or an amino acid sequence at least 70%, 80%, 90%, 95% or 99% identity to SEQ ID NO:55; the light chain variable region comprises SEQ ID NO:58 or an amino acid sequence at least 70%, 80%, 90%, 95% or 99% identity to SEQ ID NO:58;
(k) the heavy chain variable region comprises SEQ ID NO:56 or an amino acid sequence at least 70%, 80%, 90%, 95% or 99% identity to SEQ ID NO:56; the light chain variable region comprises SEQ ID NO:59 or an amino acid sequence at least 70%, 80%, 90%, 95% or 99% identity to SEQ ID NO:59;
(l) the heavy chain variable region comprises SEQ ID NO:96 or an amino acid sequence at least 70%, 80%, 90%, 95% or 99% identity to SEQ ID NO:96; the light chain variable region comprises SEQ ID NO:59, 101, 102, 103 or an amino acid sequence at least 70%, 80%, 90%, 95% or 99% identity to SEQ ID NO:59, 101, 102 or 103; (m) the heavy chain variable region comprises SEQ ID NO:97 or an amino acid sequence at least 70%, 80%, 90%, 95% or 99% identity to SEQ ID NO:97; the light chain variable region comprises SEQ ID NO:59 or an amino acid sequence at least 70%, 80%, 90%, 95% or 99% identity to SEQ ID NO:59; (n) the heavy chain variable region comprises SEQ ID NO:98 or an amino acid sequence at least 70%, 80%, 90%, 95% or 99% identity to SEQ ID NO:98; the light chain variable region comprises SEQ ID NO:103 or an amino acid sequence at least 70%, 80%, 90%, 95% or 99% identity to SEQ ID NO:103; (o) the heavy chain variable region comprises SEQ ID NO:99 or 100 or an amino acid sequence at least 70%, 80%, 90%, 95% or 99% identity to SEQ ID NO:99 or 100; the light chain variable region comprises SEQ ID NO:57 or an amino acid sequence at least 70%, 80%, 90%, 95% or 99% identity to SEQ ID NO:57.

11. The compound of any of the claims 5-10, wherein the antibody further comprises a coupling moiety linked to the polypeptide, the coupling moiety is selected from the group consisting one or more of radionuclides, drugs, toxins, cytokines, enzymes, fluorescein, carrier proteins, lipids, and biotin, wherein the polypeptide or antibody is selectively linked to the coupling moiety by a linker, preferably the linker is a peptide or polypeptide.

12. The compound of any of the claims 5-10, wherein the antibody is selected from monoclonal antibodies, polyclonal antibodies, antisera, chimeric antibodies, humanized antibodies, and human antibodies.

13. The compound of any of the claims 5-10, wherein the antibody is selected from multispecific antibodies, single chain Fv (scFv), single chain antibodies, anti-idiotype (anti-Id) antibodies, diabodies, minibodies, nanobodies, single domain antibodies, Fab fragments, F(ab') Fragments, disulfide-linked bispecific Fvs (sdFv) and intracellular antibodies.

14. An antigenic epitope peptide, wherein the antigenic epitope peptide is derived from SEMG2 protein, and the amino acid of the antigenic epitope peptide comprises an amino acid sequence selected from the group consisting of SEQ ID NO:2 (QIEKLVEGKS), SEQ ID NO:3 (QIEKLVEGKSQIQ), SEQ ID NO:4 (QIEKLVEGKSQ), and SEQ ID NO:5 (QIEKLVEGKSQI).

15. A protein, wherein the protein comprises the antigenic epitope peptide of claim 14 and a tag sequence which can selectively be linked at the N-terminus or C-terminus.

16. The protein of claim 15, wherein the protein comprises an amino acid sequence of SEQ ID NO:2 (QIEKLVEGKS), SEQ ID NO:86 (QIEKLVEGKS(x)I(x)), SEQ ID NO:87 (QIEKLVEGKS(x))I), or SEQ ID NO:88 (QIEKLVEGKS(x)), preferably the polypeptide comprises an amino acid sequence of SEQ ID NO:3 (QIEKLVEGKSQIQ), SEQ ID NO:4 (QIEKLVEGKSQ), or SEQ ID NO:5 (QIEKLVEGKSQI) or an amino acid sequence at least 90% identity to any one of SEQ ID NOs:2-5, more preferably SEQ ID NOs:89-94 and SEQ ID NO:3.

17. A method for preparing an antibody, including using an antigenic epitope peptide of claim 14 or a protein of claim 15 or 16 as immunogen to immunize mammals or obtained by screening in natural antibody library.

18. An isolated polynucleotide encoding the compound of any one of the claims 3-13, the antigenic peptide of claim 14, or the protein of claim 15 or 16.

19. A recombinant vector comprising the polynucleotide of claim 18 and optional regulatory sequences; preferably, the recombinant vector is a cloning vector or an expression vector.

20. The polynucleotide of claim 19, wherein the regulatory sequence is selected from a leading sequence, a polyadenylation sequence, a leaderpetide sequence, a promoter, a signal sequence, a transcription terminator, or any combination thereof.

21. A host cell comprising the recombinant vector of claim 19 or 20.

22. The host cell of claim 21, wherein, the host cell is a prokaryotic cell or a eukaryotic cell.

23. A pharmaceutical composition comprising the compound of any one of the claims 1 to 13, the antigenic peptide of claim 14, the protein of claim 15 or 16, the polynucleotide of claim 18, the recombinant vector of claim 19 or 20, and one or more types of the host cells of claim 21 or 22.

24. The pharmaceutical composition of claim 23, wherein, the pharmaceutical composition further comprises a pharmaceutically acceptable carrier or adjuvant.

25. The use of the compound of any of the claims 1 to 13, the antigenic peptide of claim 14, the protein of claim 15 or 16, the polynucleotide of claim 18, the recombinant vector of claim 19 or 20, or the host cells of claim 21 or 22 in preparation of products for agonizing or antagonizing the interaction between SEMG2 and CD27, preferably SEMG2 is expressed in tumor cells and CD27 is expressed in immune cells.

26. The use of the compound of any of the claims 1 to 13, the antigenic peptide of claim 14, the protein of claim 15 or 16, the polynucleotide of claim 18, the recombinant vector of claim 19 or 20, or the host cells of claim 21 or 22 in preparation of a drug for preventing or treating tumors.

27. The use of the compound of any of the claims 1 to 13, the antigenic peptide of claim 14, the protein of claim 15 or 16, the polynucleotide of claim 18, the recombinant vector of claim 19 or 20, or the host cells of claim 21 or 22 in preparation of a drug for modulating an immune response elicited against tumors.

28. The use of claims 26 and 27, wherein the tumor is selected from one or more of colorectal cancer, lung cancer, melanoma, lymphoma, liver cancer, head and neck cancer, stomach cancer, kidney cancer, bladder cancer, prostate cancer, testicular cancer, endometrial cancer, breast cancer and ovarian cancer.

29. A method of screening drugs or reagents for preventing or treating tumors, comprising obtaining candidate drugs or reagents by screening inhibitors or antibodies which inhibit the interaction between SEMG2 and CD27.

30. A method of preventing or treating tumors, comprising:
contacting immune cells such as lymphocytes and/or tumor cells of the subject with an effective dose of the compound of any one of the claims 1 to 13.

31. The method of claim 30, wherein the expression of SEMG2 in tumor cells is detected before contacting immune cells such as lymphocytes and/or tumor cells of the subject with an effective dose of the compound.

32. The method of claim 30, wherein the subject has received or is receiving or will receive additional anti-cancer therapy.

33. The method of claim 30, wherein the additional anti-cancer therapy comprises surgery, radiotherapy, chemotherapy, immunotherapy, or hormone therapy.

34. A kit comprising one or more of the compounds of any of the claims 1 to 13, the antigenic peptide of claim 14, the protein of claim 15 or 16, the polynucleotide of claim 18, and the recombinant vector of claim 19 or 20, and one or more types of the host cells of claim 21 or 22, and the above components are contained in a suitable container.

35. A method of detecting the presence or absence of SEMG2 in a biological sample *in vitro,* comprising: contacting the biological sample with the compound of any one of the claims 1-13.

36. A method of inhibiting the growth of tumor cells, comprising the following steps: A) analyzing the expression of SEMG2 in tumor cells; B) contacting the tumor cells with an antibody recognizing SEMG2, the binding of the antibody to SEMG2 is KD $<2\times10^{-8}$; C) contacting T lymphocytes with the antibody and tumor cells.

Figure 1

A

B   The effect of CD27 concentration on binding to SEMG2

Figure 2

Figure 3

Interaction between GFP-fused protein of full length and truncated mutant of human SEMG2 and human CD27

Interaction between GFP-fused protein of full length and truncated mutant of human SEMG2 and mouse CD27

## Figure 4

A  Glycine substitution scanning: vector design and numbering

| Mutant number | Amino acid sequence |
| --- | --- |
| 1 | AIEKLVEGKSQIQ |
| 2 | QAEKLVEGKSQIQ |
| 3 | QI**A**KLVEGKSQIQ |
| 4 | QIEALVEGKSQIQ |
| 5 | QIEKAVEGKSQIQ |
| 6 | QIEKLAEGKSQIQ |
| 7 | QIEKLVAGKSQIQ |
| 8 | QIEKLVEAKSQIQ |
| 9 | QIEKLVEG**A**SQIQ |
| 10 | QIEKLVEGKAQIQ |
| 11 | QIEKLVEGKS**A**IQ |
| 12 | QIEKLVEGKSQAQ |
| 13 | QIEKLVEGKSQIA |

B

## Figure 5

A

The effect of CD27 concentration on binding BSA-SEMG2 (497-509)

B

The interaction between full length SEMG2 protein and CD27 is competitively inhibited by a polypeptide

Figure 6

A  Apoptosis assay of HCT116 cells cocultured with activated human peripheral blood monocytes (PBMC)

Phase contrast microscopy    Nucleus (DAPI)    Apoptotic cells (CASP3 substrate)

Control empty vector

SEMG2 expression vector

B    Apoptotic ratio of tumor cells

Control empty vector    SEMG2 overexpression

Figure 7

SEMG2 protein expression in tumor cells

Figure 8

Immunohistochemistry (IHC) staining detection of SEMG2 in different normal and tumor tissues

Normal colorectal tissue    Colorectal cancer    Magnified image    Colorectal cancer    Magnified image
tissue    tissue

Case 1    Case 4

Case 2    Case 5

Case 3    Case 6

B    Normal lung tissue    Lung cancer tissue, case 1    Lung cancer tissue, case 2

C    D
Prostate cancer    Rate of SEMG2 positive expression among different types of tumors

Melanoma

Gastric cancer

Figure 9

Association between SEMG2 expression and
overall survival in colorectal cancer patients

Figure 10

Number of tumor tissue-infiltrating Treg/field of view (FOXP labelled)

Treg cells infiltration in tumor
(arrows, Foxp3 histochemical staining)

SEMG2 histochemical staining
in lung cancer tissue

Case (1)

Case (2)

Figure 11

Blocking effect of different antibodies on SEMG2 and CD27 (ELISA)

Polyclonal antibody produced by SEMG2 (497-509) as immunogen

Polyclonal antibody produced by full length SEMG2 protein as immunogen

← Median inhibition concentration

**Figure 12**

Effect of monoclonal antibodies produced by different immunogens on inhibition of SEMG2-CD27 binding

■ Clones inhibiting SEMG2-CD27 binding
□ Clones not inhibiting SEMG2-CD27 binding

**Figure 13**

Figure 14

Figure 15

Figure 16

Figure 17

T cell killing assay: sensitivities of different tumor cells
to SEMG2 antibody

Figure 18

| ELISA immobilized antigen | MM02 | MM05 | MM07 | MM08 | MM13 | MM14 | HPA042767 | HPA042835 |
|---|---|---|---|---|---|---|---|---|
| SEMG2 ( 1 – 582 ) | 1. 200 | 1. 168 | 1. 144 | 1. 130 | 1. 133 | 1. 123 | 1. 157 | 1. 173 |
| SEMG2 ( 354 – 403 ) | 0. 004 | 0. 008 | 0. 001 | 0. 012 | 0. 001 | 0. 003 | 1. 140 | 0. 009 |
| SEMG2 ( 442 – 453 ) | 0. 001 | 0. 009 | 0. 011 | 0. 008 | 0. 008 | 0. 007 | 0. 007 | 0. 002 |
| SEMG2 ( 497 – 509 ) | 1. 182 | 1. 171 | 1. 144 | 1. 140 | 1. 138 | 1. 112 | 0. 002 | 0. 009 |
| SEMG2 ( 563 – 574 ) | 0. 005 | 0. 000 | 0. 012 | 0. 001 | 0. 010 | 0. 000 | 0. 011 | 1. 163 |

Figure 19

| SEMG (497-509) and mutants | MM02 | MM05 | MM07 | MM08 | MM13 | MM14 | HPA042767 | HPA042835 |
|---|---|---|---|---|---|---|---|---|
| QIEKLVEGKSQIQ | 1.264 | 1.272 | 1.185 | 1.264 | 1.226 | 1.165 | 0.005 | 0.005 |
| AIEKLVEGKSQIQ | 0.450 | 0.638 | 0.919 | 0.942 | 0.480 | 0.813 | 0.004 | 0.000 |
| QAEKLVEGKSQIQ | 0.541 | 0.796 | 0.833 | 0.671 | 0.467 | 0.540 | 0.000 | 0.010 |
| QIAKLVEGKSQIQ | 0.788 | 0.728 | 0.480 | 0.544 | 0.670 | 0.776 | 0.009 | 0.010 |
| QIEALVEGKSQIQ | 0.470 | 0.726 | 0.822 | 0.789 | 0.712 | 0.777 | 0.004 | 0.008 |
| QIEKAVEGKSQIQ | 0.051 | 0.259 | 0.123 | 0.213 | 0.049 | 0.161 | 0.005 | 0.007 |
| QIEKLAEGKSQIQ | 0.928 | 0.558 | 0.814 | 0.509 | 0.658 | 0.804 | 0.009 | 0.008 |
| QIEKLVAGKSQIQ | 0.480 | 0.615 | 0.899 | 0.566 | 0.738 | 0.766 | 0.006 | 0.004 |
| QIEKLVEAKSQIQ | 0.766 | 0.723 | 0.580 | 0.824 | 0.842 | 0.470 | 0.003 | 0.004 |
| QIEKLVEGASQIQ | 0.949 | 0.852 | 0.790 | 0.939 | 0.818 | 0.621 | 0.003 | 0.005 |
| QIEKLVEGKAQIQ | 0.173 | 0.168 | 0.296 | 0.023 | 0.211 | 0.022 | 0.005 | 0.003 |
| QIEKLVEGKSAIQ | 1.259 | 1.256 | 1.161 | 1.223 | 1.186 | 1.178 | 0.006 | 0.003 |
| QIEKLVEGKSQAQ | 0.894 | 0.868 | 0.509 | 0.798 | 0.699 | 0.588 | 0.004 | 0.002 |
| QIEKLVEGKSQIA | 1.153 | 1.208 | 1.262 | 1.187 | 1.250 | 1.228 | 0.001 | 0.007 |

Figure 20

Figure 21

Figure 22

Figure 23

Figure 24

A

B

Figure 25

Figure 26

No difference in morphology of gene knockout mice

No abnormality in organ tissue slices of gene knockout mice

No difference in CD8+, CD4+, CD27+ cell ratio of gene knockout mice

| Genotype | Number | CD27+ of CD8+(%) | CD27+ of CD4+(%) |
|---|---|---|---|
| Wild-type | 79 | 88.71 | 66.33 |
| | 80 | 87.55 | 73.58 |
| | 92 | 72.79 | 41.17 |
| | 93 | 70.36 | 43.58 |
| | 110 | 80.95 | 48.46 |
| Mutant | 81 | 85.47 | 49.8 |
| | 82 | 39.23 | 71.19 |
| | 96 | 80.59 | 51.22 |
| | 106 | 68 | 33.69 |
| | 108 | 74.73 | 41.11 |

No significant difference in blood biochemistry test of gene knockout mice

No significant difference in liver function test of gene knockout mice

No significant difference in routine blood test of knockout mice

## Figure 27

## INTERNATIONAL SEARCH REPORT

International application No.

**PCT/CN2021/073100**

**A.    CLASSIFICATION OF SUBJECT MATTER**

C07K 16/00(2006.01)i;  C12N 15/13(2006.01)i;  A61K 39/395(2006.01)i;  A61P 35/00(2006.01)i

According to International Patent Classification (IPC) or to both national classification and IPC

**B.    FIELDS SEARCHED**

Minimum documentation searched (classification system followed by classification symbols)

C07K C12N A61K A61P

Documentation searched other than minimum documentation to the extent that such documents are included in the fields searched

Electronic data base consulted during the international search (name of data base and, where practicable, search terms used)

CNABS, CNKI, CNTXT, DWPI, SIPOABS, EPTXT, USTXT, WOTXT, JPTXT, NCBI, 中国专利生物序列检索系统, Chinese Patent Biological Sequence Retrieval System, Genbank, EMBL, STN and Search Items: SEMG2, 人精囊腺凝固蛋白2, 精囊蛋白, semenogelin, 抗体, CD27, 配体, 表位, epitope, SEQ ID Nos: 6, 12, 17, 21, 26, 30, 35, 42, 2-5, 86-94

**C.    DOCUMENTS CONSIDERED TO BE RELEVANT**

| Category* | Citation of document, with indication, where appropriate, of the relevant passages | Relevant to claim No. |
|---|---|---|
| A | CN 108025048 A (THE BROAD INSTITUTE INC.; DANA-FARBER CANCER INSTITUTE INC.; MASSACHUSETTS GEN HOSPITAL) 11 May 2018 (2018-05-11) claims 26, 49 and 85-88 | 1-29, 34 and 35 (in part) |
| A | US 2006084072 A1 (UNIVERSITY OF WASHINGTON) 20 April 2006 (2006-04-20) description, paragraphs [0007]-[0011], [0063], [0064], [0115]-[0120]、 [0152]-[0157], [0210] and [0210] | 1-29, 34 and 35 (in part) |
| A | 温彦 (WEN, Yan). "可溶性重组人精囊蛋白1片段的原核表达及其对精子活力的影响 (Prokaryotic Expression of Truncated Soluble Human Semenogelin 1 and Its Inhibition Tospermatozoa Motility )" 中国优秀硕士学位论文全文数据库 医药卫生科技辑 (Chinese Master's Theses Full-Text Database, Medical and Health Sciences), No. 05, 15 May 2017 (2017-05-15), ISSN: 1674-024, p. 54 | 1-29, 34 and 35 (in part) |
| A | CN 106414415 A (THE UNIVERSITY OF NORTH CAROLINA AT CHAPEL HILL) 15 February 2017 (2017-02-15) description, paragraphs [0051]-[0064] | 1-29, 34 and 35 (in part) |

☑ Further documents are listed in the continuation of Box C.      ☑ See patent family annex.

```
*    Special categories of cited documents:
"A"  document defining the general state of the art which is not considered
     to be of particular relevance
"E"  earlier application or patent but published on or after the international
     filing date
"L"  document which may throw doubts on priority claim(s) or which is
     cited to establish the publication date of another citation or other
     special reason (as specified)
"O"  document referring to an oral disclosure, use, exhibition or other
     means
"P"  document published prior to the international filing date but later than
     the priority date claimed
```

```
"T"  later document published after the international filing date or priority
     date and not in conflict with the application but cited to understand the
     principle or theory underlying the invention
"X"  document of particular relevance; the claimed invention cannot be
     considered novel or cannot be considered to involve an inventive step
     when the document is taken alone
"Y"  document of particular relevance; the claimed invention cannot be
     considered to involve an inventive step when the document is
     combined with one or more other such documents, such combination
     being obvious to a person skilled in the art
"&"  document member of the same patent family
```

| Date of the actual completion of the international search | Date of mailing of the international search report |
|---|---|
| **19 March 2021** | **15 April 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

## INTERNATIONAL SEARCH REPORT

| International application No. |
|---|
| **PCT/CN2021/073100** |

**C.    DOCUMENTS CONSIDERED TO BE RELEVANT**

| Category* | Citation of document, with indication, where appropriate, of the relevant passages | Relevant to claim No. |
|---|---|---|
| A | CN 109715209 A (OMEROS CORPORATION; UNIVERSITY OF LEICESTER) 03 May 2019 (2019-05-03)<br>claim 43, and sequence list | 1-29, 34 and 35 (in part) |

## INTERNATIONAL SEARCH REPORT

| International application No. |
|---|
| **PCT/CN2021/073100** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT

| International application No. |
|---|
| **PCT/CN2021/073100** |

**Box No. II     Observations where certain claims were found unsearchable (Continuation of item 2 of first sheet)**

This international search report has not been established in respect of certain claims under Article 17(2)(a) for the following reasons:

1. ☑ Claims Nos.: **30-33和36**
   because they relate to subject matter not required to be searched by this Authority, namely:

   [1]   PCT Rule 39.1(iv) - Methods for the treatment of the human or animal body by therapy

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

[1]   Invention 1: claims 1-29, 34 and 35 (in part);

[2]   Which relates to anti-SEMG2 monoclonal antibodies with amino acid sequences SEQ ID Nos: 6, 12, 17, 21, 26, 30 (6 CDRs), 35 (VH) and 42 (VL), and corresponding nucleic acids, vectors, host cells, pharmaceutical compositions, methods of the preparation of antibodies, use in the preparation of drugs for the prevention or treatment of tumors or for the modulation of immune responses against tumors , kits and methods for in vitro detection of the presence or absence of SEMG2 in biological samples.

[3]   Invention 2-18: claims 1-29, 34 and 35 (in part); having the same subject matter as Invention 1, but the related amino acid sequences are as follows:

[4]   Invention 2: SEQ ID NOs: 7, 13, 18, 22, 27, 31 (6 CDRs), 36 (VH) and 43 (VL);

[5]   Invention 3: SEQ ID NOs: 6, 16, 17, 21, 26, 30 (6 CDRs), 37 (VH) and 44 (VL)

[6]   Invention 4: SEQ ID NOs: 8, 13, 18, 23, 27, 32 (6 CDRs), 38 (VH) and 45 (VL)

[7]   Invention 5: SEQ ID NOs: 9, 14, 19, 24, 28, 33 (6 CDRs), 39 (VH) and 46 (VL)

[8]   Invention 6: SEQ ID NOs: 10, 15, 20, 25, 29, 34 (6 CDRs), 40 (VH) and 47 (VL)

[9]   Invention 7: SEQ ID NOs: 11, 15, 20, 25, 29, 34 (6 CDRs), 41 (VH) and 47 (VL)

[10]   Invention 8: SEQ ID NOs: 60, 62, 65, 68, 71, 73 (6 CDRs), 54 (VH) and 57 (VL)

[11]   Invention 9: SEQ ID NOs: 61, 63, 66, 69, 72, 74 (6 CDRs), 55 (VH) and 58 (VL)

[12]   Invention 10: SEQ ID NOs: 60, 64, 67, 70, 28, 75 (6 CDRs), 56 (VH) and 59 (VL)

[13]   Invention 11: SEQ ID NOs: 60, 64, 77, 70, 28, 75 (6 CDRs), 96 (VH) and 59 (VL)

[14]   Invention 12: SEQ ID NOs: 60, 64, 77, 70, 83, 75 (6 CDRs), 96 (VH) and 101 (VL)

[15]   Invention 13: SEQ ID NOs: 60, 64, 77, 70, 84, 75 (6 CDRs), 96 (VH) and 102 (VL)

[16]   Invention 14: SEQ ID NOs: 60, 64, 77, 82, 28, 85 (6 CDRs), 96 (VH) and 103 (VL)

[17]   Invention 15: SEQ ID NOs: 60, 64, 79, 70, 28, 75 (6 CDRs), 97 (VH) and 59 (VL)

[18]   Invention 16: SEQ ID NOs: 60, 64, 78, 82, 28, 85 (6 CDRs), 98 (VH) and 103 (VL)

[19]   Invention 17: SEQ ID NOs: 76, 62, 80, 68, 71, 73 (6 CDRs), 99 (VH) and 57 (VL)

[20]   Invention 18: SEQ ID NOs: 60, 62, 81, 68, 71, 73 (6 CDRs), 100 (VH) and 57 (VL)

Form PCT/ISA/210 (continuation of first sheet) (January 2015)

## INTERNATIONAL SEARCH REPORT

| International application No. |
| --- |
| **PCT/CN2021/073100** |

**Box No. III      Observations where unity of invention is lacking (Continuation of item 3 of first sheet)**

[22] The corresponding technical feature among inventions 1-18 is "monoclonal antibody conjugated to SEMG2".
Document 1 discloses (CN108025048 A 20180511, see claims 26, 49, 85-88): the tumor-specific mutant
antigen SEMG2: p.R292C, given a corresponding antibody to recognize the tumor-specific neoepitope, wherein
said antibody can be humanized, fully humanized or chimeric. It can be seen that the above technical features
are disclosed in Document 1. Therefore, the technical solutions claimed by claims 1-29, 34 and 35 do not share
a same or corresponding specific technical feature, are not so linked as to form a single general inventive
concept and therefore do not comply with the requirement of unity of invention as defined in PCT Rule 13.1
and 13.2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-29, 34 and 35 (monoclonal antibodies relating to SEMG2 with amino acid sequences SEQ ID Nos: 6, 12, 17, 21, 26, 30 (6 CDRs), 35 (VH) and 42 (VL))**

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/CN2021/073100**

| Patent document cited in search report | | | Publication date (day/month/year) | Patent family member(s) | | | Publication date (day/month/year) |
|---|---|---|---|---|---|---|---|
| CN | 109715209 | A | 03 May 2019 | PH | 12019500204 | A1 | 20 January 2020 |
| | | | | EC | SP19015192 | A | 29 March 2019 |
| | | | | US | 10745274 | B1 | 18 August 2020 |
| | | | | AU | 2017306069 | A1 | 03 January 2019 |
| | | | | CR | 20190105 | A | 14 May 2019 |
| | | | | CL | 2019000230 | A1 | 12 July 2019 |
| | | | | SG | 10202010277S | A | 27 November 2020 |
| | | | | CO | 2019001212 | A2 | 19 February 2019 |
| | | | | MX | 2019000965 | A | 29 August 2019 |
| | | | | CA | 3031980 | A1 | 08 February 2018 |
| | | | | WO | 2018026722 | A1 | 08 February 2018 |
| | | | | CU | 20190006 | A7 | 04 September 2019 |
| | | | | UY | 37349 | A | 28 February 2018 |
| | | | | IL | 264592 | D0 | 28 February 2019 |
| | | | | US | 2020317510 | A1 | 08 October 2020 |
| | | | | JO | P20170154 | A1 | 30 January 2019 |
| | | | | AU | 2017306069 | B2 | 03 September 2020 |
| | | | | EP | 3490603 | A1 | 05 June 2019 |
| | | | | SG | 10202010279Y | A | 27 November 2020 |
| | | | | US | 2020270125 | A1 | 27 August 2020 |
| | | | | PE | 20190389 | A1 | 13 March 2019 |
| | | | | JP | 2019533982 | A | 28 November 2019 |
| | | | | KR | 20190035796 | A | 03 April 2019 |
| | | | | AU | 2020250188 | A1 | 05 November 2020 |
| | | | | BR | 112019001247 | A2 | 25 June 2019 |
| | | | | EP | 3490603 | A4 | 04 November 2020 |
| | | | | US | 2018140697 | A1 | 24 May 2018 |
| | | | | AU | 2020204551 | A1 | 30 July 2020 |
| | | | | SG | 11201900606V | A | 27 February 2019 |
| | | | | US | 10639369 | B2 | 05 May 2020 |
| | | | | TW | 201805304 | A | 16 February 2018 |
| | | | | WO | 2018026722 | A8 | 27 December 2018 |
| CN | 106414415 | A | 15 February 2017 | EP | 3116861 | A1 | 18 January 2017 |
| | | | | EP | 3116861 | A4 | 13 September 2017 |
| | | | | WO | 2015138919 | A1 | 17 September 2015 |
| | | | | CN | 111620830 | A | 04 September 2020 |
| | | | | US | 2017057934 | A1 | 02 March 2017 |
| | | | | EP | 3116861 | B1 | 16 December 2020 |
| | | | | US | 10472334 | B2 | 12 November 2019 |
| | | | | CA | 2942078 | A1 | 17 September 2015 |

Form PCT/ISA/210 (patent family annex) (January 2015)

# EP 4 079 758 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **CLAUS C ; RIETHER C ; SCHÜRCH C ; MATTER MS ; HILMENYUK T ; OCHSENBEIN AF.** *Cancer Res.,* 15 July 2012, vol. 72 (14), 3664-76
- **REMEDIOS KA ; ZIRAK B ; SANDOVAL PM ; LOWE MM ; BODA D ; HENLEY E et al.** *Sci Immunol.,* 21 December 2018, vol. 3 (30), eaau2042
- **WINKELS H ; MEILER S ; LIEVENS D ; ENGEL D ; SPITZ C ; BÜRGER C et al.** *Eur Heart J.,* 2017, vol. 38 (48), 3590-3599
- **GOODWIN, R . G . et al.** *Cell,* 1993, vol. 73, 447-56
- **BOWMAN et al.** *Immunol,* 1994, vol. 152, 1756-61
- **HINTZEN et al.** *J. Immunol.,* 1994, vol. 152, 1762-1773
- **OSHIMA et al.** *Int. Immunol.,* 1998, vol. 10, 517-26

- **TESSELAAR et al.** *J. Immunol.,* 2003, vol. 170, 33-40
- **JUNKER et al.** *J. Urol.,* 2005, vol. 173, 2150-3
- **SLOAN et al.** *, Am J Pathol.,* 2004, vol. 164, 315-23
- **HELD-FEINDT ; MENTLEIN et al.** *Int J Cancer,* 2002, vol. 98, 352-6
- **YOSHIDA K ; KARZAI ZT ; KRISHNA Z ; YOSHIKA M ; KAWANO N ; YOSHIDA M et al.** *Cell Motil Cytoskeleton.,* 2009, vol. 66 (2), 99-108
- **EDSTRÖM AM ; MALM J ; FROHM B ; MARTEL-LINI JA ; GIWERCMAN A ; MÖRGELIN M et al.** *J Immunol.,* 2008, vol. 181 (5), 3413-21
- **JONSSON M ; LINSE S ; FROHM B ; LUNDWALL A ; MALM J.** *Biochem J.,* 2005, vol. 387, 447-53